# EUROPEAN PATENT APPLICATION

(11) **EP 2 527 426 A1**
(43) Date of publication of application: **28.11.2012**
(21) Application number: 12179223.8
(22) Date of filing: 07.08.2008
(51) Int. Cl.: C12N 5/075

(54) **Isolation, characterization and propagation of germline stem cells**

(30) Priority: 07.08.2007 US 954496 P; 06.02.2008 US 26502 P; 10.06.2008 US 60356 P
(62) Divisional of application: 08797432.5
(71) Applicant: PrimeGen Biotech LLC, Irvine, CA 92618 (US)
(72) Inventor: Izadyar, Fariborz, Irvine, CA 92606 (US); Pacchiarotti, Jason, Garden Grove, CA 92844 (US); Maki, Chad, Huntington Beach, CA 92648 (US); Romas, Thomas,V., Pomona, CA 91766 (US)
(74) Representative: Green, Mark Charles

(57) **Abstract**

Methods are provided for the isolation, characterization and propogation of germline cells stem cells from fetal and adult mammals. Additionally, isolated populations of germ line cells having different phenotypes are disclosed wherein the subpopulations are capable of forming long-term cultures of multipotent or pluripotent cells or are capable of differentiating into mature germline cells and repopulating a sterile reproductive organ. The multipotent or pluripotent germline cells are also suitable for differentiation into tissue-specific somatic cells for therapeutic purposes.

## Description

### CROSS REFERENCE TO RELATED APPLICATIONS

The present application claims priority under 35 U.S.C. §119(e) to U.S. Provisional Patent Application Numbers 60/954,496 filed August 7, 2007 and 61/060,356 filed June 10, 2008, the entire contents of which are incorporated herein by reference in their entirety.

### FIELD OF THE INVENTION

The present invention relates to the field of male and female germline stem cells, isolated during fetal and post-natal development and the use of these cells for therapeutic purposes. Specifically, the present invention relates to identification, isolation, and differentiattion of distinct germline stem cell populations, and cell lines generated therefrom, with different potential uses in cell replacement therapy.

### BACKGROUND OF THE INVENTION

Generation of pluripotent cell lines which can be safely used in regenerative medicine has a great potential impact in cell replacement therapy. In this regard, embryonic stem (ES) cells have been considered as potential cell sources because they can be propagated indefinitely and can be differentiated into phenotypes of all three germ layers. However, before ES cell applications can be realized, at least ethical issues must be resolved, and teratomas formation after transplantation must also be overcome. Adult stem cells originating from tissues have also been considered as alternative sources for cell-based therapy particularly since they do not form teratomas after transplantation, and they maintain the ability to differentiate into phenotypes of the same tissue lineage. Adult stem cells can also be induced to trans-differentiate into cell types of different lineages, or reprogrammed to become pluripotent stem cells for possible clinical applications. Among all adult stem cells, only germline stem cells (GSC) retain the ability to transmit pristine genetic information to offspring. Several lines of evidence suggest that GSCs acquire pluripotentiality through reprogramming processes that occur during normal development. Therefore, GSCs are theoretically a model for generation of pluripotent or multipotent adult stem cell lines.

Stem cells are primitive cells that give rise to other types of cells. Also called progenitor cells, there are several kinds of stem cells. Totipotent cells are considered the "master" cells of the body because they contain all the genetic information needed to create all the cells of the body plus the placenta, which nourishes the human embryo. Human cells have this totipotent capacity only during the first few divisions of a fertilized egg. After three to four divisions of totipotent cells, there follows a series of stages in which the cells become increasingly specialized. The next stage of division results in pluripotent cells, which are highly versatile and can give rise to any cell type except the cells of the placenta or other supporting tissues of the uterus. At the next stage, cells become multipotent, meaning they can give rise to several other cell types, but those types are limited in number. An example of multipotent cells is hematopoietic cells - blood cells that can develop into several types of blood cells, but cannot develop into brain cells. At the end of the long chain of cell divisions that make up the embryo are "terminally differentiated" cells - cells that are considered to be permanently committed to a specific function.

Stem cells are a rare population of cells that can give rise to vast range of cells tissue types necessary for organ maintenance and function. These cells are defined as undifferentiated cells that have two fundamental characteristics; (i) they have the capacity of self-renewal, (ii) they also have the ability to differentiate into one or more specialized cell types with mature phenotypes. There are three main groups of stem cells; (i) adult or somatic (post-natal), which exist in all post-natal organisms, (ii) embryonic, which can be derived from a pre-embryonic or embryonic developmental stage and (iii) fetal stem cells (pre-natal), which can be isolated from the developing fetus. Each group of stem cells has their own advantages and disadvantages for cellular regeneration therapy, specifically in their differentiation potential and ability to engraft and function de novo in the appropriate or targeted cellular environment.

In the post-natal animal there are cells that are lineage-committed progenitor stem cells and lineage-uncommitted pluripotent stem cells, which reside e.g. in connective, muscle and adipose tissues providing the post-natal organism the cells required for continual organ or organ system maintenance and repair. These cells are termed somatic or adult stem cells and can be quiescent or non-quiescent. Typically adult stem cells share two characteristics: (i) they can make identical copies of themselves for long periods of time (long-term self renewal); and (ii) they can give rise to mature cell types that have characteristic morphologies and specialized functions.

Unfortunately, virtually every somatic cell in the adult animal's body, including stem cells, possess a genome ravaged by time and repeated cell division. During the lifetime of an organism, cells are exposed to a wide range of factors, such as environmental carcinogens, UV light and solar radiation, all of which are capable of inducing genomic damage. Reactive oxygen species, as byproducts of cellular metabolism, are also intrinsic factors compromising the genomic integrity of cells. Increasingly, scientists are being led to believe that organs and tissues must have systems for self-renewal, and that those systems probably involve resident tissue stem cells or stem cells derived from circulation. As an organism ages, it is possible that damage to resident stem cells accumulates and that the repair systems are compromised.

Telomeres are the physical ends of chromosomes that contain highly conserved, tandemly repeated DNA sequences. Telomeres are involved in the replication and stability of linear DNA molecules and serve as counting mechanism in cells; with each round of cell division the length of the telomeres shortens and at a pre-determined threshold, a signal is activated to initiate cellular senescence. Stem cells and somatic cells produce telomerase, which inhibits shortening of telomeres, but their telomeres still progressively shorten during aging and cellular stress. Telomeric regions of the genomes in the reproductive germline stem cells could theoretically represent a more pristine prime state that is closer to that found in embryonic and prenatal stem cells.

Germ line stem cells resident in the reproductive organs, i.e., the ovaries and testes, represent potentially one of the most genetically protected classes of stem cells in the mammalian body. Genetic conservation has been suggested by findings of high telomerase activity in stem cells derived from these tissues, as well as, extensive DNA modification with chromatin chromosomal modifications. Scientists have differed about what types of stem cells are resident in adult reproductive tissues, as well as, their potentiality in differentiation.

The ontogeny of mammalian development provides a central role for stem cells. Early in embryogenesis, cells from the proximal epiblast destined to become germ cells (primordial germ cells) migrate along the genital ridge. These cells express high levels of alkaline phosphatase as well as expressing the transcription factor Oct4. Upon migration and colonization of the genital ridge, the primordial germ cells undergo differentiation into male or female germ cell precursors (primordial germline precursor cells). During male reproductive development, the primordial germline precursor cells become closely associated with precursor sertoli cells leading to the beginning of the formation of the seminiferous cords. When the primordial germline precursor cells are enclosed in the seminiferous cords, scientist presently believe that they differentiate into mitotically quiescent gonocytes, i.e., precursors of spermatogonial stem cells (SSC). These gonocytes, in turn, divide for a few days followed by arrest at G₀/G₁ phase of the cell cycle. In mice and rats these gonocytes resume division within a few days after birth to generate SSC which, in turn, differentiate and undergo meiosis in the process of spermatogenesis. The existence of residual primordial germline sex cells in adult tissues has been controversial. If they exist in adult tissues, these cells could be directly responsible for generating gonocytes and/or SSC. Theoretically, primordial germline sex cells might not be lineage committed and could be comparable in genomic quality to embryonic stem cells. Methods for their routine identification and isolation are therefore useful.

Spermatogonial stem cells (SSC) are the male germline stem cells that through their self-renewal, maintain the continuous production of spermatogonia leading to spermatozoa and sperm. It has been postulated that similar types of stem cells may exist in the female germline, but those cells have proven difficult to identify and isolate. Whether precursor stem cells exist for SSC, i.e., germline precursor stem cells, has been difficult to ascertain. Presumably present in very small numbers, their sheltered location has complicated isolation and purification. Precursors of SSC are potentially a good source of high quality stem cells for human therapies, if they can be isolated and identified. Unfortunately, the location and identifying characteristics of these cells within the seminiferous tubule have been difficult to discern.

Male germline stem cells (GSC) maintain spermatogenesis by self renewal of spermatogonial stem cells (SSC) and generation of spermatogonia committed to differentiation. Under certain in vitro conditions, GSC from both neonatal and adult mouse testis can reportedly generate multipotent germ cell lines (mGC) that have characteristics and differentiation potential similar to embryonic stem cells (ESC). However, mGCs generated in different laboratories showed different germ cell characteristics i.e. some retain their SSC properties and some have lost it completely. Thus, the possibility remains that the derivative multipotent germ cell lines may have been derived from different subpopulations of germline stem cells resident within the mouse testes.

Generation of embryonic stem cell lines had been thought to provide a renewable source of embryonic stem cells for both research and therapy but reports now indicate that existing cell lines are less robust than originally believe and many have also been contaminated during culture with immunogenic animal molecules. Embryonic stem cells (ESC) were originally proposed as "universal donor" cells because they were believed to be immunologically privileged and thus not subject to graft rejection; and, they could be differentiated into customized multipotent or lineage-committed cells. ESC are derived from the inner cell mass of the pre-implantation blastocyst-stage embryo and have great differentiation potential, being capable of giving rise to cells found in all three germ layers of the embryo proper. From a practical standpoint, embryonic stem cells are an artifact of cell culture since, in their natural epiblast environment, they only exist transiently during embryogenesis. Manipulation of embryonic stem cells *in vitro* has lead to the generation and differentiation of a wide range of cell types, including cardiomyocytes, hematopoietic cells, endothelial cells, nerves, skeletal muscle, chondrocytes, adipocytes, liver and pancreatic islets. However, embryonic stem cells may not be appropriate for direct transplant as they form teratomas after transplantion. Additionally there are moral and ethical issues associated with the isolation of embryonic stem cells from human embryos. Stem cell types with ESC-like properties are thus highly desirable alternatives for use in regenerative medicine.

Therefore, there is a need for novel sources of biologically useful, non-embryonic and non-fetal, pluripotent stem cells having genomes in a nearly physiologically prime state. Furthermore, there is a need for sources of stem cells with the latter properties for use in human and veterinary therapies.

### SUMMARY OF THE INVENTION

The present disclosure provides methods for isolation, purification and culture expansion of germline stem cells from both males and females. Also provided is evidence that germline stem cells isolated according to the disclosed methods demonstrate molecular characteristics similar to pluripotent stem cells and have telomerase activities that t compare favorably with the that of undamaged, pre-natal or embryonic stem cells. Also disclosed are distinct classes of germline stem cells from the male gonad that have 1) the potential to become spermatogonial stem cells with the ability of restoring spermatogenesis and 2) multipotent cell characteristics and can be differentiated into multiple cell types.

In one embodiment, an isolated population of germline cells is provided consisting of isolated gonadal cells characterized by the expression of at least one marker selected from the group consisting of germline stem cell surface markers GFR-α1, α6-integrin, Thy-1, SSEA-4, CD9, *Dolichos biflourus* agglutinin (DBA), germ cell markers VASA and DAZL, and spermatogonial stem cell marker PLZF; the expression of at least one germline stem cell gene selected from the group consisting of telomerase, VASA, c-RET,GFR- α1, DAZL, and PLZF and the expression of a high level of telomerase.

In another embodiment, the isolated population of germline cells are male. In another embodiment, the cells are female. In another embodiment, the cells are mammalian. In yet another embodiment, the cells are isolated from a fetus, such as a fetus between 11 and 22 weeks of gestation. In another embodiment, the cells are isolated from a post-natal individual.

In another embodiment, the isolated population of germline cells optionally express pluripotent markers selected from the group consisting of Oct-4, Nanog and alkaline phosphatase.

In another embodiment, the isolated population of germline cells express cell surface markers for both GFR-α1 and α6-integrin. In yet another embodiment, the isolated population of germline cells express cell surface markers for both Thy-1 and α6-integrin and a gene encoding a telomerase. In yet another embodiment, the cells express cell surface markers for SSEA4, GFR-α1 and germ cell marker VASA. In one embodiment, greater than about 50% of the cells express both GFR-α1 and VASA. In yet another embodiment, the cells do not express at least one marker selected from the group consisting of Adhesion/Activating Molecule (EpCAM) and c-Kit.

In one embodiment, the isolated population of germline cells express both Oct-4 and c-Kit and exhibit at least one of the properties selected from the group consisting of a cell cycle doubling time of about 72 hours; expression of germline-specific genes at a higher level than expressed in embryonic stem cells (ESC); expression of pluripotency genes at a lower level than ESC; a greater dependent on glial cell dependent neurotrophic factor (GDNF) for their self renewal than on leukemia inhibitory factor (LIF) or fibroblast growth factor (FGF2); and a lower level of SSEA-1 expression than ESC. In one embodiment, the pluripotent genes are selected from the group consisting of Oct-4, Nanog, Dppa-5, Sox2, alkaline phosphatase and Crypto.

In another embodiment, the isolated population of germline cells are isolated from a male, do not express c-Kit and are capable of differentiating into spermatogenic cells. In another embodiment, these cells s express both α-integrin and Thy-1.

In another embodiment, the isolated population of germline cells are isolated from a female and express Oct-4, Nanog, alkaline phosphatase and VASA. In another embodiment, the isolated population of germline cells are isolated from a female and are capable of differentiating into an oocyte-like cell.

In another embodiment, the higher level of telomerase activity is at least 20% of the telomerase activity of embryonic stem cells. In another embodiment, the higher level of telomerase activity is at least 50% the telomerase activity of embryonic stem cells.

In another embodiment, the isolated population of germline cells are pluripotent or multipotent. In another embodiment, the isolated population of germline cells are capable of differentiating into cells of the ectoderm, cells of the endoderm and cells of the mesoderm. In yet another embodiment, the cells are capable of differentiating into dopamine-producing cells. In yet another embodiment, the cells are not capable of forming teratomas.

In one embodiment, the isolated population of germline cells are isolated from a primate testis and the cells express Thy-1, α6-integrin, and SSEA-4 and do not express c-Kit. In another embodiment, these cells further express GFRα, Nanog and VASA. In yet another embodiment these cells are capable of differentiating into spermatogenic cells.

In one embodiment, a long-term culture of cells is provided comprising the germline cells disclosed herein.

In one embodiment, a method is provided for isolating a population of germline stem cells, comprising the steps of: selecting a gonadal cell exhibiting a germline stem cell surface marker phenotype; and determining that said gonadal cells expresses at least one germline stem cell gene.

In another embodiment, the gonadal cells express both Oct-4 and c-Kit. In further embodiments, the gonadal cells are female or male.

In another embodiment, the germline surface marker phenotype comprises at least one phenotype selected from the group consisting of Epithelial Cell Adhesion/Activating Molecule (EpCAM) negative, GDNF receptor (GFR-α1) positive, c-Kit negative, *Dolichos biflourus* agglutinin (DBA) positive, CD9 positive, CD90 positive, CD49f positive, SSEA4 positive and α6-integrin positive. In another embodiment, the germline stem cell gene is selected from the group consisting of telomerase, VASA, c-RET,GFR-α1, DAZL and PLZF.

In another embodiment, the step of selecting a germline stem cell surface marker phenotype comprises selecting for cells that do not express c-Kit and express both CD90 and CD49f.

In another embodiment, the step of selecting a germline stem cell surface marker phenotype comprises selecting for cells that do not express c-Kit and do express SSEA4.

In another embodiment, the step of selecting a germline stem cell surface marker phenotype comprises selecting for cells that do not express c-Kit and do express all of SSEA4, CD90 and CD49f.

In another embodiment, the step of selected a germline stem cell surface marker phenotype comprises selecting for cells that express both of α6-integrin and CD90 and do not express c-Kit.

In another embodiment, the cells further express SSEA-4.

### BRIEF DESCRIPTION OF THE DRAWINGS

Figure 1 depicts human fetal gonocytes during the expansion process. Figure 1A depicts alkaline-positive gonocytes at the onset of culture. Figure 1B depicts mushroom colonies after one week of culture in PM culture medium. Figure 1C depicts a flat colony after two weeks of culture. Figure 1D depicts a flat colony after several passages in serum-free culture medium.

Figure 2 depicts the expression of pluripotent markers on human fetal gonocyte-derived cells. Alkaline phosphatase (Figure 2A); SSEA-4 (Figure 2B); TRAI-60 (Figure 2C); TRAI-81 (Figure 2D); Oct-4 and human mitochondrial protein (MP, Figure 2E); Nanog and human MP (Figure 2F).

Figure 3 depicts the growth of fetal gonocyte-derived colonies under serum-free and feeder-free conditions. HF-89-p2 colony (Figure 3A); HF-22-p7 colony (Figure 3B).

Figure 4 depicts expression of pluripotent and germ cells markers by human fetal gonocyte-derived cells.

Figure 5 depicts telomerase activity in human fetal gonocyte-derived cells.

Figure 6 depicts formation of embryoid bodies (Figures 6A and 6B) and expression of markers associated with cell types derived from all three germ layers (Figure 6C) on human fetal gonocyte-derived cells.

Figure 7 depicts directional differentiation of human fetal gonocyte-derived cells into cardiomyocytes (Figure 7A), smooth muscle (Figure 7B), chondrocytes (Figure 7C), neurons (Figure 7D), astrocytes (Figure 7E), neuronal cell types (Figure 7F), oligodendritic (Figure 7G), and neuroprogenitor cells (Figure 7H).

Figure 8 depicts RT-PCR results of differentiation of human fetal gonocyte-derived cells that have been differentiated to cell types of the neural lineage.

Figure 9 depicts RT-PCR results of differentiation of human fetal gonocyte-derived cells that have been differentiated to cell types derived from all three germ layers.

Figure 10 depicts RT-PCR results of differentiation of human fetal gonocyte-derived cells that have been differentiated to cell types of the neural lineage.

Figure 11 depicts RT-PCR results of differentiation of human fetal gonocyte-derived cells that have been differentiated to cell types of the cardiac lineage.

Figure 12 depicts enrichment of GFP (green fluorescent protein) positive subpopulations of testicular stem cells isolated from the transgenic OG2 mouse using flow cytometry. Oct-4 positive cells as indicated by GFP expression were found as a distinct cell population in both neonatal (Figure 12B) and adult (Figure 12C) OG2 mouse compared to the wild type (Figure 12A). Among the Oct-4 positive cells, two clear subpopulations consisting of c-Kit positive (R5) and c-Kit negative (R2) were found (Figures 12D-12E). Correlation between expression of GFP and c-Kit is also shown (Figures 12F-12H).

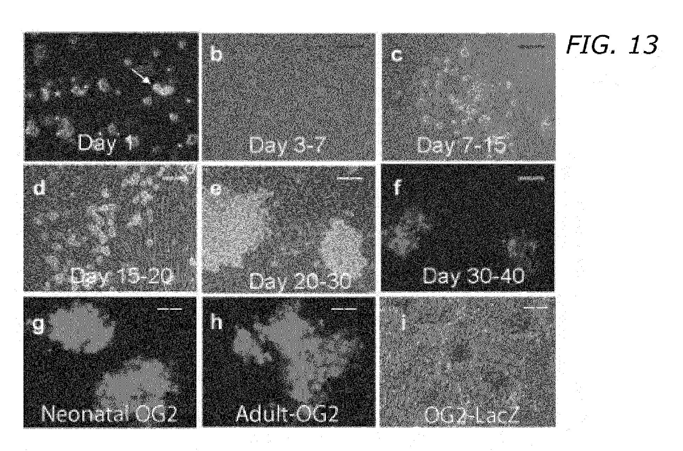

Figure 13 depicts morphological changes during the development of multipotent germ cell (mGC) lines in culture. Mouse Oct-4-GFP+ cells were observed in cell preparations before culture (Figure 13A; arrows; Day 1-3). Shortly after culture, down regulation of Oct-4 was observed Figure 13 B; Day 3-7). After the attachment of the cells in the second week of culture obvious morphological changes occurred (Figure 13C Day 7-15; Figure 13D Day 15-20). Approximately three weeks after culture, colonies containing small round cells were formed (Figure 13E; Day 20-30). Up-regulation of Oct-4 was observed about one month after culture (Figure 13F; Day 30-40). Images of three established mGC lines derived from neonatal OG2, adult OG2 or neonatal OG2-LacZ are presented in Figure 13G-20I (Figure 13G, Neonatal OG2; Figure 13H, Adult OG2; Figure 13I, OG2 LacZ), respectively. Scale bars: 50 µm.

Figure 14 depicts multipotent germline precursor cells cultured on mouse embryonic fibroblast (MEF) feeder layers in PM-1™ medium supplemented with 15% fetal bovine serum (FBS). At different time points during culture, the number of GFP positive cells was determined using fluorescence-assisted cell sorting (FACS), (Figure 14A, Day 3; Figure 14B, Day 5; Figure 14C, Day 9; Figure 14D, Day 15). Figure 14E depicts a graph of cell numbers vs. time. Scale bars are equivalent to 60 µm.

Figure 15 depicts phenotypic and molecular characterization of mGCs. Immunolocalization of pluripotent and germ cell markers are depicted in Figures 15A-15D for Oct-4, Figures 15E-15H for Nanog, Figures 15M-15O for SSEA-1 and Figures 15I-15L for the germ cell marker, VASA. Scale bars: Figures 15A-15H: 25 µm; Figure 15I-15O: 20 µm. Expression of pluripotent and germ-specific markers determined by RT-PCR is shown in Figure 15Q. The Western blot analysis of protein contents of Oct-4, Nanog and Sox2 in mGC cells before and after immunoprecipitation (IP) is presented in Figure 15Q.

Figure 16 depicts telomerase activity and karyotype analysis in adult adipose-derived stem cells (ADSC), mouse ES cells, freshly isolated germline stem cells after c-Kit sorting and multipotent germline stem cells at passage 10 (neonatal OG2). The telomerase activity in the germline stem cells is comparable to mouse ES cells and higher than the ADSC cells (Figure 16A). Figure 16B depicts the karyotype of the same neonatal OG2 cell line. The picture is representative of the 80 metaphase spreads that were analyzed. After 15 passages the cells exhibit a normal karyotype.

Figure 17 depicts imprinting analysis of multipotent germline precursor cells before (NGC) and after (GC) culture and compared with mouse ES cells for differentially methylated regions Meg3, Peg10, Oct-4, Igf2r and Rasgrf1.

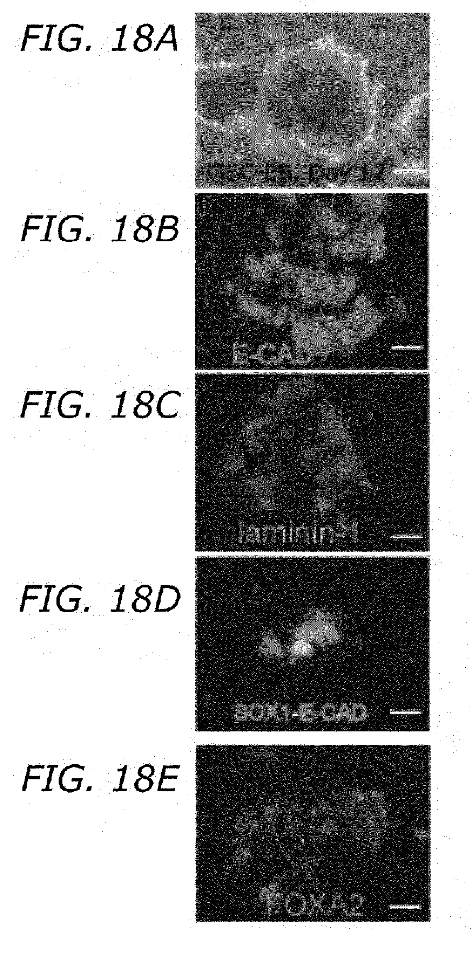

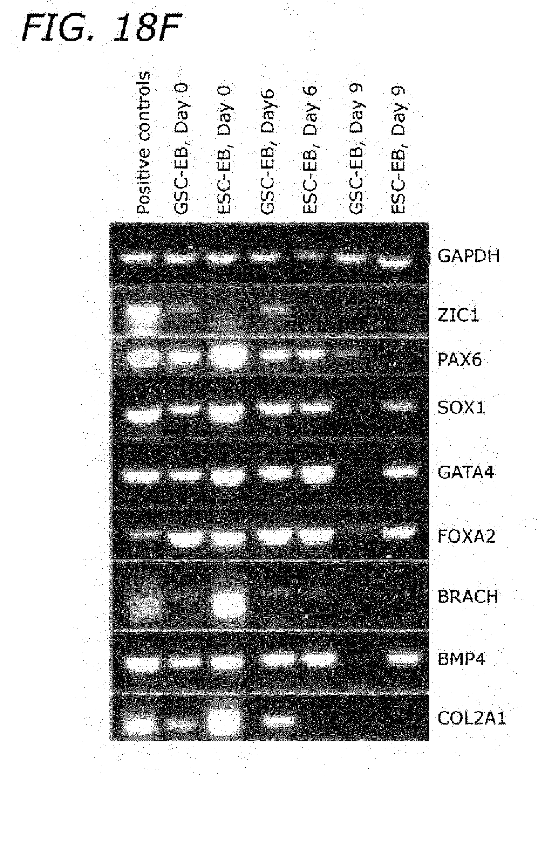

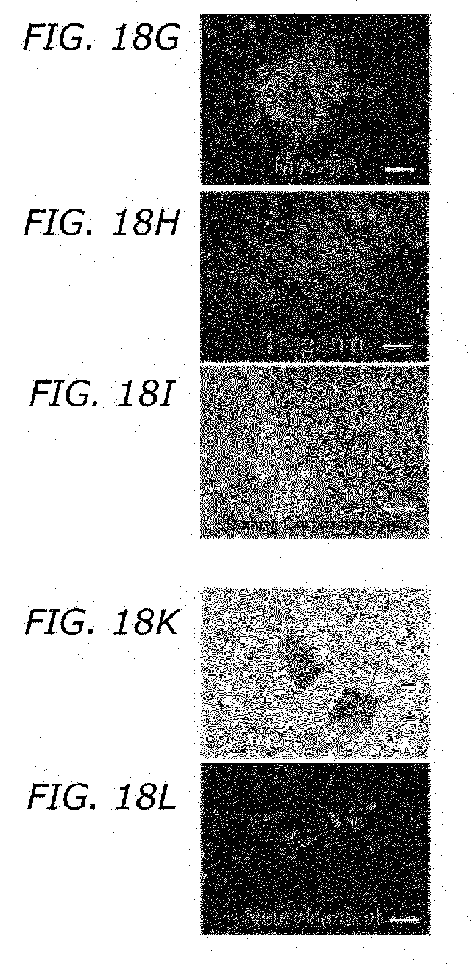

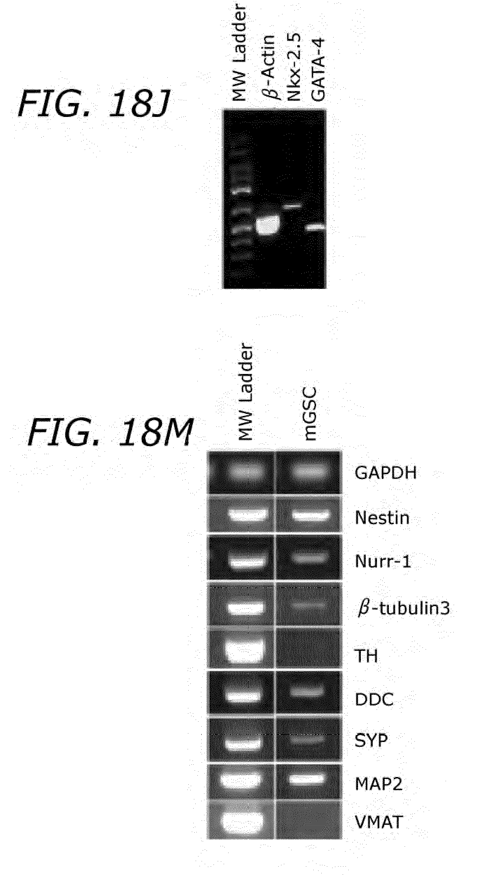

Figure 18 depicts the spontaneous differentiation of mGCs. Gastulation of embryoid body (EB; Figure 18A) and the expression of markers indicative of polarized epithelium (E-cadherin and laminin1; Figure 18B-18C) and early development of the three germ layers, i.e., ectoderm (ZIC1, PAX6, SOX1), endoderm (GATA4, FOXA2) and mesoderm (BRACHYURY, BMP4 and COL2A1) are shown in Figure 18D-18F. During culture reprogramming mGCs also differentiated spontaneously into cardiomyocytes (Figure 18G-18J), adipocytes (Figure 18K) and neural cells (Figure 18L and 18M). Scale bars: Figure 18A and 18I: 50 µm; Figure 18C and 18E: 30 µm; Figure 18B and 18D: 25 µm; Figure 18G, 18H and 18L: 45 µm; Figure 18K: 12 µm.

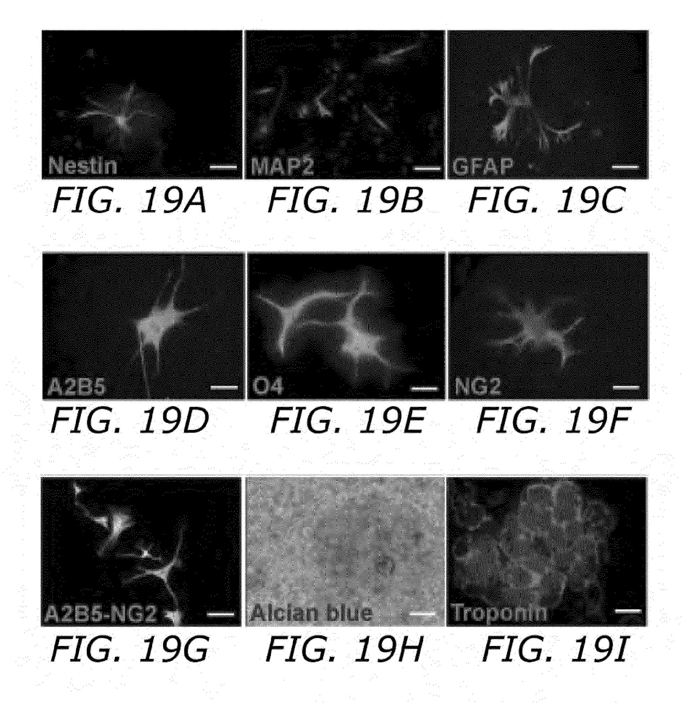

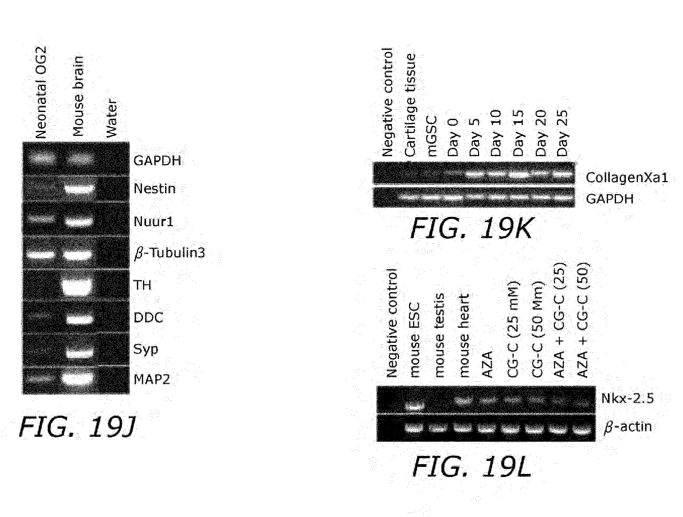

Figure 19 depicts the induced differentiation of mGCs into lineage-specific phenotypes. Confocal images of the cells expressed neural markers are shown in Figure 19A-19G. Expression of the neural gene markers is shown in Figure 19J. Confocal image of mGCs differentiated into cardiomyocytes are presented in Figure 19I. Expression of the cardiac gene markers is shown in Figure 19L. Alcian blue positive chondrocyte after differentiation of mGCs is shown in Figure 19H. Expression of the chondrocyte specific genes is shown in Figure 19K. Scale bars: Figure 19A, 19C and 19G: 20 µm; Figure 19B and 19H: 50 µm; Figure 19D-19F and 19J: 10 µm.

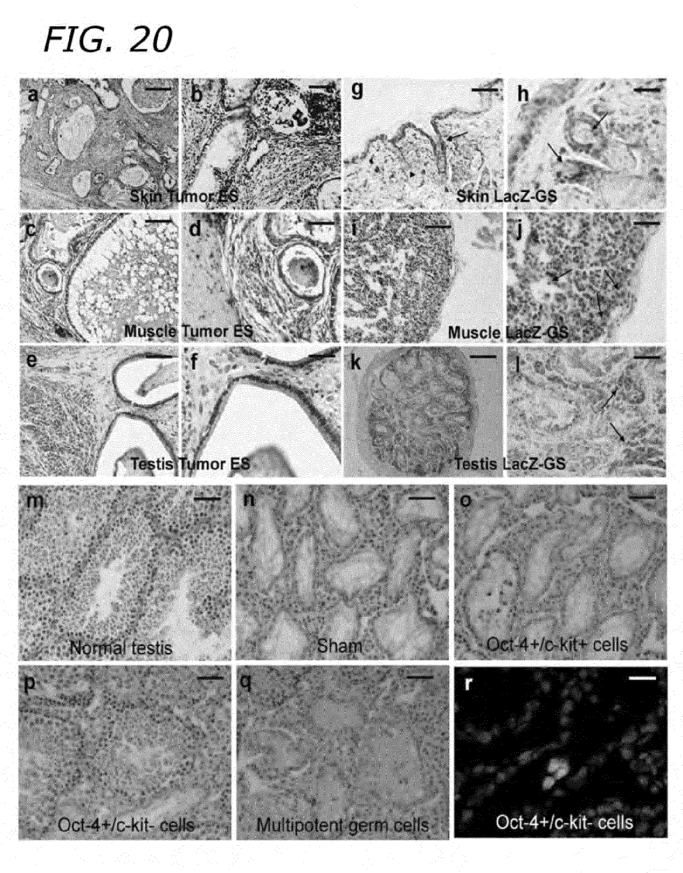

Figure 20 depicts the formation of teratomas after transplantation of mouse ES cells (Figure 20A-20F) but not multipotent LacZ-GFP mGCs (Figure 20G-20L) into the skin, muscle and testis. The morphology of ESC-derived teratomas was identified by H&E staining on thin paraffin sections, whereas the fate of transplanted multipotent germ cells (mGCs) was identified by LacZ staining shown in blue. Six weeks after transplantation, GFP-LacZ⁺ mGCs were found in the skin (in the bulge area of hair follicles and adjacent sebaceous glands, arrow head; Figure 20G-20H), in the muscle (arrows; Figure 20I-20J), and in the testis (arrows; Figure 20K). Testes regeneration following transplantation of germline stem cells before and after culture is presented in Figure 20M-20R. Cross section of the normal testis of an immune deficient mouse is shown in Figure 20M. One month after busulfan treatment the majority of the seminiferous tubules are depleted from endogenous spermatogenesis (Figure 20N). Testes of a mouse transplanted with freshly isolated Oct-4 positive cells showed spermatogenesis in more than 50% of seminiferous tubule cross sections indicating the presence of cells with SSC property in this population (Figure 20O). While more than 80% of seminiferous tubules of the mice transplanted with Oct-4 positive c-Kit negative cells showed some degree of spermatogenesis (Figure 20P), the majority of tubule cross sections of the mice received Oct-4 positive c-Kit positive cells were empty (Figure 20Q). Transplanted mGC also failed to repopulate recipient testes indicating that they do not have SSC properties (Figure 20R). Scale bars: Figure 20A and 20K: 275 µm; Figure 20B, 20D and 20I: 60 µm; Figure 20C: 140 µm; Figure 20E: 100 µm; Figure 20F: 50 um; Figure 20G and 20L: 125 µm; Figure 20H and 20J: 40 µm; Figure 20M-20R: 60 µm.

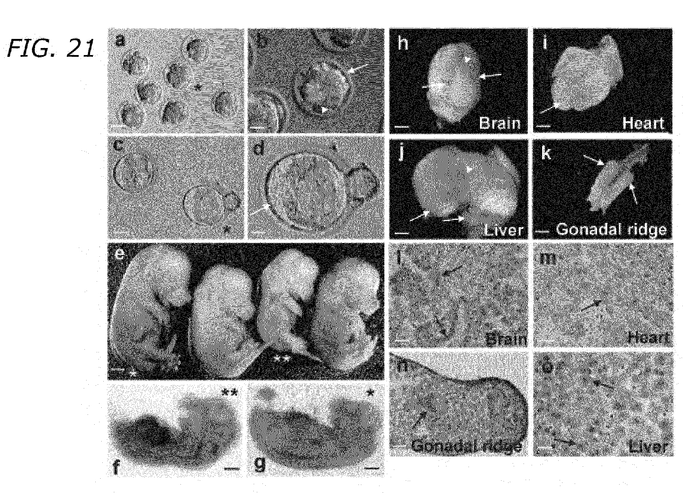

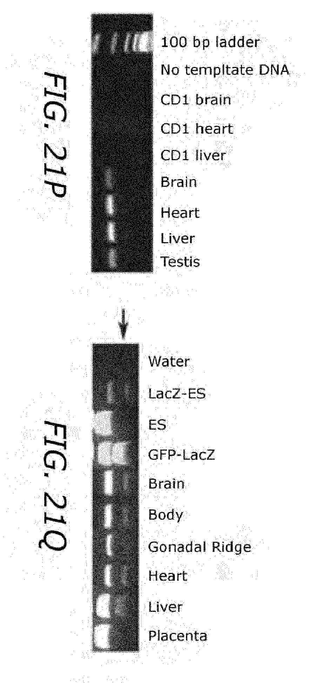

Figure 21 depicts chimera formation after incorporation of mGCs into blastocysts and host embryos. The incorporation of LacZ-GFP⁺ mGC cells during early embryonic development and blastocyst formation is presented in Figure 21A-21D. The majority of the GFP-lacZ cells injected at 8-cell stage have been incorporated at day two of the embryonic development (arrow head) and some cells have not been incorporated yet (arrows). GFP positive cells were further found at day 3.5 incorporated in inner cell mass (arrow) of the blastocyst. An example of four chimeric embryos showing different degree of chimerism is shown in Figure 21E as whole embryo staining. To visualize the internal organs, sagital sections of two of the embryos (indicated by asterisks) are also shown (Figure 21F and 21G). Figure 21H-21K show the chimeric pattern in dissected organs and Figure 21L-21O show the chimeric cell population in histological sections in the brain, heart, liver and gonadal ridge (chimeric LacZ-GFP cells appear in blue). Amplification of the GFP and LacZ DNA in tissues of the chimeric pups is shown in Figure 21P and 21Q, respectively. Scale bars: Figure 21A and 21C: 50 µm; Figure 21B and 21D: 25 µm; Figure 21E-21D: 1250 µm; Figure 21H-21 K: 625 µm; Figure 21L-21N: 50 µm; Figure 21O: 10 µm.

Figure 22 depicts graphically in a flow cytometric plot the results of freshly isolated neonatal and adult ovarian cells from transgenic OG2 mice wherein the Oct-4 promoter drives expression of GFP. Figure 22A shows adult ovarian germline stem cells as depicted graphically with the fluorescence intensity of GFP. Figure 22B shows neonatal ovarian germline stem cells as depicted graphically with the fluorescence intensity of GFP. Figure 22C depicts graphically the fluorescence intensity of neonatal GFP positive cells, from Figure 22B, expressing c-Kit, also known as CD117.

Figure 23 depicts germline stem cells identifiable by expression of green fluorescent protein in a cross section of the ovary of a transgenic OG2 mouse at day 2 after birth. Figures 23A and 23B show total fluorescence and Figure 23C shows a computer enhanced image with removal of auto-fluorescence.

Figure 24 depicts the results of RT-PCR analysis of mRNA isolated from mouse embryonic stem cells (lane 3), mouse embryonic fibroblasts (MEF, lane 4), GFP-positive germline stem cells (GFP+ cells, lane 5) and GFP-negative (GFP- cells, lane 6) isolated from an OG2 transgenic mouse.

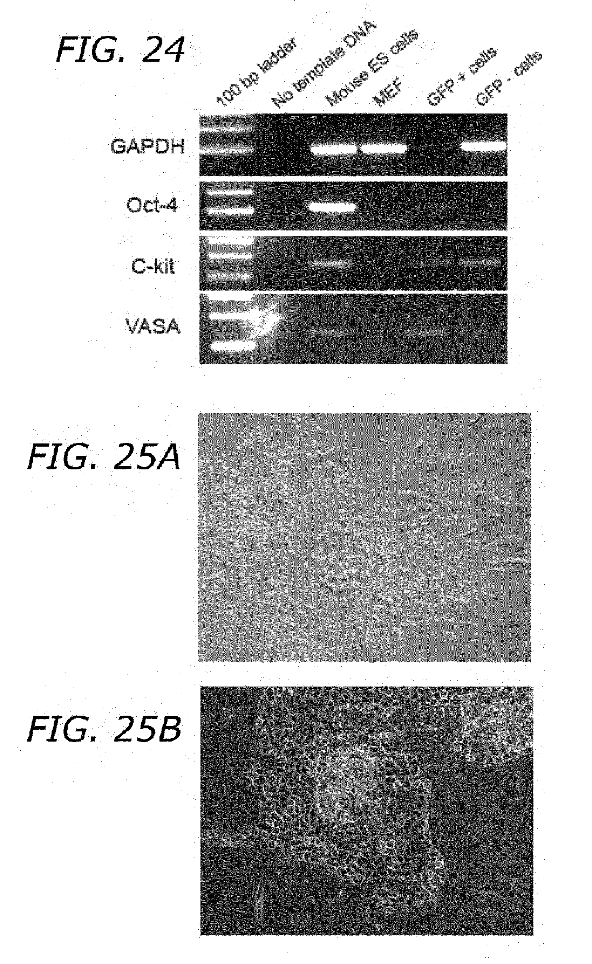

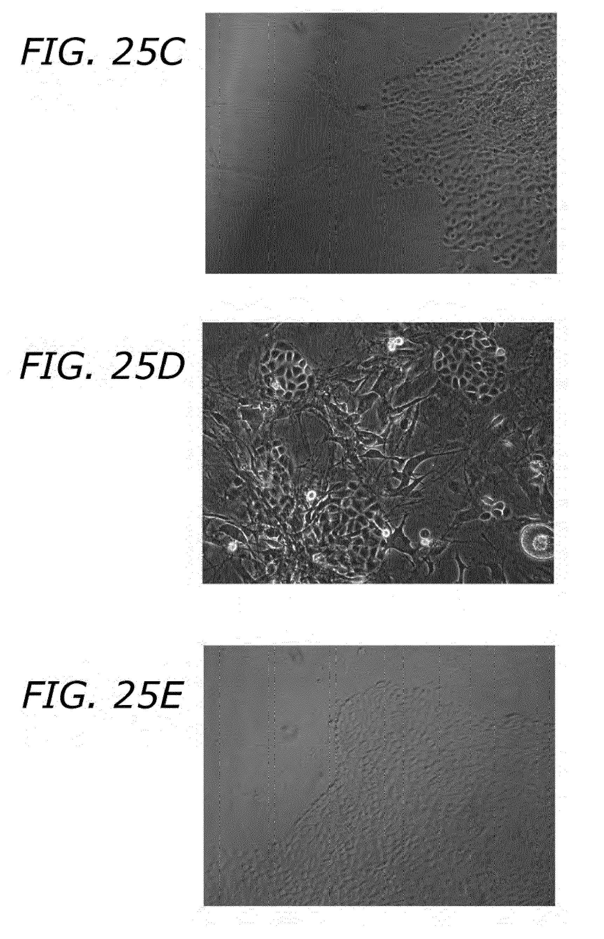

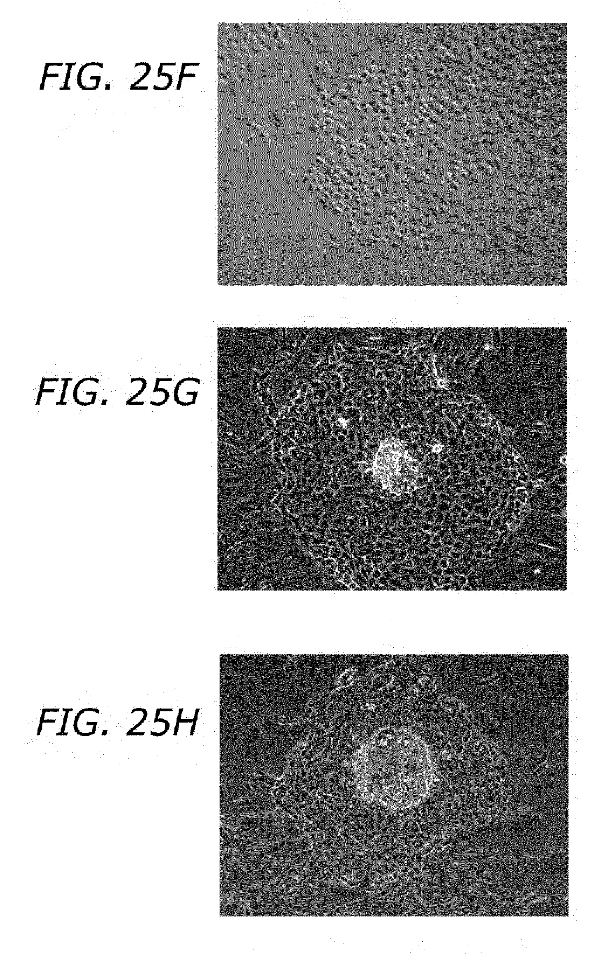

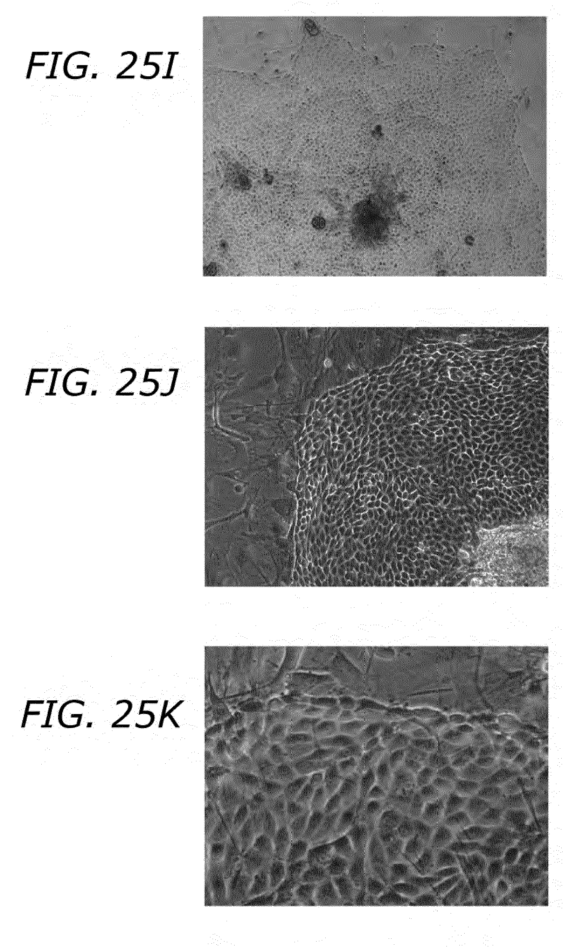

Figure 25 depicts microscopic images of isolated and substantially purified ovarian germline stem cells established and growing as colonies on a feeder layer of MEF cells. Figure 25A shows colonies after 4 days in culture; Figure 25B shows a first type of representative colony morphology; Figure 25C shows a second type of representative colony morphology; Figure 25D shows colony morphology after passage with collagenase; Figure 25E shows a third type of representative colony morphology after collagenase passage having a clearly defined border; Figure 25F shows a fourth type of representative colony morphology after collagenase passage having a poorly defined border; Figure 25G shows colony morphology after passage #1; Figure 25H shows colony morphology after passage #2; Figure 25I shows colony morphology after passage #3; and Figures 25J and 25K show two different magnifications of ovarian germline stem cell colonies after passage #4.

Figure 26 depicts immunocytochemical staining of isolated and substantially purified ovarian germline stem cells, stained to reveal expression of pluripotent stem cell marker Oct-4 (Figure 26A and 26B, with a negative control to confirm proper staining Figure 26C); pluripotent stem cell marker Nanog (Figure 26D and 26E; with a negative control to confirm proper staining Figure 26F); germ cell marker VASA (Figure 26G and 26H); and pluripotent stem cell marker alkaline phosphatase (Figures 26I-26K).

Figure 27 depicts images of differentiating ovarian germline stem cells. Figure 27A shows GFP positive cells resembling primary oocytes growing at the center of the female germ cell colony; Figure 27B shows images of follicle-like structures; Figure 27C shows GFP positive cells resembling primary oocytes growing in the vicinity of the female germ cell colony; and Figure 27 depicts a pigmented colony.

Figure 28 graphically depicts in a scatter plot the results of cytofluorimetric size analysis of the cultured ovarian cells of Figure 27 confirming and characterizing the large (>15µm) oocyte-like cells: Figure 28A depicting MEF control cells (<15µm); and Figure 28B depicting the oocyte-like cells of Figure 27.

Figure 29 depicts immunohistochemical localization of spermatogonial stem cell and germline cell markers in adult primate testes.

Figure 30 depicts the distribution of cells positively stained with stem cell markers at the basement membrane of seminiferous tubules of primate testes.

Figure 31 depicts phenotypic characterization of primate germline stem cells using flow cytometry.

Figure 32 depicts flow cytometric analysis of primate germline stem cells.

Figure 33 depicts immunohistochemical localization of spermatogonial stem cell and germ cell markers in enriched primate germline cell populations.

Figure 34 depicts GFRα positive/VASA positive cells in different enriched primate germline cell populations.

Figure 35 depicts the carboxyfluorescein diacetate succinimidyl ester (CSFE) activity of subpopulations of primate germline stem cells.

Figure 36 depicts repopulation of busulfan-treated primate testes with primate germline stem cells. Figure 36A: seminiferous tubules of recipient mice transplanted by non sorted cells; Figure 36B: cells sorted by triple markers; Figure 36C: SSEA-4 positive sorted cells ; and Figure 36D: sham transplanted control testes.

Figure 37 depicts the DNA content determined by flow cytometry of propidium idodine stained populations of primate germline stem cells.

Figure 38 depicts the quantitative PCR analysis of PLZF expression in primate germline stem cells.

Figure 39 depicts the quantitative PCR analysis of telomerase activity in primate germline stem cells.

Figure 40 depicts the percentages of proliferating primate germline stemcells determined by proliferating cell nuclear antigen (PCNA).

Figure 41 depicts the gene expression profile of subpopulations of primate germline stem cells.

Figure 42 depicts the morphology of an expanded primate germline stem cell colony 10 days after culture on MEF feeder layer.

Figure 43 depicts the morphology of an expanded primate germline stem cell colony.

Figure 44 depicts SSEA-4 staining of expanded primate germline stem cell colonies.

Figure 45 depicts GFR-α staining of an expanded primate germline stem cell colony after passage 4.

Figure 46 depicts α6-integrin staining of an expanded primate germline stem cell colony after passage 4.

Figure 47 depicts germ cell marker VASA staining of an expanded primate germline stem cell colony after passage 4

Figure 48 depicts primate germline stem cells prior to differentiation into dopamine-producing cells. Figure 48A depicts cells cultured for 5 days; Figure 48B depicts the cells after addition of retinoic acid; Figure 48C depicts the retinoic acid-treated cells after 5 days of culture in fresh medium and Figure 48D depicts differentiated cells after an additional 15 days in culture.

Figure 49 depicts immunocytochemistry analysis of primate germline stem cells differentiated into dopamine-producing cells and stained for dopamine receptor 1 (Figure 49A), human nuclear protein which stains primate nuclei (Figure 49B) and co-localization of the two markers (Figure 49C).

Figure 50 depicts immunocytochemistry analysis of primate germline stem cells differentiated into dopamine-producing cells and stained for dopamine receptor 2 (Figure 50A), human nuclear protein which stains primate nuclei (Figure 50B) and co-localization of the two markers (Figure 50C).

Figure 51 depicts immunocytochemistry analysis of primate germline stem cells differentiated into dopamine-producing cells and stained for tyrosine hydroxylase (Figure 51 A), human nuclear protein which stains primate nuclei (Figure 51 B) and co-localization of the two markers (Figure 51 C).

Figure 52 depicts immunocytochemistry analysis of primate germline stem cells differentiated into dopamine-producing cells and stained for vesicular monamine transporter (Figure 52A), human nuclear protein which stains primate nuclei (Figure 52B) and co-localization of the two markers (Figure 52C).

Figure 53 depicts immunocytochemistry analysis of primate germline stem cells differentiated into dopamine-producing cells and stained for dopa decarboxylase (Figure 53A), human nuclear protein which stains primate nuclei (Figure 53B) and co-localization of the two markers (Figure 53C).

Figure 54 depicts negative controls for Figures 49-53 (Figure 54A), human nuclear protein which stains primate nuclei (Figure 50B) and co-localization (Figure 54C).

Figure 55 depicts immunocytochemistry analysis of primate germline stem cells differentiated into dopamine-producing cells and stained for dopamine transporter (Figure 55A), human nuclear protein which stains primate nuclei (Figure 55B) and co-localization of the two markers (Figure 55C).

Figure 56 depicts negative controls for Figure 55 (Figure 56A), human nuclear protein which stains primate nuclei (Figure 56B) and co-localization (Figure 56C).

Figure 57 depicts human THT-1 stained with SSEA4 (Figure 57A) and VASA (Figure 57B) and the two merged (Figure 57C).

Figure 58 depicts THT2 stained with GFR-alpha (Figure 58A) and VASA (Figure 58B) and the two merged (Figure 58C).

Figure 59 depicts THT-1 stained for VASA (Figure 59A) and Nanog (Figure 59B) and the two merged (Figure 59C).

Figure 60 depicts human bHT-1 stained for SSEA-4 (Figure 60A) and alpha-6 integrin (Figure 60B) and the two combined (Figure 60C).

Figure 61 depicts negative controls for Figures 57-60 consisting of human testis sections stained only with secondary antibody.

Figure 62 depicts human THT-2 SSEA4 positive magnetic bead sorted cells transplanted into busulfan treated recipient mouse testes and after one month stained for SSEA4 (Figure 62A) and human nuclear protein (Figure 62B) and the two merged (Figure 62C).

Figure 63 depicts the negative control for Figure 62 consisting of human testis sections stained only with second antibody.

Figure 64 depicts human THT-2 SSEA4 positive magnetic bead sorted cells transplanted into busulfan treated recipient mouse testes and after one month stained for alpha-6 integrin (Figure 64A) and human nuclear protein (Figure 64B) and the two merged (Figure 64C).

Figure 65 depicts human THT-2 SSEA4 positive magnetic bead sorted cells transplanted into busulfan treated recipient mouse testes and after one month stained for SSEA-4 (Figure 65A) and alpha-6 integrin(Figure 65B) and the two merged (Figure 65C)

Figure 66 depicts the negative control for Figures 64 and 65 consisting of human testis sections stained only with second antibody.

### DEFINITION OF TERMS

The following definition of terms is provided as a helpful reference for the reader. The terms used in this patent have specific meanings as they related to the present invention. Every effort has been made to use terms according to their ordinary and common meaning. However, where a discrepancy exists between the common ordinary meaning and the following definitions, these definitions supercede common usage.

Committed: As used herein, "committed" refers to cells which are considered to be permanently committed to a specific function. Committed cells are also referred to as "terminally differentiated cells."

Culture: As used herein, "culture" or "cultured" refers to the propagation of cells under controlled conditions such that cell division and increase in cell numbers occurs.

Dedifferentiation: As used herein, "dedifferentiation" refers to loss of specialization in form or function. In cells, dedifferentiation leads to a less committed cell.

Differentiation: As used herein, "differentiation" refers to the adaptation of cells for a particular form or function. In cells, differentiation leads to a more committed cell.

Embryo: As used herein, "embryo" refers to an individual in the early stages of growth and differentiation that are characterized implantation and gastrulation, where the three germ layers are defined and established and by differentiation of the germs layers into the respective organs and organ systems. The three germ layers are the endoderm, ectoderm and mesoderm.

Embryonic Stem Cell: As used herein, "embryonic stem cell" refers to any cell that is totipotent and derived from a developing embryo that has reached the developmental stage to have attached to the uterine wall. In this context embryonic stem cell and pre-embryonic stem cell are equivalent terms. Embryonic stem cell-like (ESC-like) cells are totipotent cells not directly isolated from an embryo. ESC-like cells can be derived from primordial sex cells that have been isolated and expanded.

Expanded: As used herein, "expanded" refers to a growing culture of cells that has increased in cell number from its original concentration.

Fetal Stem Cell: As used herein, "fetal stem cell" refers to a cell that is multipotent and derived from a developing multi-cellular fetus that is no longer in early or mid-stage organogenesis.

Germ Cell: As used herein, "germ cell" refers to a reproductive cell such as a spermatocyte or an oocyte, or a cell that will develop into a reproductive cell.

Germ Line Precursor Stem Cell: As used herein, "germline precursor stem cell" refers to a reproductive cell such as a precursor of a spermatogonial stem cells or an oocyte precursor stem cell.

Germ Line Stem Cell: As used herein, "germline stem cell" refers to a reproductive cell such as a spermatogonial stem cell (SSC) or an oocyte precursor stem cell.

Gonocyte: As used herein, "gonocyte" refers to fetal germ cells from 11-22 weeks of gestation that have not yet differentiated into spermatogonial stem cells and are mitotically quiescent and are no longer considered to be primordial germ cells.

Long-term culture: As used herein, "long-term culture" refers to the propagation of cells under controlled conditions for longer than at least two months or more than 10 passages. Preferably the long-term cultures are cultured for more than 4 months, more than 6 months or more than 1 year. Preferably the long-term cultures are passaged for more than 15 passages, more than 18 passages or more than 20 passages. The duration of the long-term cultures is highly dependent on the individual cells and there can be variability from cell line to cell line.

Maturation: As used herein, "maturation" refers to a process of coordinated biochemical steps leading toward a terminally differentiated cell type.

Multipotent: As used herein, "multipotent" refers to cells that can give rise to several other cell types, but those cell types are limited in number. An example of multipotent cells is hematopoietic cells - blood stem cells that can develop into several types of blood cells but cannot develop into brain cells.

Multipotent Adult Progenitor Cells: As used herein, "multipotent adult progenitor cells" refers to multipotent cells isolated from the bone marrow which have the potential to differentiate into cells of the ectoderm, mesoderm and endodermal lineages.

Pluripotent: As used herein, "pluripotent" refers to cells that can give rise to any cell type except the cells of the placenta or other supporting cells of the uterus.

Post-natal Stem Cell: As used herein, "post-natal stem cell" refers to any cell that is derived from a multi-cellular organism after birth.

Primordial Germ Cell: As used herein, "primordial germ cell" (PGC) refers to cells present in early embryogenesis that are destined to become germ cells.

Primordial Germ Line Sex Stem Cell: As used herein, "primordial germline sex stem cell", also referred to in short form as a "germline sex cell" abbreviated PGLSC, refers to a cell that is derived from adult male or female reproductive tissue, and which is able to generate Germ Line Stem Cells and their progeny as evidenced by its ability to repopulate reproductively sterile testicular or ovarian tissues after e.g. radiation or chemotherapy. Germ line sex cells can be quiescent or actively dividing in adult reproductive tissues.

Reprogramming: As used herein "reprogramming" refers to the resetting of the genetic program of a cell such that the cell exhibits pluripotency and has the potential to produce a fully developed organism. In addition this reprogramming gives the cell undergoing reprogramming characteristics that would normally not be expressed or found in the cell in its pre-programming state.

Selection" As used herein, "selection" refers to fluorescence-activated cell sorting, magnetic bead sorting or other means of collecting cells bearing a particular marker profile

Sex Cell: As used herein, "sex cell" refers to diploid or haploid cells derived from the mammalian male or female reproductive tissues. Representative examples of these cells include male gonocytes, female gonocytes, oogonia, type-A spermatogonia and Type-B spermatogonia.

Somatic Cell: As used herein, "somatic cell" refers to any tissue cell in the body except sex cells and their precursors.

Somatic Stem Cells: As used herein, "somatic stem cells" refers to diploid multipotent or pluripotent stem cells. Somatic stem cells are not totipotent stem cells.

Stem Cells: As used herein, "stem cells" refers to cells capable of self-renewal (the ability to go through numerous cycles of cell division while maintaining the undifferentiated state and being at least multipotent (the capacity to differentiate into more than one specialized cell type.

Substantially Pure: As used herein, "substantially pure" refers to a population of cells wherein greater than 75%, greater than 85%, greater than 90%, greater than 95%, greater than 98% or greater than 99% of the cells have the desired characteristic(s).

Totipotent: As used herein, "totipotent" refers to cells that contain all the genetic information needed to create all the cells of the body plus the placenta. Human cells have the capacity to be totipotent only during the first few divisions of a fertilized egg.

### DETAILED DESCRIPTION OF THE INVENTION

The present disclosure provides methods for the isolation, characterization and expansion of germline stem cells that are biologically useful and multipotent and have telomerase activity that compares favorably with the telomerase activity of undamaged, prenatal or embryonic stem cells. Methods for identification and isolation of germline stem cells are provided. The germline stem cell populations and cell lines derived therefrom express different spectra of embryonic and germ stem cell markers

One population of germline stem cells, the Oct-4 positive/c-Kit positive subset of germline stem cells (germline precursor cells), when isolated from either neonatal or adult mouse testes and cultured in a mixture of growth factors, was able to generate cell lines expressing both pluripotent embryonic stem (ES) cell markers (Oct-4, Nanog, Sox2, Rex-1, Dppa5, SSEA-1, alkaline phosphatase) and high telomerase activity. In contrast, the c-Kit negative subset of germline stem cells (germline sex cells) was capable of repopulating a mouse testes rendered sterile by treatment with the chemotherapeutic drug busulfan. The Oct-4 positive/c-Kit positive germline precursor cells differentiate into multiple lineages including beating cardiomyocytes, neural cells and chondrocytes following induced differentiation protocols. This data clearly show the existence of distinct populations within germline stem cells from which only the germline precursor c-Kit positive/Oct-4 positive cells can generate multipotent cell lines with ESC-like properties.

Furthermore, distinct subpopulations of germline stem cells are provided that, when maintained under defined culture conditions, generate cell lines that have pluripotent characteristics but, unlike embryonic stem cells, do not form teratomas. These highly useful properties provide a valuable potential source of high quality stem cells for therapeutic applications.

There is strong evidence that stem cells can be differentiated *in vitro* into tissue-specific cell types. In addition, pluripotent stem cells have shown the potential to become multipotent stem cells following engraftment into tissues. Therefore, the present disclosure provides methods and compositions for providing functional immunocompatible pluripotent and multipotent stem cells, as well as, derivative tissue cells for uses in cellular regenerative, restorative, reparative and rejuvenative therapies.

In one embodiment, biologically useful multipotent or pluripotent cells are provided from human fetal gonocytes which have minimal oxidative damage and telomerase activity that compares favorably with that of embryonic stem cells. Moreover the cultured fetal gonocytes disclosed herein are immunologically privileged and are suitable for therapeutic applications. Fetal gonocytes arise from primordial sex cells, residing in the lining of the seminiferous tubules of the testes (in males) and represented a differentiation state between primordial sex cells and spermatogonial stem cells (in males) or oogonia (in females). Fetal gonocytes are isolated from fetuses between 11 and 22 weeks of gestation and are undamaged by to the effects of aging and cell division. Thus, fetal gonocytes possess genomes in a nearly physiologically prime state.

Embodiments disclosed herein provide methods for identifying, isolating and preparing primordial germline stem cells. The instant methods involve collecting c-kit negative cells from a testicular cell sample. In alternative embodiments, the methods involve collecting c-Kit negative, α-integrin positive and Thy-1 positive cells from a testicular cell sample. The c-Kit+/α6-integrin+/Thy-1+ cells are capable of repopulating a sterile (non-reproductive) testes to produce spermatogonial stem cells (SSC), spermatogonia and sperm. However, the c-Kit+/α6-integrin+/Thy-1+ germline stem cells isolated from mice are not capable of giving rise to multipotent stem cells, or to lineage committed tissue cells such as neuron, cardiomyocytes, chondrocytes, osteocytes and endothelial cells.

Embodiments provide methods for identifying, isolating and preparing germline precursor cells. The instant methods involve collecting c-Kit positive cells from a testicular cell sample. In alternative embodiments, the methods involve collecting c-Kit positive and Oct-4 positive cells from a testicular cell sample. The c-Kit+/Oct-4+ cells are not capable of repopulating a sterile (non-reproductive) testes to produce SSC, spematogonia and sperm. However, the c-Kit+/Oct-4+ germline precursor cells are capable of giving rise to multipotent stem cells, as well as, to lineage committed tissue cells such as neuron, cardiomyocytes, chondrocytes, osteocytes and endothelial cells.

The pluripotent stem cells made in accordance with the teachings of the present invention can be used for therapeutic purposes as is, they can be propogated in long-term culture, cryopreserved for future use or they can be differentiated into multipotent or tissue specific cell lineages.

Embodiments of the present invention provide methods for further maturing or differentiating pluripotent and multipotent germline stem cells for uses in cellular regenerative/reparative therapy. In addition the maturation and differentiation process provides therapeutic cells that can be used to treat or replace damaged cells in pre- and post-natal organs.

The germline stem cells made in accordance with the teachings of the present invention are useful in a wide range of therapeutic applications for cellular regenerative/reparative therapy. For example, and not intended as a limitation, the germline stem cells of the present invention can be used to replenish resident multipotent stem cells in the tissues of animals whose natural stem cells have been depleted due to age or ablation therapy such as cancer radiotherapy and chemotherapy. In another non-limiting example, germline stem cells can be differentiated to dopamine-producing cells and used for treatment of Parkinson's disease. In another non-limiting example, the germline stem cells of the present invention are useful in organ regeneration and tissue repair. In one embodiment of the present invention, germline stem cells can be used to reinvigorate damaged muscle tissue including dystrophic muscles and muscles damaged by ischemic events such as myocardial infarcts. In another embodiment of the present invention, the germline stem cells disclosed herein can be used to ameliorate scarring in animals, including humans, following a traumatic injury or surgery. In this embodiment, the germline stem cells of the present invention are administered systemically, such as intravenously, and migrate to the site of the freshly traumatized tissue recruited by circulating cytokines secreted by the damaged cells. In another embodiment of the present invention, the germline stem cells can be administered locally to a treatment site in need or repair or regeneration.

The germline cells of a mature or developing individual contain the genetic material that may be passed to a child. For example, sex cells, such as the sperm or the egg, are part of the germline. So are the precursors of sex cells and all the way back to the zygote, the cell from which the individual developed.

Cells that are not in the germline are somatic cells. For example, all cells of the mammalian liver are somatic. If there is a mutation or other genetic change in the germline, it can be passed to offspring, but a change in a somatic cell will not be.

Germline cells are immortal, in the sense that they can reproduce indefinitely. This function is enabled, in part, by telomerase. Telomerase is dedicated to lengthening the DNA primer of the chromosome, allowing for unending duplication. Somatic cells, by comparison, can only divide around 30-50 times, as they do not contain telomerases. There is an inverse correlation between telomerase levels and replication capacity. In one embodiment, the instant germline cells express a high level of telomerase. A high level of telomerase is defined as an amount of telomerase that is 20-100% of the amount of telomerase as embryonic stem cells.

The germline cells disclosed herein are isolated from gonadal tissues from mammals including but not limited to, rodents, domesticated animals, dogs, cats and primates. The term "primates" includes, but is not limited to, humans.

The germline cells are isolated based on their expression, or lack of expression of a variety of germline, embryonic and pluripotent cell markers. Germline cell markers include, but are not limited to, VASA, promyelocytic leukemia zinc factor (PLZF), glial derived neurotrophic factor receptor α1 (GFR- α1), α6-integrin, Thy-1, SSEA-4, CD9, CD90, CD49f, *Dolichos biflourus* agglutinin (DBA), neural cell adhesion molecule (NCAM), germ cell nuclear antigen 1 (GCNA1) and DAZL.

Pluripotent cell markers include, but are not limited to, Oct-4 (POU5F1), Nanog, alkaline phosphatase, TRA1-60, TRA1-81.

Furthermore, germline cells can also be isolated based on the express of germline and/or pluripotent stem cell genes. Germline stem cell genes include, but are not limited to, telomerase, VASA, c-RET, Nanog and GFR-α1. Pluripotent cell genes include, but are not limited to, Oct-4, Nanog, Dppa-5, Sox2, alkaline phosphatase and Crypto.

Additional embodiments presented herein include the long-term culture of certain populations of germline cells such that long-term multipotent or pluripotent cells lines are generated. These cells lines can be used as a source of cells for differentiation into tissue-specific lineages. Long term culture of the instant germ line cells comprises the steps of isolating a substantially pure population of the desired germline cell based on expression, or lack of expression, of germline and/or pluripotent cell markers and germ line and/or pluripotent genes; culturing the cells in growth medium as disclosed in the Examples section which allow continued cell division while maintaining an undifferentiated multipotent or pluripotent state. The long-term cultures described herein can be cryopreserved for future uses.

In certain embodiments, isolated, substantially pure populations of germline cells or germline cell lines can be used for therapeutic applications in regenerative medicine. According to the teachings herein, certain subpopulations of germline cells disclosed herein are capable of generating long-term cultures of multipotent or pluripotent cell lines and are suitable as a source of differentiated cells for regenerative therapies. Other subpopulations of germline cells disclosed herein are capable of forming more differentiated germline cells such as spermatogonial stem cells, spermatogonia, sperm, oogonia and oocytes. This second subpopulation of cells is capable of re-populating a sterile reproductive organ *in vivo.*

Transplanting the isolated substantially pure population of germline stem cells into the recipient can be accomplished by direct injection using standard injection means known to persons of ordinary skill in the art. In another embodiment, support cells, such as Leydig or Sertoli cells that provide hormonal stimulus to spermatogonial differentiation, can be transferred to a recipient testis along with the germline stem cells. These transferred support cells can be unmodified, or, alternatively, can be genetically modified. These transferred support cells can be autologous or heterologous to either the donor or recipient testis. A preferred concentration of cells in the transfer fluid can easily be established by simple experimentation, but will likely be within the range of about 10³ - 10¹⁰ cells per ml. The injection means can be introduced into the vasa efferentia, the rete testis or the seminiferous tubules, optionally with the aid of a pump to control pressure and/or volume, or this delivery can be done manually. A suitable dyestuff or bubbles (less than 1 mm in diameter) can also be incorporated into the carrier fluid for easy identification of satisfactory delivery of the transplanted germline stem cells to testes. Similar methods can be used to delivery germline stem cells of female origin to the ovaries.

Suitable cell transplant vehicles are known to persons of ordinary skill in the art and can include molecules such as serum albumin, cholesterol and/or lecithin, selenium and inorganic salts as well as serum components and/or growth factors and/or cytokines. Typically the cell transplant vehicle has a pH which is roughly physiologic, i.e. 7.0 to 7.6.

The germline cells generated as disclosed herein can be used to treat a variety of disease or injury indications depending on the need as a cellular composition. The route of delivery is determined by the disease and the site where treatment is required. For topical application, it may prove desirable to apply the cellular compositions at the local site (such as by placing a needle into the tissue at that site or by placing a timed-release implant or patch); while in a more acute disease clinical setting it may prove desirable to administer the instant cellular compositions systemically or locally into an organ. For other indications the instant cellular compositions may be delivered by administration routes including, but not limited to, intravenous, intraperitoneal, intramuscular, intracerebroventricularly, subcutaneous and intradermal injection, as well as, by intranasal and intrabronchial instillation, transdermal delivery, or gastrointestinal delivery. The preferred therapeutic cellular compositions for inocula and dosage will vary with the clinical indication. The inocula may typically be prepared from a frozen cell preparation such as by thawing the cells and suspending them in a physiologically acceptable diluent such as saline, phosphate-buffered saline or tissue culture medium. Some variation in dosage will necessarily occur depending upon the condition of the patient being treated, and the physician will, in any event, determine the appropriate dose for the individual patient.

The instant cellular compositions may to be administered alone or in combination with one or more pharmaceutically acceptable carriers, in either single or multiple doses. Suitable pharmaceutical carriers may include inert biodelivery gels or biodegradable semi-solid matrices, as well as diluents or fillers, sterile aqueous solutions and various nontoxic solvents. The subject pharmaceutically acceptable carriers generally perform three functions:, (1) to maintain and preserve the cells in the instant cellular composition; (2) to retain the cells at a tissue site in need of regeneration, restoration or rejuvenation; and (3) to improve the ease of handling of the instant composition by a practitioner, such as, but not limited to, improving the properties of an injectable composition or the handling of a surgical implant. The pharmaceutical compositions formed by combining an instant cellular composition with a pharmaceutically acceptable carrier may be administered in a variety of dosage forms such as injectable solutions, and the like. The subject pharmaceutical carriers can, if desired, contain additional ingredients such as binders, excipients, and the like. The subject aqueous solution is preferably suitably buffered if necessary and the liquid diluent first rendered isotonic with sufficient saline or glucose. Such aqueous solutions of instant cellular composition may be particularly suitable for intravenous, intramuscular, subcutaneous, and intraperitoneal injection. The subject sterile aqueous media employed are obtainable by standard techniques well known to those skilled in the art. For use in one or more of the instant methods, it may prove desirable to stabilize a instant cellular composition, such as, but not limited to, increasing shelf life, viability and efficacy. Methods for preserving, storing and shipping frozen cells in preservative solutions are known in the art. Improving the shelf-life stability of cell compositions, e.g., at room temperature or 4°C, may be accomplished by adding excipients such as: a) hydrophobic agents (e.g., glycerol); b) non-linked sugars (e.g., sucrose, mannose, sorbitol, rhamnose, xylose); c) non-linked complex carbohydrates (e.g., lactose); and/or d) bacteriostatic agents or antibiotics.

The preferred compositions for inocula and dosage for use in the instant methods will vary with the clinical indication. The inocula may typically be prepared from a concentrated cell solution by the practicing physician at the time of treatment, such as by thawing and then diluting a concentrated frozen cell suspension in a storage solution into a physiologically acceptable diluent such as phosphate-buffered saline or tissue culture medium. Some variation in dosage will necessarily occur depending upon the condition of the patient being treated, and the physician will, in any event, determine the appropriate dose for the individual patient.

The effective amount of the instant cellular composition per unit dose depends, among other things, on the body weight, physiology, and chosen inoculation regimen. A unit dose of the instant cellular composition refers to the number of cells in the subject suspension. Generally, the number of cells administered to a subject in need thereof according to the practice of the invention will be in the range of about 10⁵/site to about 10⁹/site. Single unit dosage forms and multi-use dosage forms are considered within the scope of the present disclosure.

In alternative embodiments, the present disclosure provides different routes for delivery of the instant cellular compositions as may be suitable for use in the different disease states and sites where treatment is required. For topical, intrathecal, intramuscular or intra-rectal application it may prove desirable to apply the subject cells in a cell-preservative salve, ointment or emollient pharmaceutical composition at the local site, or to place an impregnated bandage or a dermal timed-release lipid-soluble patch. For intra-rectal application it may prove desirable to apply the instant cellular compositions in a suppository. In other embodiments, for pulmonary airway restoration, regeneration and rejuvenation it may prove desirable to administer the instant cellular compositions by intranasal or intrabronchial instillation (e.g., as pharmaceutical compositions suitable for use in a nebulizer). Also contemplated are suppositories for urethral and vaginal use in regenerative medical treatments of infertility and the like. In one embodiment, the subject pharmaceutical compositions are administered via suppository taking advantage of the migratory capacity of instant cells, e.g., migration between the cells in the epithelial lining cells in the rectum, into the interstitial tissues and into the blood stream in a timed-release type manner. Where conventional methods of administration may be ineffective in certain patients and a more continuous regenerative, restorative or rejuvenative source of therapy is desired, administering the instant cellular compositions in a continuous from such as via an implantable mini-pump (such as used for delivery of insulin in patients with Type 1 insulin-dependent diabetes mellitus). Alternatively, in other cases it may desirable to deliver the instant cellular compositions over a longer period of time, such as by infusion.

In certain alternative embodiments, the method may involve administration of an intravenous bolus injection or perfusion of the instant cellular compositions, or may involve administration during (or after) surgery, or a prophylactic administration. In certain other embodiments, the instant administration may involve a combination therapy such as the instant cellular composition and a second drug including, but not limited to, an anticoagulant, anti-infective or anti-hypertensive agent.

In summary, the present germlines may be used as a cellular replacement therapy in different disease/trauma states including, but not limited to, treatments of Parkinson's disease, multiple sclerosis, amyotrophic lateral sclerosis (ALS), Alzheimer's disease, cystic fibrosis, fibromyalgia, diabetes, non-union bone fractures, cosmetic and reconstructive surgery for skin, cartilage and bone, myocardial infarct, stroke, spinal cord injury, traumatic injury, infertility, and restoring, regenerating and rejuvenating damaged and aged tissues.

### EXAMPLES

The following examples are meant to illustrate one or more embodiments are not limited to that which is described below.

### Example 1

### Isolation of human fetal gonocytes

Aborted fetuses from 11-22 weeks gestation were donated by approved clinics and maintained under sterile conditions. Gonads were decapsulated and one gonad was placed in 2.5 mL of phosphate buffered saline (PBS) and minced with sterile scissors. The minced gonadal tissue was incubated with 2.5 mL of a 2x collagenase solution (2 mg collagenase and 20 unites of DNase I per milliliter of DPBS(-)) for 30 minutes at 37°C in a shaking water bath. During the dissociation period the sample is triturated several times to promote dissociation of the tissue. After dissociation, 1 mL of fetal bovine serum is added to deactivate the collagenase and the sample is triturated once more. The sample is then centrifuged at 400xg for 5 minutes and the cells resuspended at 5-7.5 x 10⁵ per mL in PM-10™ medium plus growth factors as disclosed in co-pending U.S. Patent Application number 11/694,687, filed on March 30, 2007 which is incorporated by reference herein for all it contains regarding culture of stem cells. PM-10™ medium is disclosed in United States patent application No. 11/488,362 filed July 17, 2006, which is incorporated herein for all it contains regarding growth-promoting factors needed for cell propagation.

### Example 2

### Long term culture of human fetal gonocytes

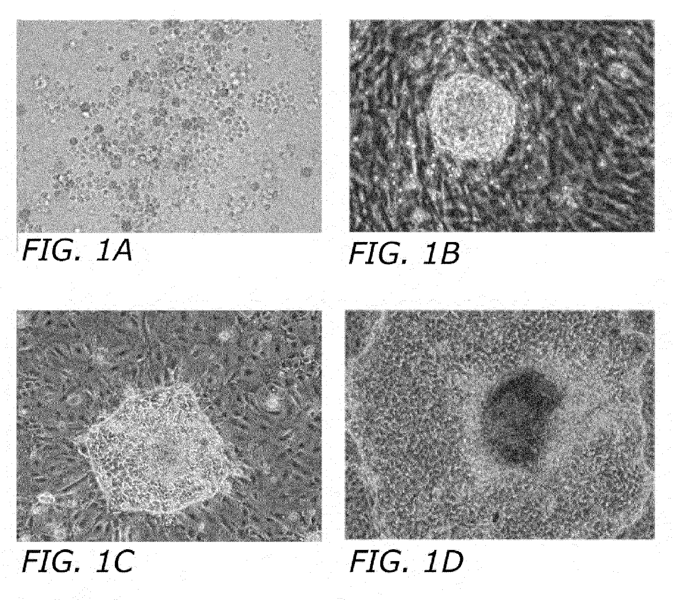

The isolated fetal gonocytes showed a high level of alkaline phosphatase staining at the onset of culture (Figure 1 A). Gonocytes were then cultured on STO/c (feeder cells) plates or under feeder-free conditions for colony formation. Alternatively, gonocytes can be cultured in PM-10™ medium plus growth factors on gelatin-coated plates prior to culturing on STO feeder cells.

After two weeks in culture, the human fetal gonocytes formed 'mushroom' colonies (Figure 1 B). At this time the mushroom colonies were transferred to fresh STO plates. After 1-3 passages, the mushroom colonies formed outgrowths indicating that the colonies are expanding (Figure 1C). These outgrowths continued to grow on the STO feeders and form flat colonies resembling human embryonic stem cell colonies (Figure 1 D).

The cultured gonocytes were passaged onto fresh STO cells every two weeks in serum-free culture medium (PM-10™ + growth factors) for further expansion.

### Example 3

### Expression of pluripotent markers by cultured human fetal gonocytes

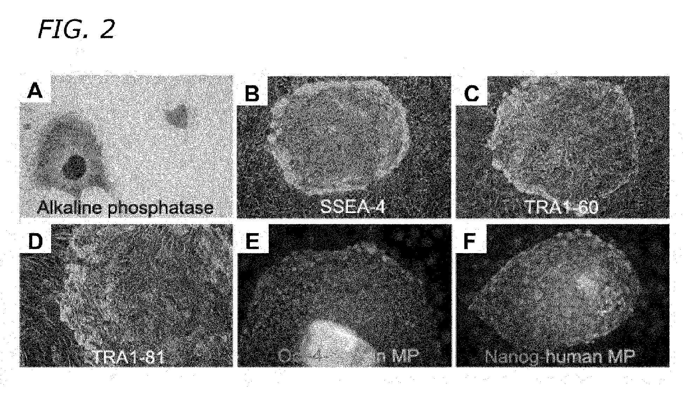

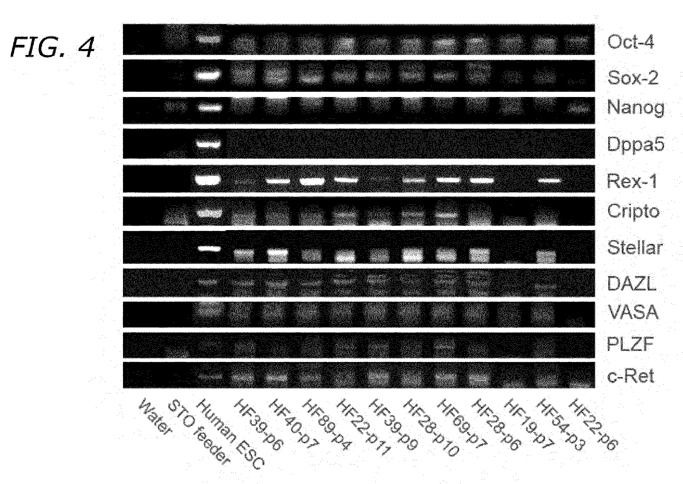

Long-term culture in the presence of growth factors changes the gene expression profile of the gonocytes, therefore cultured gonocytes express markers that normally would be expressed in pluripotent stem cells (Figures 2 and 4) including alkaline phosphatase (Figure 2A), SSEA-4 (Figure 2B), TRA1-60 (Figure 2C), TRA1-81 (Figure 2D), Oct-4 and human mitochondrial protein (MP, Figure 2E), and Nanog and human MP (Figure 2F). Human MP is used as a human-specific marker.

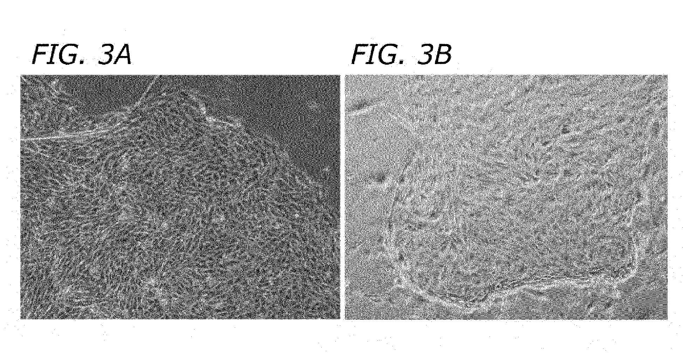

Figure 3 depicts two colonies of long-term cultured gononocyes (Figure 3A: HF-89-p2 colony; Figure 3B: HF-22-p7 colony) that have been cultured under serum and feeder-free conditions such that they are not exposed to animal products in the reprogramming or culture process and are not contaminated with immunogenic non-human molecules.

Figure 4 depicts expression of a wide range of pluripotency-associated markers such as Oct-4, Nanog, Sox2 and Rex1 in expanded gonocytes. The expression of these markers suggests that these cells have therapeutic potential.

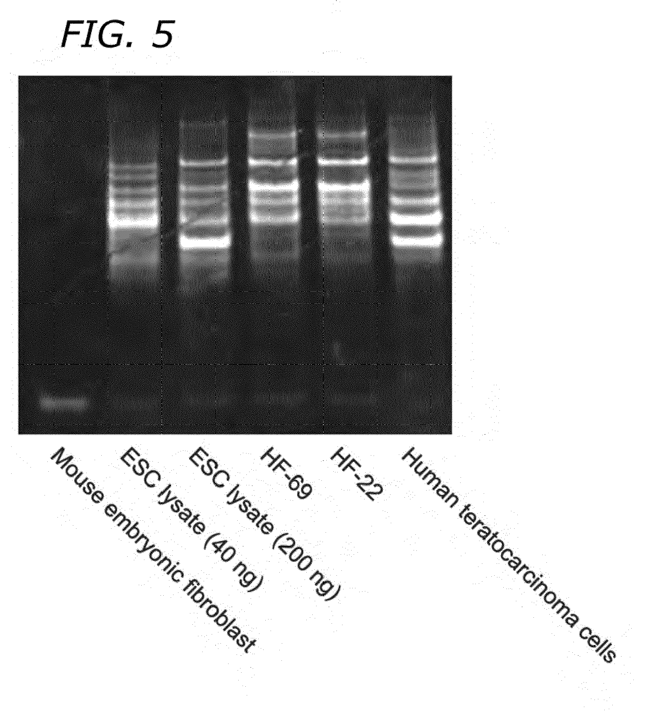

Figure 5 depicts telomerase activity in long-term cultures of human gonocytes. Telomerase activity is found in cells that are not actively aging and have the ability to self renew indefinitely.

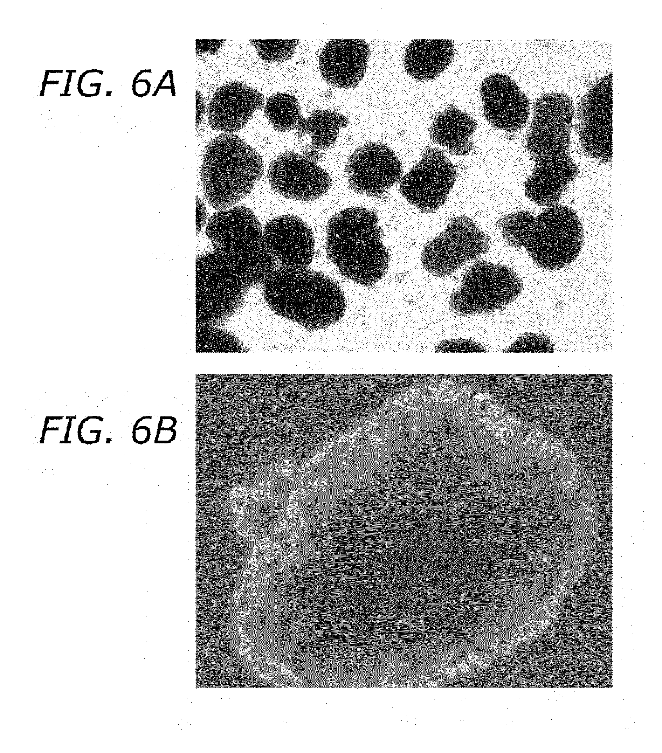

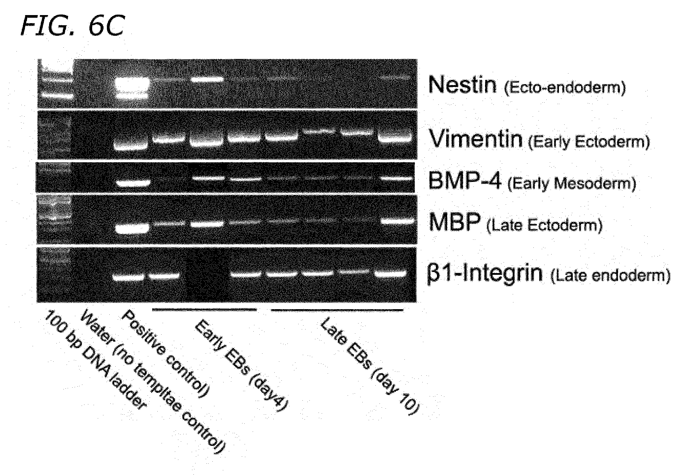

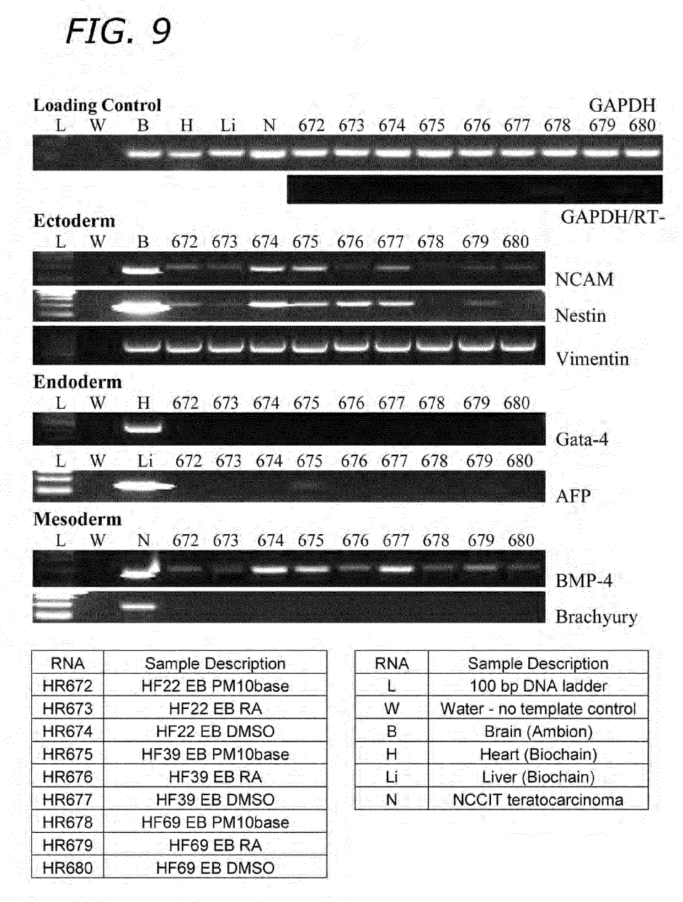

Figures 6 and 9 demonstrate that cultured gonocytes are pluripotent. In these Figures, the expanded gonocytes are aggregated into embryoid bodies (Figures 6A and 6B) and the embryoid bodies were screened for markers and/or genes associated with cell types derived from one or all of the three germ layers (endoderm, mesoderm and ectoderm) (Figure 6C and Figure 9).

### Example 4

### Differentiation of long-term cultured human fetal gonocytes into tissue specific cells

Human fetal gonocytes from Example 2 can be differentiated into a number of different cell types.

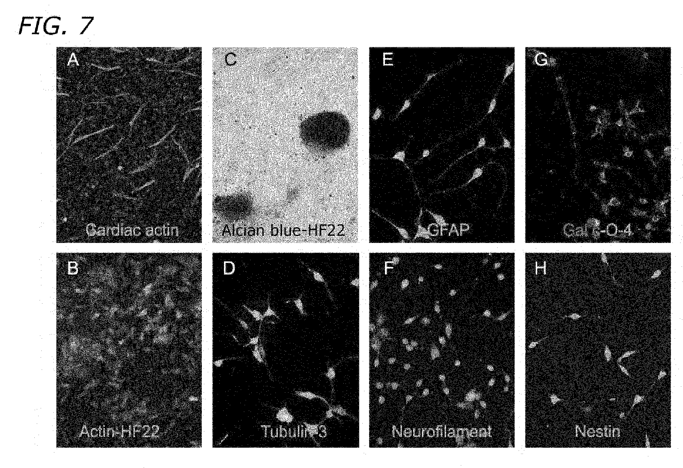

To generate cardiac cells, the cells were then plated out onto 6 cm tissue culture dishes previously treated with 0.1% gelatin and cultured for 30 days in a standard CO₂ cell culture incubator at a temperature range of 34°C to 42°C in media containing aza-2'-deoxycytidine (AZA) which was changed every other day for 30 days. Aza-2'-deoxycytidine was added to PM-10™ at a concentration of approximately 0.1 µM to 9.0 µM. The AZA-containing media described herein is an exemplary embodiment of the differentiation method. It will be understood by persons skilled in the art that the concentrations of the components of the AZA-containing media can vary and still achieve the intended results. After approximately 35 days in culture with AZA-containing media, the human fetal gonocytes have differentiated and stain positive for the cardiac marker cardiac alpha actin (Figure 7A).

For generation of astroglial cells, isolated cultured human gonocytes from Example 2 were cultured in PM-SHH media (PM-10 medium containing 200 ng/ml sonic hedgehog protein) at 34°C for 24 days before they were assayed for expression of the glial cell marker, glial fibrillary acidic protein (GFAP) by fluorescence immunocytochemistry using a monoclonal anti-GFAP antibody (Figure 7E). The human fetal gonocytes were directed to differentiate into neural cells, including GFAP⁺ glial cells, *in vitro.* Since there is no known evidence for the existence of glial cells in the fetal testis, the GFAP+ glial cells differentiated from gonadal cells.

In addition to astroglial differentiation, human fetal gonocytes were also be differentiated into neuronal cells in the PM-SHH medium as evidenced by the expression of neurofilament (Figure 7F).

Oligodendroglial cells were differentiated from cultured human fetal gonocytes as follows. For determining galactocerebroside-C (Gal-C) expression (Figure 7G), human fetal gonocytes were plated onto fibronectin-coated coverslips in DMEM low glucose with GlutaMax™ (Invitrogen) with 40% MCDB-201 media and supplemented with ITS+LA-BSA (10 µg/mL insulin from bovine pancreas, 5.5 µg/mL human transferrin (substantially iron-free), 5 ng/mL sodium selenite, 0.5 mg/mL bovine serum albumin and 4.7 µg/mL linoleic acid), 1 nM dexamethasone, 100 µM ascorbic acid and treated with 200 ng/mL sonic hedgehog (SHH) and 100 ng/mL FGF-8 for 3 days and then switched to N2 supplements with 20 ng/mL BDNF. Control cells were cultured without the addition of SHH and FGF-8 on 0.2% gelatin-coated coverslips in DMEM low glucose with GlutaMax™ with 2% FBS, MCDB-201 40% and supplemented with ITS+LA-BSA, 1 nM dexamethasone and 100 µM ascorbic acid.

Human fetal gonogytes can also be differentiated into chondrocytes, osteocytes and hepatocytes. Figure 7B depicts the directional differentiation of human fetal germ cells into smooth muscle cells that express actin. Figure 7C depicts the directional differentiation of human fetal germ cells into chondrocytes, as evidenced by staining for alcian blue, which targets glycosaminoglycans found in chondrocytes. Figure 7D depicts human fetal germ cells that have been directionally differentiated to neurons as evidenced by expression of beta tubulin III. Figure 7H depicts human fetal germ cells that have been directionally differentiated to neuroprogenitor cells expressing nestin. These neuroprogenitor cells can be further differentiated into other more terminally differentiated cells of the neuronal lineage.

For chondrocyte differentiation, human fetal gonocytes were plated onto 0.2% gelatin-coated plates in SingleQuots^{®} medium (Cambrex) with 20% FBS and 10 ng/mL TGF-3β added to the medium just before the medium change. Control cells were cultured in DMEM low glucose with L-glutamine and penicillin/streptomycin supplemented with 20% FBS.

For osteocyte differentiation, human fetal gonocytes were plated onto 0.2% gelatin-coated plates in DMEM low glucose with L-glutamine and penicillin/streptomycin supplemented with 20% FBS and the addition of 100 nM dexamethasone, 0.25 mM ascorbic acid and 10 mM B-glycerolphosphate. The medium was changed every 2-3 days. Control cells were plated on 0.2% gelatin-coated plates in DMEM low glucose with L-glutamine and penicillin/streptomycin supplemented with 20% FBS.

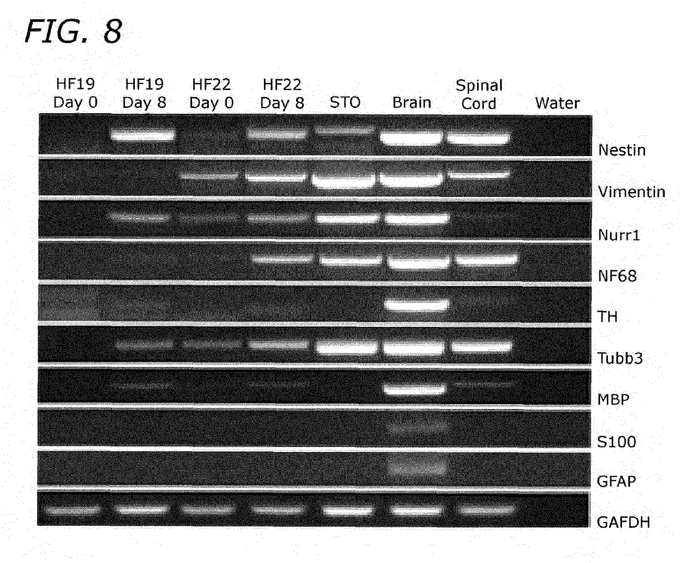

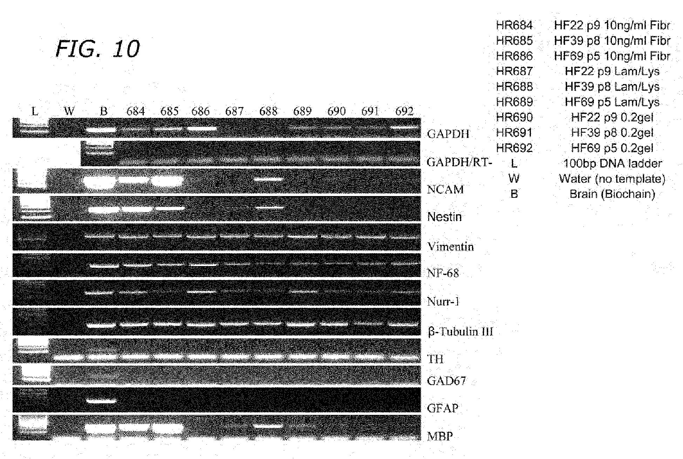

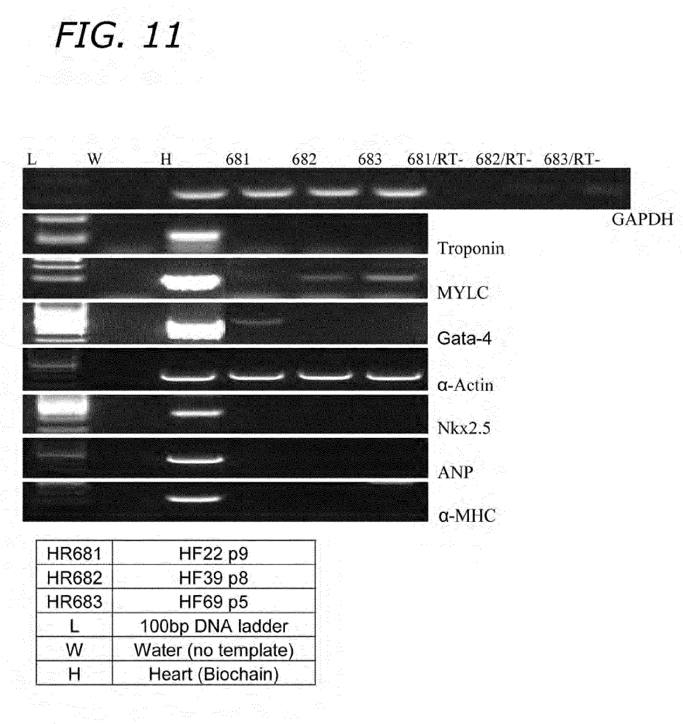

Figures 8 and 10 depict the directional differentiation of gonocytes into cells of the neuronal lineage by RT-PCR analysis of expressed genes in the differentiated cells. Figure 11 depicts the directional differentiation of gonocytes into cell types of the muscle lineage by RT-PCR analysis of genes expressed by the differentiated cells.

### Example 5

### Identification and Isolation of Distinct Populations of Male Germ Line Stem Cells

Generation of pluripotent cell lines which can be safely used in regenerative medicine has a great potential impact in cell replacement therapy. In this regard, embryonic stem cells (ESC) have been considered as potential cell sources because they can be propagated indefinitely and can be differentiated into phenotypes of all three germ layers. Adult stem cells originating from tissues have also been considered as alternative sources for cell-based therapy particularly since they do not form teratomas after transplantation, and they maintain the ability to differentiate into phenotypes of the same tissue lineage. Adult stem cells can also be induced to trans-differentiate into cell types of different lineages, or reprogrammed to become pluripotent stem cells for possible clinical applications. Among all adult stem cells, only germline stem cells (GSC) retain the ability to transmit pristine genetic information to offspring. Several lines of evidence suggest that GSCs acquire pluripotentiality through reprogramming processes that occur during normal development. Therefore, GSCs are theoretically a model for generation of pluripotent or multipotent adult stem cell lines.

Recently, multipotent germ cell lines have reportedly been generated from neonatal and adult mouse testes. These cell lines appear to exhibit similar molecular and functional characteristics to ES cells and generate teratomas when injected into immune compromised mice. However, cell lines generated in different laboratories showed differences in their ability to function as germ stem cells. For example, cells generated by Kanatsu-Shinohara et al. (Cell 119:1001-1012, 2004) did not show spermatogonial stem cell (SSC) properties while those generated by Guan et al. (Nature 440:1199-1203, 2006) and Seandel et al. (Nature doi10.1038/nature06129, 2007) repopulate recipient testes indicating they retained their SSC functionality. Moreover, these studies did not conclusively show whether the SSCs or a different subpopulation among the germline stem cells were the source of the multipotent germ cell lines. To unambiguously identify and track germline stem cell in culture, a transgenic mouse model expressing the green fluorescence protein (GFP) driven by a germline-specific Oct-4 promoter was used. Octamer-binding transcription factor-3/4 (Oct-4) was originally identified as an embryonic stem cell and germline-specific marker. The expression of Oct-4 is regulated by a proximal promoter, a germ specific distal enhancer and a retinoic acid responsive element. At gastrulation, Oct-4 expression is down regulated and thereafter is maintained only in primordial germ cells. Primordial germ cells (PGC), of both males and females, continue to express Oct-4 as they proliferate and migrate to the genital ridges. In the male, expression in germ cells persists throughout fetal development, and is maintained post-natally in proliferating gonocytes, prospermatogonia and in undifferentiated spermatogonia.

Using this model, pure populations of germline stem cells were isolated by sorting the Oct-4-GFP germ cells. Oct-4 positive germ cells were then subdivided based on the expression of c-Kit receptor molecule. C-Kit, a tyrosine kinase receptor, and its ligand stem cell factor (SCF; also known as Kit ligand or Steel factor), are key regulators of PGC growth and survival. C-Kit is expressed in PGCs from their initial segregation to their arrival at the genital ridge. In postnatal mouse testes, it has been reported that c-Kit can be used as a marker for differentiation of undifferentiated and differentiating type A spermatogonia. Combinations of Oct-4 and c-Kit allow the isolation of two distinct populations in germline stem cells: one containing more primitive germ cells or germline progenitors (Oct-4+/ckit+) and other contains germline stem cells destined to be SSCs (Oct-4+/c-Kit-) and with the ability to regenerate a sterile testes. The molecular and phenotypic characteristics of these cells were analyzed both before and after culture and compared their ability to generate multipotent cell lines under a defined culture condition with a mixture of growth factors. In addition, the functionality of these subpopulations and their descendent mGC lines to repopulate recipient testes was evaluated using spermatogonial stem cell transplantation technique.

Male germline stem cells (GSC) maintain spermatogenesis by self renewal of spermatogonial stem cells (SSC) and generation of spermatogonia committed to differentiation. Under certain *in vitro* conditions, GSC from both neonatal and adult mouse testis can reportedly generate multipotent germ cell (mGC) lines that have characteristics and differentiation potential similar to ESC. However, mGCs generated in different laboratories showed different germ cell characteristics; for example, some retain their SSC properties and some have lost it completely. Thus, the possibility remains that the derivative multipotent germ cell lines may have been derived from different subpopulations of germline stem cells resident within the mouse testes. To investigate this question, a transgenic mouse model expressing GFP under control of a germ cell-specific Oct-4 promoter was used. Two distinct populations were found among the germline stem cells with regard to their expression of transcription factor Oct-4 and c-Kit receptor. Only the Oct-4+/c-Kit+ subset of mouse germline stem cells, when isolated from either neonatal or adult testes and cultured in a complex mixture of growth factors, generate cell lines that express pluripotent ES markers i.e. Oct-4, Nanog, Sox2, Rex-1, Dppa5, SSEA-1, alkaline phosphatase, exhibited high telomerase activity and differentiated into multiple lineages including beating cardiomyocytes, neural cells and chondrocytes following induced differentiation protocols. This data clearly show the existence of distinct populations within germline stem cells from which only the germline progenitors can generate multipotent cell lines with some pluripotent characteristics.

Materials and Methods appear at the end of this example.

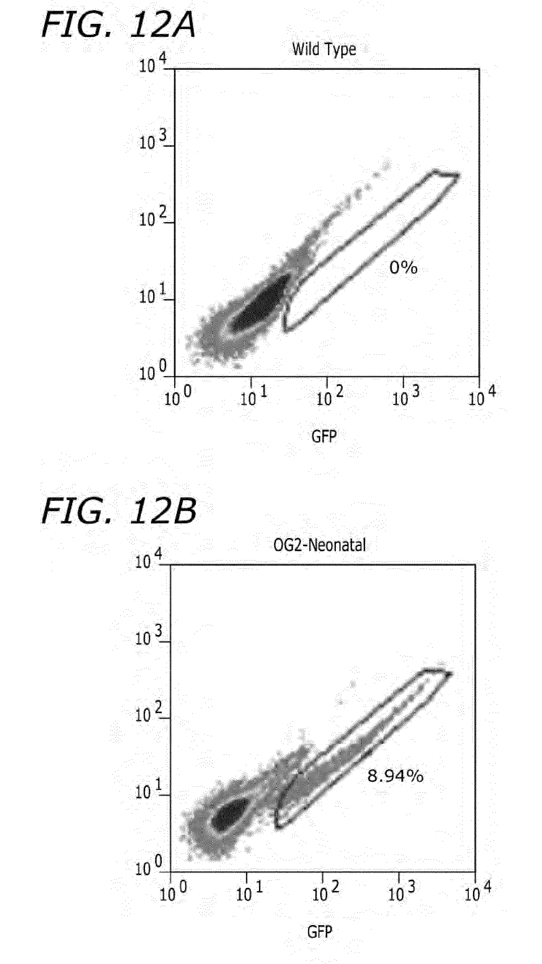

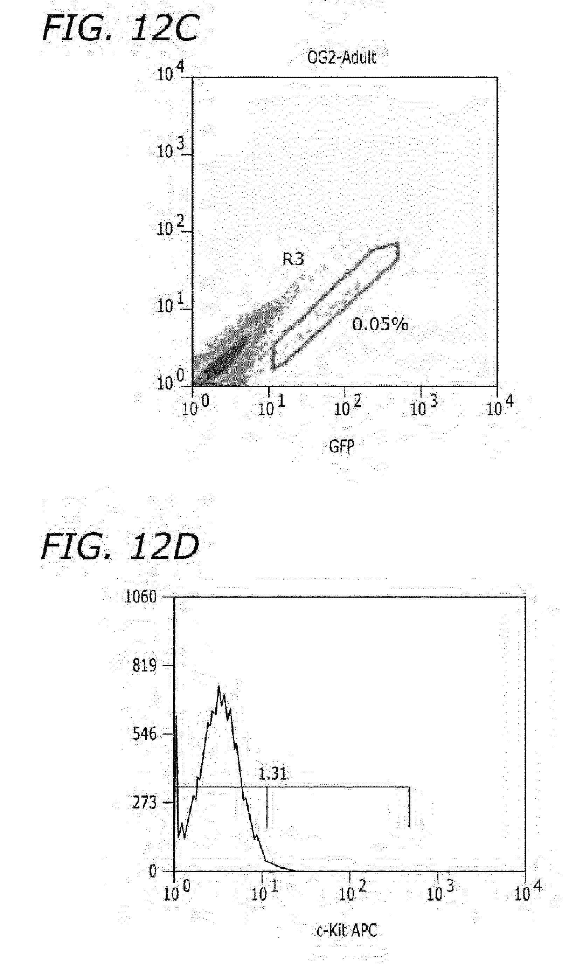

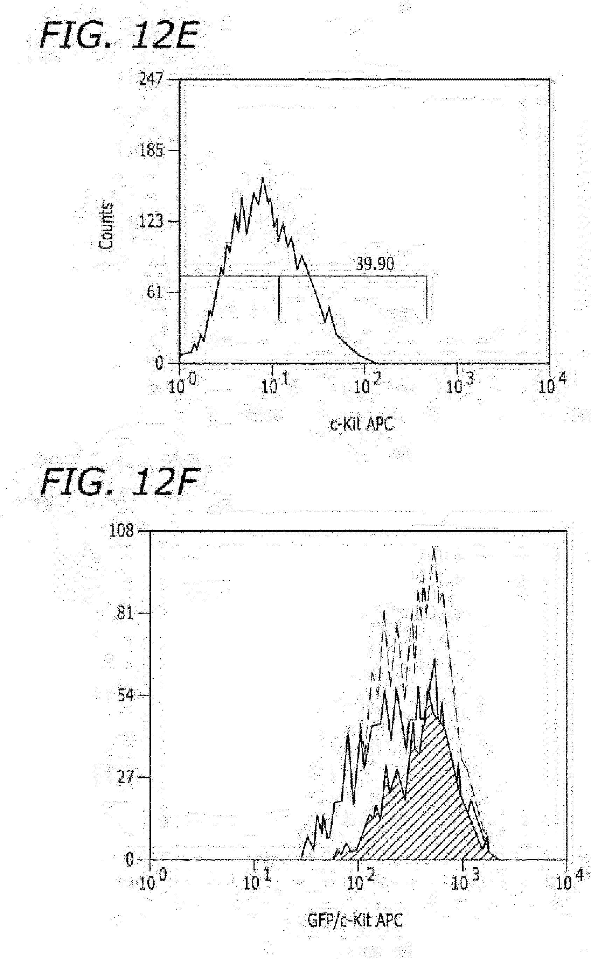

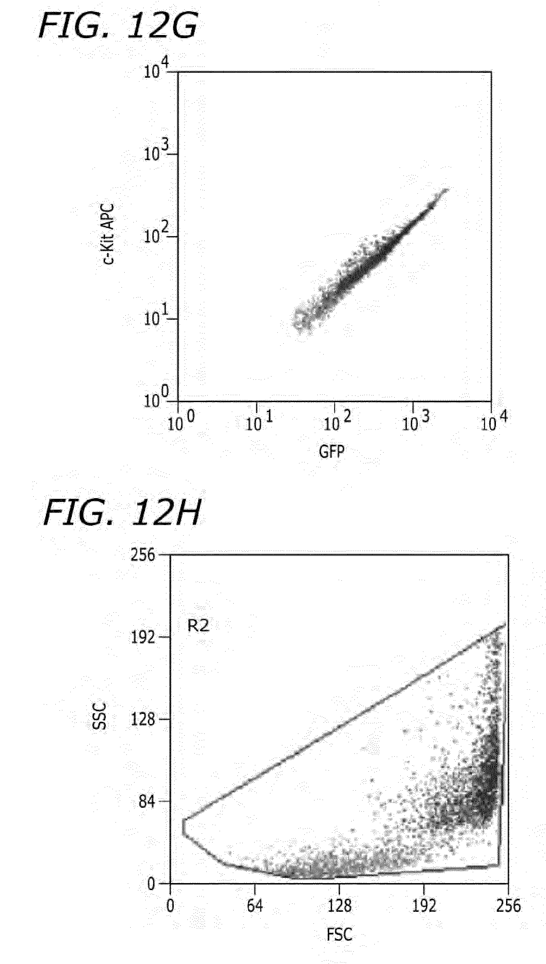

For enrichment of germline stem cells, both neonatal and adult testicular tissues were cultured on gelatin-coated culture dishes for 2 hr for differential adhesion to remove somatic cells but not germ cells. After differential adhesion, cell suspensions containing GFP positive cells (4-10% in the neonates; 0.01-0.05% in the adults) could be retrieved (Figure12A-C). There is correlation between expression of GFP and c-Kit (Figures 12F-12H).

Harvested neonatal germ cells were cultured in a stem cell culture medium with a mixture of growth factors as described on culture dishes. Initial GFP signals (Figure 13A) disappeared after a few days in culture (Figure 13B). Thereafter, cells underwent distinct morphological changes, forming chain-like colonies that continued to grow without GFP signal (Figures 13C-13D). Up-regulation of Oct-4, indicated by the occurrence of GFP positive cells within colonies appeared after 3-4 weeks of culture (Figure 13F). After 2-4 weeks in culture, GFP positive colonies were mechanically transferred into culture dishes with mitomycin C-treated murine embryonic fibroblast feeder layers (MEF; see below) in the same culture medium supplemented with 15% FBS. After passing 3-4 times, via mechanical transfer to new MEF cultures the colonies were established and could be removed from the culture plate enzymatically (collagenase 1 mg/ml, 5-10 min) for further expansion and/or storage in liquid nitrogen. For derivation of cell lines from adult mice, GFP positive cells harvested after differential adhesion were sorted using FACS and were directly cultured on MEF. Using OG2 or OG2-lacZ mice, in total 19 cell lines (10 neonatal and 9 adult) have been generated. All cell lines expressed either GFP (14 lines) or GFP-LacZ (5 lines) (Figures 13G-13I).

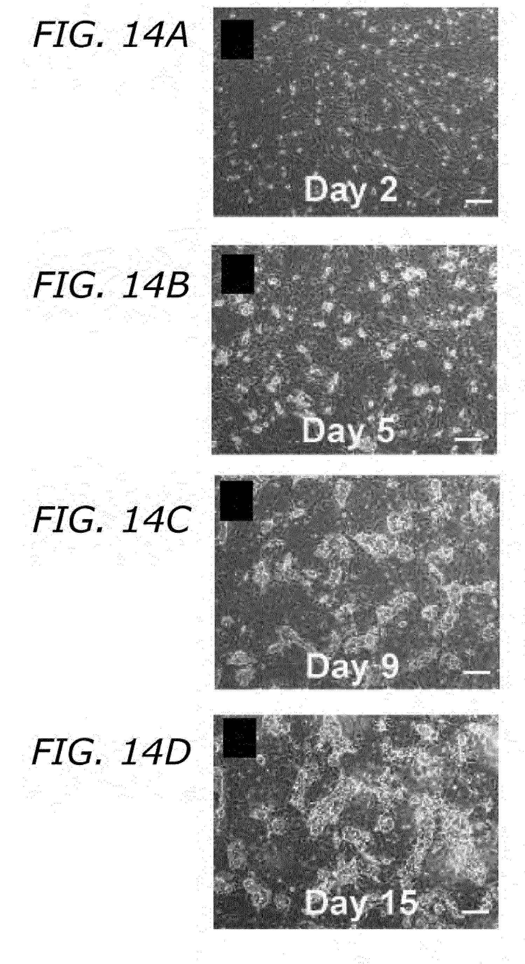

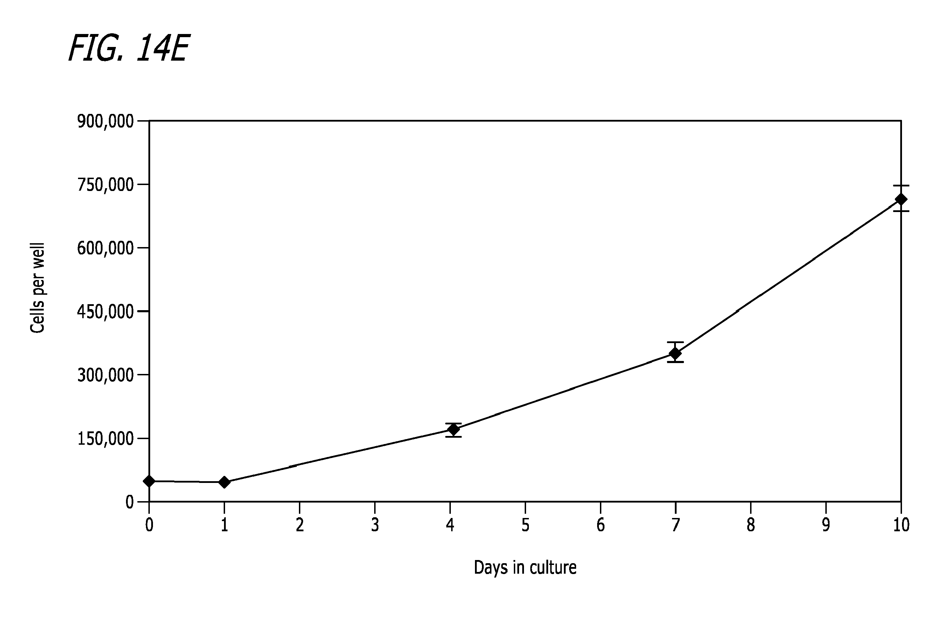

In addition, a mGC line has been generated from neonatal wild type CD-1 mice indicating that the method is not limited to transgenic OG2 mice. Selected cell lines have been frozen/thawed and propagated for more than 40 passages with an estimated cell doubling time of 72 hr (using both manual cell count and GFP sorting (Figure 14). At different time points during culture (day 2, Figure 14A; day 5, Figure 14B; day 9, Figure 14C; day 15, Figure 14D), the number of GFP-positive cells were analyzed by FACS (Figure 14E).

C-kit positive/GFP positive cells were separated from the c-Kit negative/GFP positive cells by FACS Figure 12D-E) and cultured on MEF feeders. Only c-Kit positive populations generated mGC colonies and no cell line could be generated from the c-Kit negative pool. Among the growth factors used in this study, removal of GDNF resulted to smaller colonies indicating the role of GDNF in self renewal of the mGCs. In contrast, removal of FGF2 resulted in differentiation of the colonies, indicating possible role of FGF2 in maintenance of the mGCs in their undifferentiated stage. Removal of LIF or EGF did not affect either the expansion or differentiation of the mGCs.

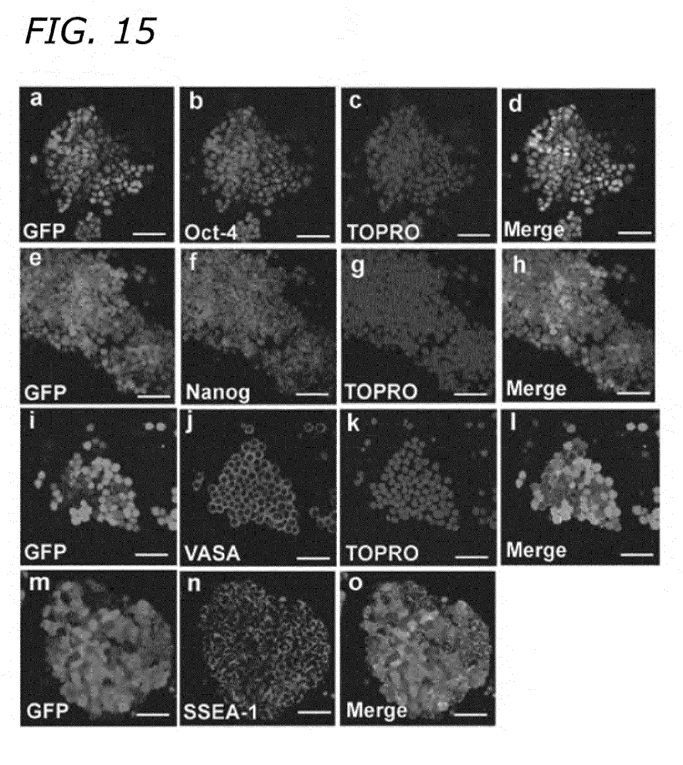

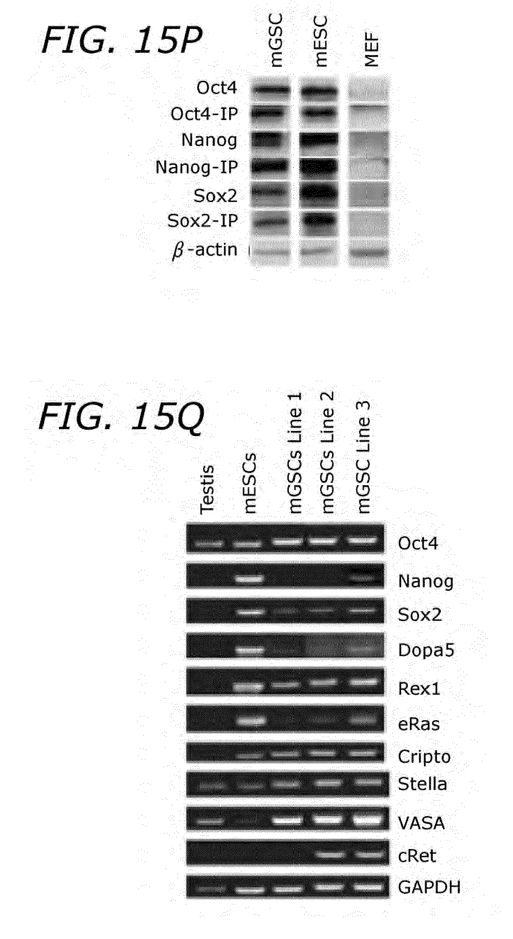

The majority of cells in the mGC colonies expressed Oct-4 (Figures 15A-15D), Nanog (Figures 15E-15H), SSEA-1 (Figures 15M-15O) and VASA (Figure 15I-15L). They also expressed pluripotent genes Sox2, DPPa5, Rex-1, eRas, and Cripto along with germline specific markers, including Stella, Dazl, Vasa and cRet (Figure 15Q). In addition, the expression of Oct-4, Nanog and Sox2 was confirmed by Western blot analysis (Figure 15P).

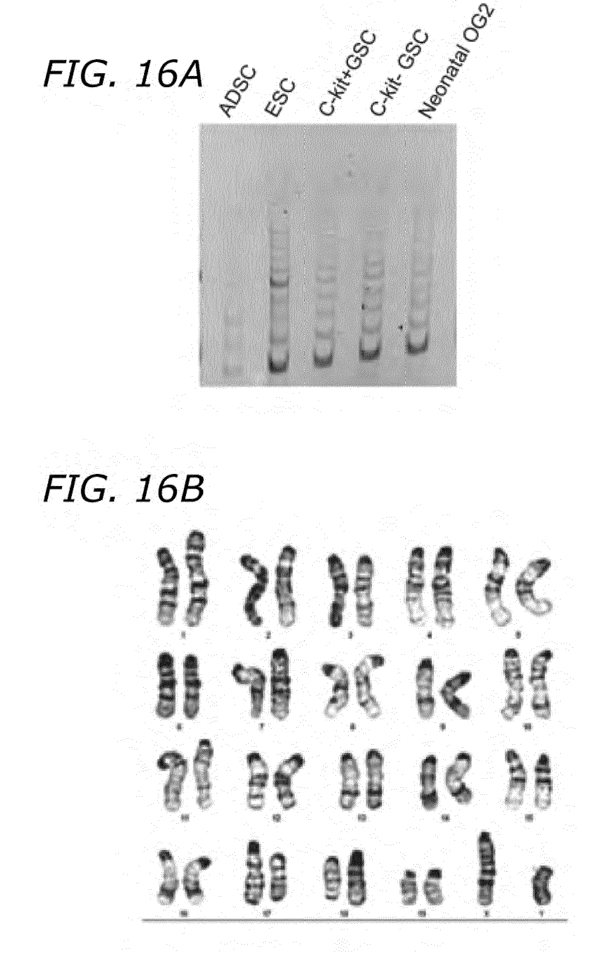

The mouse cell line at passage 20 showed high telomerase activity (Figure 16A, similar to ESC) and normal karyotype (40, XY) (Figure 16B).

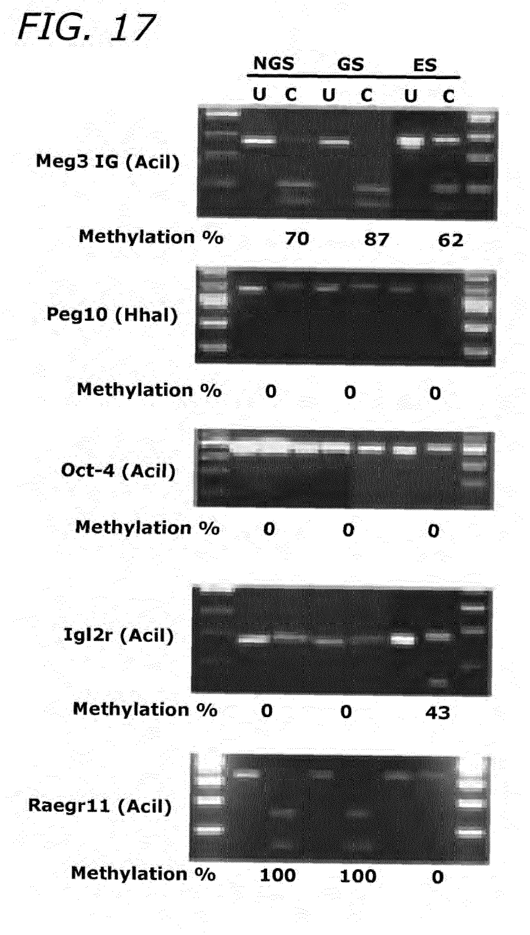

The global gene expression and imprinting patterns of the mGCs were also analyzed before and after culture and compared with that of ESC. Interestingly, culture conditions did not change the imprinting pattern of the mGCs in all the DMR (differentially methylated region) sites tested. In contrast to mouse ESC that showed only a partial androgenetic imprinting, the mGCs clearly exhibited a 100% androgenetic imprinting pattern (Figure 17). Somewhat surprisingly, microarray analysis showed that the global gene expression pattern of the mGCs had 87% similarity before and after culture.

When mGCs were aggregated to form embryoid bodies (EBs), gastrulation was observed within 9-15 days (Figure 18A). Cells in the EBs expressed early developmental markers including E-cadherin and laminin1 (markers of polarized epithelium (Figure 18B-18C), Zic1, PAX6 and Sox1 (early ectoderm markers, Figure 18D and 18F), GATA4 and FoxA2 (early endoderm markers, Figure 18E-18F), and Brachyury, BMP4 and COL2A1 (early mesoderm markers, Figure 18F). In culture, mGC colonies spontaneously differentiated into phenotypes expressing markers of cardiomyocytes (Figure 18G-18J), adipocytes (Figure 18K) and neural cells (Figure 18L and 18M). Some of the cells that spontaneously differentiated to cardiomyocytes exhibited rhythmic contractions for up to 3 days. Using directed differentiation protocols, mGC lines could be induced to differentiate into neural cells representing neural progenitors (nestin, neuroD1), neurons (MAP2, NF-68, GAD67) and glial cells (GFAP, MBP, A2B5, 04, NG2) as shown in Figures 19A-19G and 19J. They could also be induced to form cardiomyocytes (troponin1, cardiac myosin, desmin, Nkx2.5, GATA4, Figure 19I and 19L) or chondrocytes (collagen Xa1, and staining by alcian blue, Figure 19H and 19K).

In a separate differentiation study with mGCs, the number of cells (nuclei) were counted with and without staining of neural markers in seven colonies within a culture and the average percentage was estimated as 17.6% for GFAP⁺ cells, 2.5% for Tuj-1⁺ cells and 2.3% for MAP-2⁺ cells. In general, the efficiency of induced differentiation by these protocols was much higher in ES cells compared to the mGCs.

Four weeks after transplantation, testes of the control animals as well as those which received Oct-4 positive/c-Kit positive cells showed no spermatogenesis in the majority of the seminiferous tubules. However, 80% of the mice which received freshly isolated Oct-4 positive/c-Kit negative testicular cells showed some degrees of spermatogenesis throughout the testes, indicating the presence of functional SSCs in the cell suspension. Only the c-Kit-negative subpopulation of germline stem cells colonized the recipient testes. Testes regeneration following transplantation of germline stem cells before and after culture in presented in Figures 20M-20R and Table 1. Cross section of the normal testis of an immune-deficient mouse is depicted in Figure 20M. One month after busulfan treatment, the majority of the seminiferous tubules were depleted from endogenous spermatogenesis (Figure 20N). While 73% of seminiferous tubules of mice transplanted with Oct-4-positive/c-Kit-negative cells showed some degree of spermatogenesis (Figure 20O), the majority of tubule cross-sections of the mice receiving Oct-4-positive/c-Kit-negative cells were empty (Figure 20P). A CSFE-tagged positive colony shortly after transplantation of Oct-4-positive/c-Kit-negative cells is depicted in Figure 20R. No spermatogenesis was found in the majority of seminiferous tubules of the recipient mice testes transplanted with the mGCs, indicating these cells do not have SSC properties (Figure 20Q).

**Table 1. Restoration of spermatogenesis following transplantation of subpopulations of germline stem cells and multipotent germ cell lines in recipient mouse testes**

| Transplanted cells | Total No. of tubule cross sections analyzed | Total No. of tubules with spermatogenesis (%) | Total No. of empty tubules (%) |
|---|---|---|---|
| Oct-4 positive | 580 | 328 (56.5)^{b} | 252 (43.5) |
| Oct-4 positive/ c-Kit positive | 440 | 68 (15.5)^{a} | 372 (84.5) |
| Oct-4 positive/ c-Kit negative | 580 | 448 (77.2)^{c} | 132 (22.8) |
| mGC | 420 | 100 (23.8)^{a} | 320 (76.2) |
| Sham | 480 | 80 (16.6)^{a} | 400 (83.4) |

For teratoma formation, equal numbers of mouse ESC (as positive control) or Oct-4-GFP/LacZ mGCs were injected into the skin, muscle and testes of different groups of nude mice (1x10⁶ cells/site). All recipient mice (6/6) receiving ESC developed teratomas in all three tissue types. In contrast, none of the mice (0/20) receiving mGCs gave rise to teratomas (Figures 20A-20F). Six weeks after transplantation, Oct-4-GFP/LacZ cells, were found in skin, muscle and testicular tissues (Figires 20G-20I). These data show that mGCs, unlike ESC, are non-tumorigenic.

Chimera formation was measured by injecting cultured Oct-4-GFP/LacZ cells into 8-cell embryos and blastocysts of CD-1 mice. As shown in Figure 21A-21D, Oct-4-GFP/LacZ cells incorporated into the inner cell mass of the mouse blastocysts. The embryos were transferred into the uterus of pseudo pregnant mice (a total of 45 fetuses from 119 transferred embryos). At 12.5 dpc (days post coitus) staining of whole embryos for LacZ (β-galactocidase activity) showed distinctive patterns in the eye, brain, and limbs (Figure 21 E). The intensity of LacZ staining was much higher in chimeric embryos received mouse ES cells than those injected with multipotent germ cell lines. The distribution of chimeric cells is also demonstrated in histological sections of the brain, heart, gonadal ridge and liver (Figure 21L-21O). The intensity and number of LacZ positive cells was much higher in chimeric embryos injected with LacZ-ES cells than those injected with LacZ-GS cells. Confirmation of Oct-4-GFP/LacZ chimeric tissues was supported by the presence of GFP DNA sequence in the ectodermal (brain), mesodermal (heart), endodermal (liver) and testis of the chimeric pups (Figure 21 P), as well as the presence of LacZ DNA (Figure 21Q) in all 4 tissues. These combined results clearly demonstrate that cultured mGCs are non-teratogenic stem cells with some pluripotent characteristics.

Application of stem cells for therapeutic purposes has been the focus of stem cell science since the successful derivation of human pluripotent ESC from pre-implantation embryos in 1998. Since then, numerous studies have explored the potential of different stem cells, including ESC, EG cells and adult stem cells. The possible existence of multipotent germ cell lines in neonatal mouse was reported using culture-induced reprogramming. Multipotent neonatal cell lines had ESC-like characteristics but also, without germline SSC functional properties and markers. Recently, the existence of such a subpopulation in the adult mouse testes, cells susceptible to culture-induced reprogramming, has been reported. Interestingly, cell lines generated from this adult cell population acquired ESC-like characteristics, but also maintained their SSC functional properties. These seemingly different properties could result from differences between neonatal and adult tissues.

Multipotent germ cell lines can be generated from adult mouse testes without reprogramming growth factors; indicating the possible presence of a subpopulation of cells with pluripotent characteristics in the adult testes.

Independently, as described supra, germline sex cell lines and germline precursor cells from post-natal mouse germline stem cells were derived with some, but not all, of the pluripotent characteristics of ESC. Both of these cell line types are distinctively different from the multipotent germ cell lines obtained by the other laboratories, most notably, with regard to the extend of pluripotentiality and teratoma formation.

Based on microarray analysis, mouse Oct-4 positive/c-Kit positive germline cell lines expressed pluripotent genes Nanog and crypto but at 1000-fold and 5000-fold lower levels than in ESC. Similarly, the germ cell lines expressed oncogenes including, but not limited to, p53, Eras, Bak, Int-2 and c-myc, but the expression levels were several fold lower than with ES cells. Remarkably, the germ cell lines did not form teratomas upon transplantation in vivo, but they did form limited chimeric cell populations in mouse embryos.

Several lines of evidence support the notion that the Oct-4 positive/c-Kit positive germline precursor cells retain their germ cell properties and thereby differ from ESC and other previously reported testicular cells: namely, 1) the derived germline precursor cell lines have a cell cycle time that doubles their cell numbers in about 72 hours (determined by both GFP sorting and manual counting), and this cell cycle time is more similar to that of germline stem cells and is about three times longer than that of the ESC; 2) based on global gene expression analysis in arrays, the instant germline precursor cells seem to have molecular characteristics different from those in ESC or other multipotent germ cell lines. Among the genes tested, the instant germline precursor cell lines showed significantly higher expression level of germline specific genes (Vasa, Plzf, GFR-α1, Dazl) and lower expression level of pluripotent genes (Oct4, Nanog, Dppa-5, Sox2, Crypto); 3) these cell lines are more dependent on GDNF for their self renewal than LIF or FGF2. GDNF has been proposed to be the key regulator of the self renewal of male germline stem cells, while LIF and FGF2 play crucial role in self renewal of ESC; 4), the expression level of SSEA-1 in these cell lines was lower than the level found either in mouse ES cells or other multipotent germ cell line as reported. It has been shown that SSEA-1 may be involved in tumor invasion and metastasis in certain animal model systems suggesting that higher expression may reflect higher potential for tumorigenesis; and 5) multipotent GCs exhibited an androgenic imprinting pattern that is different from mouse ESC or other mGC lines reported by other laboratories.

Despite of all the similarities to their germline ancestors, the instant germline precursor cell lines did not regenerate testes following transplantation demonstrating that they were not germline sex cells.

The transgenic mouse model allowed the isolation germline stem cells from both neonatal and adult testes based on their Oct-4 expression. The germline stem cells were further fractionated into two subpopulations according to their expression of c-Kit with the following observations: 1) only the Oct-4 negative/GFP positive cells that possess the c-Kit receptor molecule responded to culture and generated multipotent germ cell lines; and, 2) only the c-Kit negative subpopulations repopulated the testis after spermatogonial stem cell transplantation. The results clearly indicate the presence of at least two distinct subset of germline stem cells within reproductive tissues: (1) a c-Kit positive pool with the ability to become multipotent germline stem cells, i.e. germline precursor cells, as well as, (2) a subset of germline stem cells that have lost their c-Kit expression and acquired the ability to colonize the testis, i.e., germline sex cells. Apparently, in adult tissues the germline stem cells in the reproductive organs are either present in different developmental stages, or alternatively, they possess differing abilities to respond to growth factor signaling and/or transcription factors.

MATERIALS AND METHODS

Testicular cells were isolated as follows. The testes of either neonatal mouse pups (age 1-5 days after birth) or adult mice were sterilely removed from the body. The capsule of the testes was removed and the seminiferous tubules were suspended in an enzyme solution consisting of 1 mg/mL collagenase 1 A and 10 units/mL DNase in PBS. The testes were digested at 37oC in a water bath until all tubules were digested. The reaction was stopped with Fetal Bovine Serum (FBS).

Preparation of mouse embryonic fibroblast (MEF) feeders: MEFs were made by standard procedures using 12.5 dpc CD-1 mouse embryos. The embryos were eviscerated before trypsinization, and the dissociated cells were plated onto 150-mm plates with plating density at approximately 1.5 embryos per plate. After the initial plating, MEFs were split 1:5 and then frozen (passage 1). Thawed MEFs (P1) were passed only once for expansion purposes prior to mitomycin C treatment. MEF feeders were plated in a density of 50-60x103 per cm2. New MEF feeders were used for pluripotent germ cell culture every 7-10 days. All the animal experiments followed the guide lines for the care and use of laboratory animals (National Research Council).

Evaluation of telomerase activity and karyotyping: For determination of telomerase activity, cell extracts were isolated from germ cell lines (passage 10 and higher), freshly isolated Oct4 positive/c-Kit positive sorted cells and Oct4+/ckit- sorted cells using CHAPS lysis buffer containing 150U/ml RNase. Cell lysates were centrifuged for 20 min at 12,000xg, 4°C and the supernatants were stored at -80°C. Protein concentration was assayed with Bradford reagent using BSA as a standard. Telomerase activity was detected by PCR-based assay using TRAPEZE Detection Kit (Chemicon). Two microliters of cell extract at 750µg/µl was added to a total volume of 50µl PCR reaction mix containing TRAP Reaction Buffer, dNTPs, substrate oligonucleotide, telomerase primer, internal standard primer, and Taq polymerase. As positive control, 2µl of mESC cell extract was added to the reaction mix, and CHAPS lysis buffer alone and heat inactivated telomerase were used as negative control for each experimental sample. Each sample was incubated at 30°C for 30 minutes for telomerase extension, followed by PCR amplification. For karyotyping, proliferating cells were incubated in culture with 0.1 µg/ml KaryoMAX Colcemid (Invitrogen) for 3-4 hr before they were re-suspended in hypotonic solution (0.075M KCL) and incubated at room temperature for 10 minutes. Cells were then resuspended in cold fixative (3:1 methanol: acetic acid) and stored at 4°C for at least 30 min. Following washing with fixative, cells were applied to clean glass slides and air dried. Metaphase chromosomes were prepared and karyotypes created using an Applied Spectral Imaging Band View digital imaging system.

In vitro differentiation: For generating embryoid bodies (EBs), mGSC colonies were dissociated with collagenase and plated in non-adhesive culture plates in PMTM medium (disclosed in co-pending US patent application no. 11/488,362 filed July 17, 2006 and incorporated by referenced herein for all it contains regarding tissue culture media) containing 15% FBS. In some experiments EBs were formed in hanging drops. EBs, for differentiation into cells representing the three germ layers, were cultured for 15 days with samples taken out every three days for marker determination. For induced differentiation, the EBs were cultured in PM medium for four days before they were cultured in the serum-free N1 medium for lineage selection: i.e., DMEM/F12 (Invitrogen) supplemented with ITS (insulin, 10 mg/l; transferrin, 5.5 mg/l; selenium, 0.67 mg/l) and fibronectin (50 µg/ml). After 5-7 days, N-1-treated cell aggregates were transferred to gelatin-coated culture plates in N2 medium for expansion of neural progenitor cells (N1 medium with ITS, without fibronectin and supplemented with 10 ng/ml bFGF). For differentiation into cardiomyocytes, EBs were cultured for two weeks in the presence of different cardiogenic compounds including 0.06 M DMSO, 5 mM 5'-aza-2'-deoxy-cytidine (AZA,) and 25-50 µM cardiogenol-C,. During the differentiation process, the morphology of cells was analyzed and samples were taken both for gene expression analysis by RT-PCR and immunohistochemical staining. Chondrocyte differentiation of mGSCs was induced by adding a chondrogenic induction medium (Chondrogenic SingleQuots, Cambrex) supplemented with 10ng/ml TGF-3ß and 20% FBS.

Immunocytochemical (ICC) and immunohistochemical (IHC) staining: Cultured cells were fixed in 4% paraformaldehyde for 10-30 min at room temperature and stored in PBS at 4°C. For fluorescent immunocytochemistry, cells were permeablized with 1x Cytoperm (BD Biosciences) or 0.2% Triton X-100 for 15 min and subsequently incubated in 2% (w/v) bovine serum albumin (BSA), 2% (v/v) normal goat serum (GS)/ 1x Cytoperm-PBS for 30-60 min both at room temperature. Primary antibody was either diluted at the optimal concentration in 2%BSA 2% GS / 1x Cytoperm-PBS and incubated for 3 hr at 4°C, or diluted in blocking buffer overnight at 4°C. After two washes, fluorescent secondary antibody was diluted accordingly in 2% BSA/2% goat serum/1x Cytoperm-PBS and incubated for 1 hr at 4°C in the dark. Cells were washed twice in PBS, wrapped in foil and stored at 4°C until microscopic analysis. Images were recorded using an Olympus IX71 microscope or Ziess LSM510 confocal microscope equipped with digital image hardware and software.

For brightfield immunocytochemistry, cells were washed once in 1x PBS. Endogenous peroxidase activity was blocked with 3% (v/v) H2O2 for 15 min followed by permeabilization- blocking with 2% BSA/2% GS/1x Cytoperm-PBS for 30 min. Primary antibody was diluted accordingly in 2% BSA/2% GS/1x Cytoperm-PBS and incubated for 3 hr at 4°C. The remainder of the staining was accomplished using ABC staining kits according to the manufacturer's instructions. Visualization was with enhanced diaminobenzidine (DAB) substrate tablet dissolved in purified water and incubated for 5-10 min. For negative controls, the primary antibody was omitted.

Flow Cytometry: Specific antibodies, including SSEA-1 and cKit were optimized for flow cytometric analysis with an Influx Cell Sorter (Cytopeia, Inc). For c-Kit sorting, freshly isolated testicular cells containing the Oct-4-GFP construct were stained with CD117 APC For some experiments, fresh germ cell colonies were dissociated and cells were stained with anti-SSEA-1 antibody following by goat anti-mouse IgM conjugated with PE-Cy7.

Gene expression, imprinting analysis and GFP amplification: Total RNA was isolated using RNeasy Mini Kit (Qiagen) and RNA was used for RT-PCR, Quantitative PCR or Microarray analysis. For RT-PCR, cDNA was synthesized with the Sensiscript RT Kit, and PCR was performed with HotStarTaq DNA Polymerase. All PCR reactions began with an initial incubation at 95°C for 15 min to activate the enzyme. This was followed by 35 cycles of 95°C for 15 sec, the appropriate annealing temperature for 1 min and 72°C for 1 min, which was then followed by 1 cycle of 72°C for 10 min for final extension. Reactions were carried out using an iCyclerTM Thermal Cycler (Bio-Rad). The procedure for RT-PCR was carried out using specific primers including, Oct-4, Nanog, Rex-1, DPPa5, Dazl, ß actin, Nkx2.5, Nestin, Mab2, and GFAP. For internal controls, GADPH was used as a house keeping gene for cellular samples and ß-actin or interleukin-2 (IL-2) was used in mouse embryos.

Imprinting patterns in mGSCs and mESCs were determined by a PCR-based analysis. PCR amplification of each dimethylated region (DMR) from bisulfite-treated DNAs was carried out by specific primers. For analysis of the imprinted genes the UVP image software was used to quantify the band intensity. For GFP and LacZ amplification, individual tissue from chimeric embryos were carefully collected by dissection, minced into small pieces, and placed in DNA extraction buffer (DNeasy kit) for DNA isolation and purification according to the manufacturer's protocol.

Spermatogonial stem cell transplantation: To test the functionality of mGCs for regeneration of spermatogenesis, spermatogonial stem cell transplantation was used. Twenty 6-8 weeks immune deficient nude male mice (Harlan) were treated with busulfan (40 mg/kg) and used as recipients. One month after busulfan treatment, 2x105 cells were transplanted into the seminiferous tubules via rete testis injection. Four mice received mGCs (GFP sorted cells). Four other mice were injected with freshly isolated GFP positive sorted cells. Four mice were transplanted with freshly isolated GFP positive c-Kit positive sorted cells, and four mice were injected with freshly isolated GFP positive c-Kit negative sorted cells. The remaining four mice served as sham control and were not injected. One month after transplantation, the animals were sacrificed and testes were harvested and used for histological evaluations. To evaluate the efficiency of transplantation, total number of tubular cross sections with spermatogenesis was counted.

Tests for teratoma and chimera formation: To test the ability of the mGCs to form teratomas or chimeras, OG2 mice (Jackson laboratories) were bred with Rosa 26 mice (Jackson laboratories) and a new strain (OG2-R26) was generated. These mice have both GFP and LacZ constructs in their germ cells. Culture was performed as described and new Oct4-GFP/LacZ germ cell lines were produced for testing teratoma and chimera formation. Mouse Oct4-GFP/LacZ mGSCs were examined for their ability to form teratomas in vivo by subcutaneous, intra muscular or injection into the seminiferous tubules of nude mice. As positive controls for teratoma formation, ES cells were injected in some mice. For subcutaneous, intramuscular or testicular injections, approximately 1x106 cells were injected. Mice were sacrificed six weeks later, and tissues were harvested for morphological and histological analysis.

The ability of mouse Oct4-GFP/LacZ GSCs to form chimeric cell populations was determined after injection into host blastocysts, or by their aggregation with morula-stage embryos or eight-cell stage embryos. Blastocyst injections of 15-20 cells were carried out using day-3.5 blastocysts collected from CD-1 mice. After injection, blastocysts were transferred (7-8 blastocysts in each horn of the uterus) into 2.5-day pseudopregnant CD-1 females, previously mated with vasectomized males. Incorporation of lacZ cells was examined in different areas of the chimeric 12.5 dpc embryos by the ß-galactocidase staining kit (Sigma). In addition, lacZ and GFP PCR were performed in DNAs isolated from the brain, heart, liver and gonadal ridges of the chimeric embryos formed from Oct4-GFP/LacZ cells.

### Example 6

### Identification, Characterization and Isolation of Primate Germ Line Stem Cells

Since quiescent and actively dividing germline stem cells existed as two discrete cell populations in mouse testes (Example 5), the possibility that these two cell populations might also be present in adult primate testes was investigated.

Spermatogenesis is a highly regulated process in which undifferentiated germ cells classified as spermatogonial stem cells (SSC) divide and mature to produce spermatozoa. In rodents, Aₛ (A_{single}) spermatogonia are considered to be the resident stem cells responsible for spermatogenesis as they are capable of both self-renewal and differentiation. Unlike rodents, in primates and humans prior histological studies of other investigators report cells with two different distinct types of nuclear staining resident on the basement membrane of the testicular seminiferous tubular epithelium, i.e., designated as A_{dark} and Aₚₐₗₑ spermatogonia.

Below are described markers and isolation methods for substantial purification of primate testicular germline stem cells.

Rhesus monkey testes was used for characterization of primate germline stem cells. Immunohistochemical examination, surface markers and fluorescence activated cell sorting were used to identify, characterize and substantially purify germline stem cells from adult Rhesus monkey testes. The presence of germline stem cells in each FACS cell population was confirmed using telomerase, RT-PCR and immunohistochemical staining with the germ specific marker VASA and SSC-specific marker GFR-α1. Spermatogonial transplantation was used to define the functional capacities of cell populations before and after enrichment.

Immunohistochemical methods were used to identify, characterize and localize germline stem cells in primate testes. For these studies antibodies specific for extracellular matrix component (ECM) α6-integrin, SSEA-4 and GFR-α1 were used to visibly stain histologic sections of primate testes.

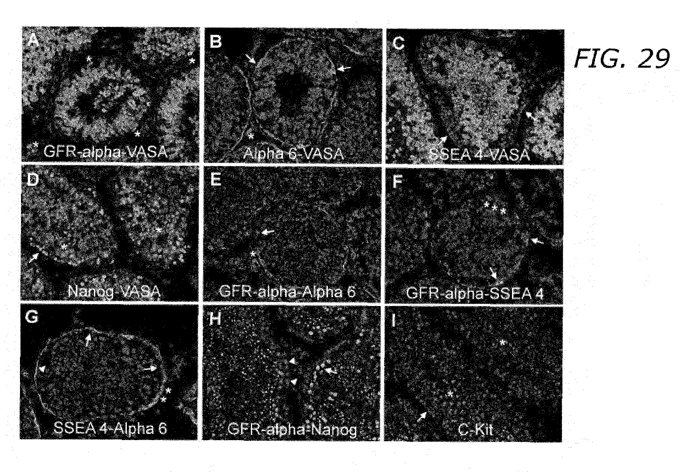

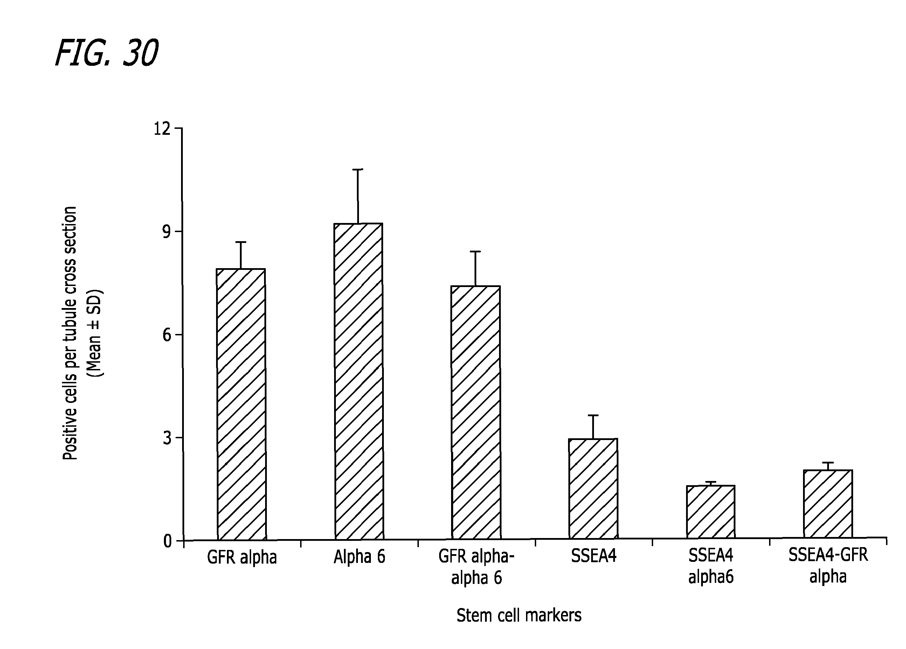

Antibodies specific for α6-integrin stained cells located adjacent to the basement membrane in the seminiferous tubules, as well as the seminiferous tubular basement membrane (Figure 29). An average of thirteen α6-integrin positive cells were found in each seminiferous tubule histologic cross section. Within seminiferous tubular sections the majority of α6-integrin positive cells were also VASA positive, confirming their germline stem cell status. Quantitatively, there were more α6-integrin positive cells per tubular cross section than GFR-α1 and co-localization studies showed that about 60% of α6-integrin positive cells were also GFR-α1 positive. These combined immunohistochemical studies of primate testis revealed cells adjacent to the basement membrane of the seminiferous tubules which had co-localization of α6-integrin and SSEA-4 cell surface markers, and, with germ cell specific marker VASA and SSC specific marker GFR-α1, i.e., markers specifically expressed in male germline stem cells (Figure 30).

The GFR-α-1 cell surface marker was specifically expressed in cells located at the basement membrane of the seminiferous tubules. All of the GFR-α-1 positive cells were also positive for germ cell specific marker VASA.

All SSEA-4 positive cells were located at the basement membrane of adult primate seminiferous tubules and these cells also were positive for VASA staining. The majority of SSEA-4 positive cells were also α6-integrin positive. There was also significant co-localization between SSEA-4 and GFR-α1 showing that SSEA-4 is an important cell surface marker for germline stem cells. About 40% of spermatogonial cells at the basement membrane of the seminiferous tubule in primate testes histologic cross-sections expressed SSEA-4.

Very few c-Kit positive cells were found in primate testes. Within tubular cross sections, c-Kit staining was only found in the cells located at the lumen of seminiferous tubules. All c-Kit positive cells were also VASA positive showing that they were differentiated germ cells. No c-Kit staining was found in cells located at the basement membrane of the seminiferous tubular cross sections. These findings indicate that in the primate germline stem cell are c-Kit negative.

Nanog was expressed in abundant in primate testes. Nanog appeared as a nuclear staining and was colocalized with VASA in almost all germ cells in seminiferous tubules. Nanog expression was stronger in advanced germ cells located at the lumen of seminiferous tubules compared to undifferentiated germ cells located at the basement membrane. Co-localization study of Nanog and GFR-α1 showed that all the germline stem cells showed a low level of Nanog expression.

CD90 antibodies stained only the basement membrane and did not stain any cellular structure in the testes.

The immunohistochemical characterization of primate testicular samples showed that germline stem cells in the adult primate testes are positive for α6-integrin, SSEA-4 and GFR-α1 and are negative for c-Kit.

Testes from euthanized Rhesus monkey, age 3-7, were surgically removed; placed in PBS supplemented with penicillin/streptomycin (Cellgro and Invitrogen, respectively) and transported overnight on ice. After surgical removal of the testicular capsule, biopsy samples were removed for histology and molecular analysis. The remaining seminiferous tubular tissues were finely minced and digested with collagenase A (1 mg/mL) (Roche) and DNase (10 U/mL) (Invitrogen) in a reciprocating 37°C water bath for 15 min. After collagenase digestion, the undigested tissue was sedimented at unit gravity and cells in the supernatant were removed. The undigested tissue was further digested in an enzyme cocktail consisting of 1.5 mg/mL collagenase A, 1.5 mg/mL hyaluronidase Type V (Sigma), 0.5 mg/mL trypsin (Worthington Biochemical Corporation), and 10 units/mL DNAse in DMEM in a reciprocating 37°C water bath for 20 min. Digested and undigested tissue were passed through a 70 µm strainer into FBS (fetal bovine serum; Hyclone) to inactivate enzymes. After centrifugation at 400Xg for 10 minutes, the cell pellets were resuspended in DMEM+10%FBS and placed in tissue culture coated 15cm dishes in a 5% CO₂/95% air humidified incubator.

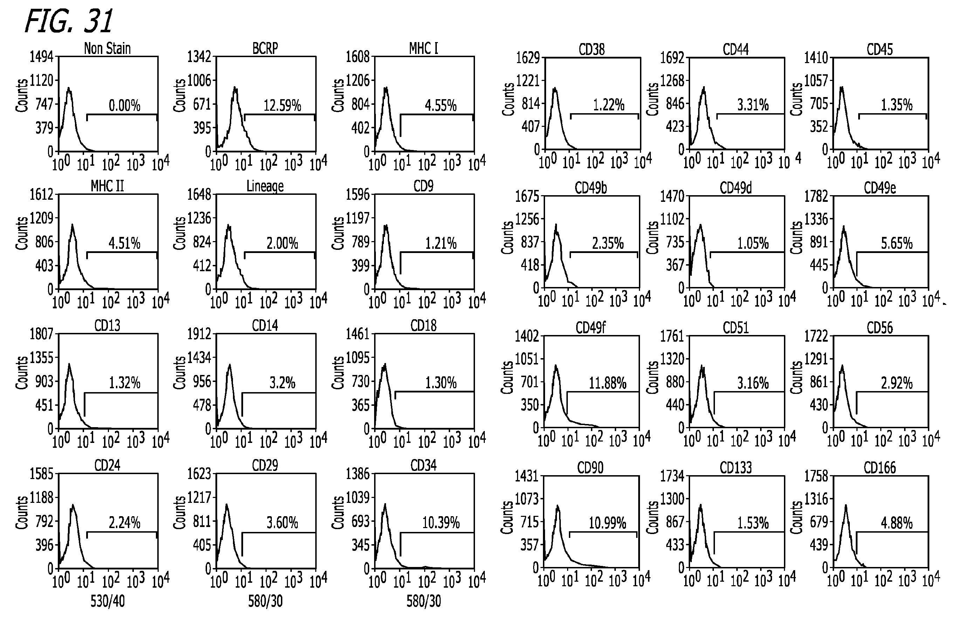

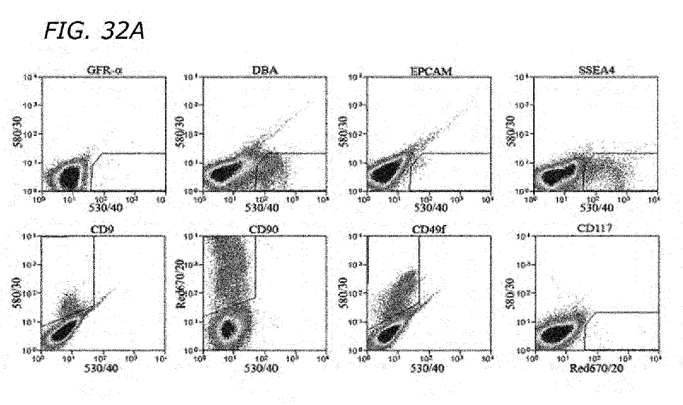

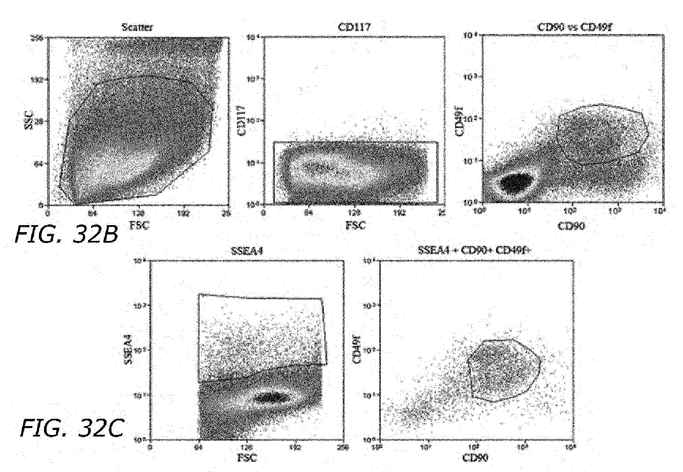

Fluorescence activated flow cytometry was used to identify cell surface markers specific for adult primate testicular germline stem cells (Figure 31). Contrary to reports from other investigators with non-primate SSCs, freshly isolated adult primate testicular cells did not express epithelial cell adhesion/activating molecule (EpCAM). However, germline stem cells were identified as a very small portion of the total adult primate testicular cell population, less than 1% of the total testicular cell isolate, by virtue of expression on their cell surface of the GDNF receptor GFR-α1. Similarly, contrary to experience with murine testicular germline stem cells (Example 5) freshly isolated adult primate germline stem cells did not express c-Kit. However, adult primate germline stem cells, (about 2% of the isolated testicular cell population), expressed cell surface carbohydrate determinants bound by *Dolichos biflourus* agglutinin (DBA), a lectin. In addition, the adult primate germline stem cells expressed the CD9, CD90 and CD49f cell surface markers (Figure 32).

For isolation of primate germline stem cells, c-Kit was gated as the negative/parent sorting window against which were plotted both CD90 positive and CD49f positive to identify the double positive CD90+/CD49f+ cells. Sorting for double positive cells resulted in isolation of germline stem cells, present as about 5.77% of total cells in the adult primate testicular isolates. The latter CD90+/CD49f+ double positive cells were collected for further use. Additional purification was achieved by selecting for c-Kit negative cells that were positive for SSEA4, resulting in isolation of a second substantially purified cell population that constituted about 2% of the total adult primate testicular cells. A yet additional purification was achieved by selecting for c-Kit negative cells that were positive for all of CD90, CD49f and SSEA4, resulting in isolation of a third substantially purified cell population that constituted about 1.47% of the total adult primate testicular cells.

These combined flow cytometric analysis resulted in isolation and substantial purification of two discrete germline stem cell populations from adult primate testis with the following properties: namely, (a) Thy-1 positive and α6-integrin positive cells and (b) SSEA-4 positive cells expressing both GFR-α1 and VASA cell surface markers and high telomerase activity, cell populations where more than 50% of the cells were positive for both GFR-α1 and VASA cells.

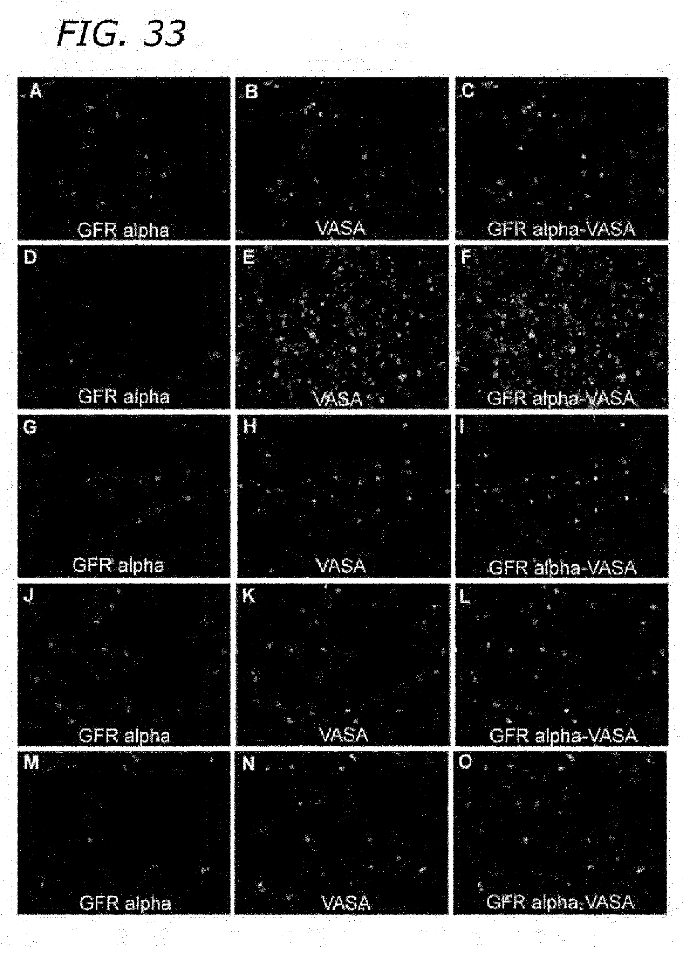

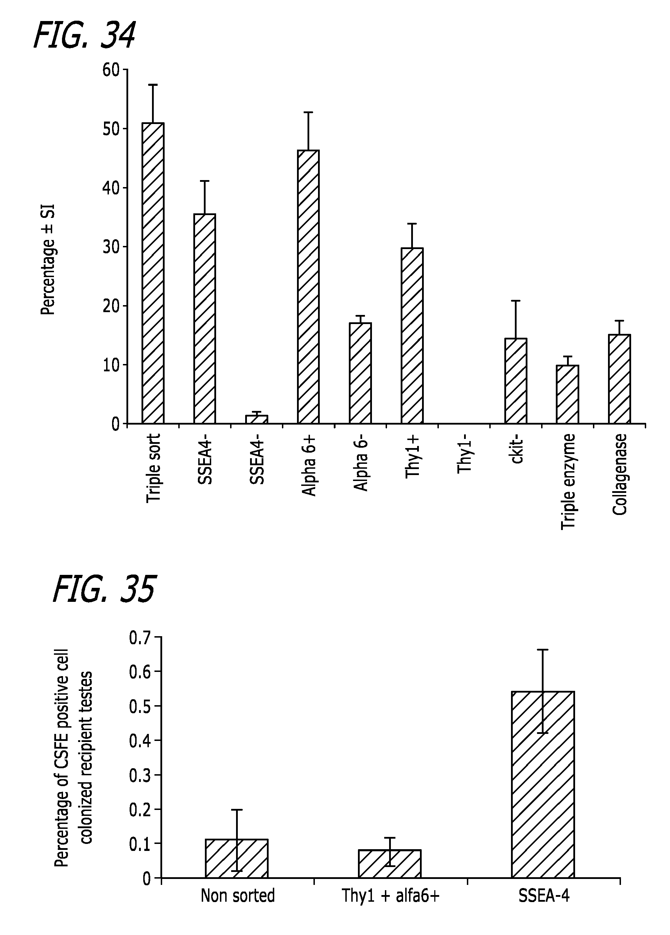

To further extend the FACS analysis and immunohistochemical staining (Figure 33) freshly isolated adult primate testicular cells were sorted as follows: (i) α6-integrin positive; (ii) CD-90 positive; (iii) CD-90 positive, α6-integrin positive and c-Kit negative (triple sort); and (iv) SSEA-4+ cells. The different isolated and purified testicular cell populations were tested for the presence of germ cell marker VASA and SSC marker GFR-α1 (Figure 34). Non-sorted cells contained about 70% VASA positive cells, but only 10% of these cells stained positive for GFR-α1. Sorting for just α6-integrin resulted in a significant increase in cells with both germline and SSC markers, i.e., populations with 42.6% VASA positive and GFR-α1 positive cells. Sorting for CD-90 alone or in combination with c-Kit- (triple sort) also significantly increased the proportion of VASA-GFR-α1 positive cells to 30% and 46.4% respectively. Sorting for SSEA4+ alone, also resulted in an enrichment for cells expressing germline and SSC markers, i.e., sorted cell populations in which 37.5% of the cells were VASA positive and GFRα-1 positive.

The functional properties of different primate testicular cell populations were determined before and after substantial purification by testing for their ability to repopulate the basement membrane of seminiferous tubules in the testes of immunodefficient nude mice treated with the chemotherapeutic drug busulfan. For these studies nine 6 to 8 week old SCID male mice (Harlan) were treated with busulfan (40 mg/kg). One month after busulfan treatment, 2x10⁵ adult primate testicular cells were transplanted into the seminiferous tubules via rete testis injection using methods essentially as described by Ogawa et al., 2000 For these studies, three mice received a transplant consisting of freshly isolated non-sorted cells; three mice received a transplant consisting of freshly isolated c-Kit negative and SSEA-4 positive sorted cells; and, three mice received a transplant consisting of freshly isolated isolated c-Kit negative and SSEA-4 negative sorted cells.

To better identify transplanted primate cells in the recipient mouse testes, the vital dye carboxyfluorescein diacetate succinimidyl ester (CSFE) was used as a fluorescent marker. CSFE is a colorless and non-fluorescent compound until the acetate groups are cleaved by intracellular esterases, yielding a highly fluorescent product. The latter fluorescent product was well retained and was fixed with aldehyde fixative, however, the fluorescent intensity diminished exponentially with each cell division. For this vital staining, cells were collected and washed once in 1XPBS containing 1%BSA; then, once in 1XPBS; followed by incubation in 8µM CSFE in 1XPBS at 37°C for 10 min. The resultant vital stained cells were washed with MEMα (Invitrogen) containing 2% FBS; collected by centrifugation at 400Xg for 5 min; re-suspended in media, and counted.

Two weeks after transplantation, mice were sacrificed and the number of CSFE positive cell colonies was determined microscopically in histologic sections of the mouse testes (Figure 35). Theoretically, if germline stem cells have a cell cycle time of about 72 hr, at two weeks post-transplantation the cells should have undergone 2-3 cell doublings, resulting in colonies of about 4-8 cells. For statistical analysis the ANOVA test was applied and p<0.05 was considered significant.

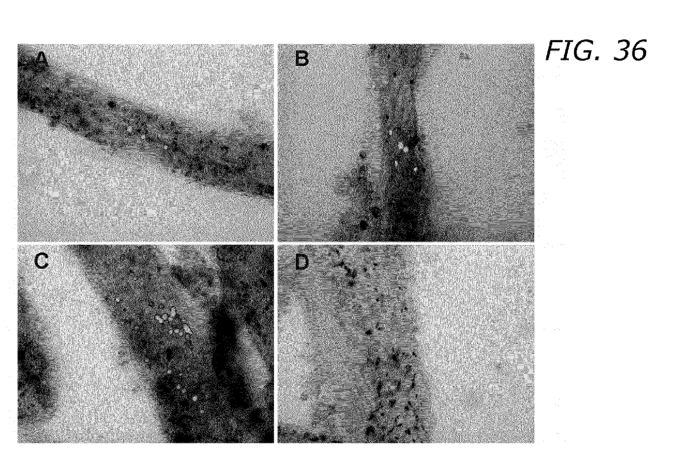

These combined transplantation studies showed that only the SSEA-4 positive cell population, containing cells expressing GFR-α1 and VASA markers, had the ability to repopulate the busulfan-treated mouse testes (Figure 36). These findings show that these cells are primordial germline stem cells.

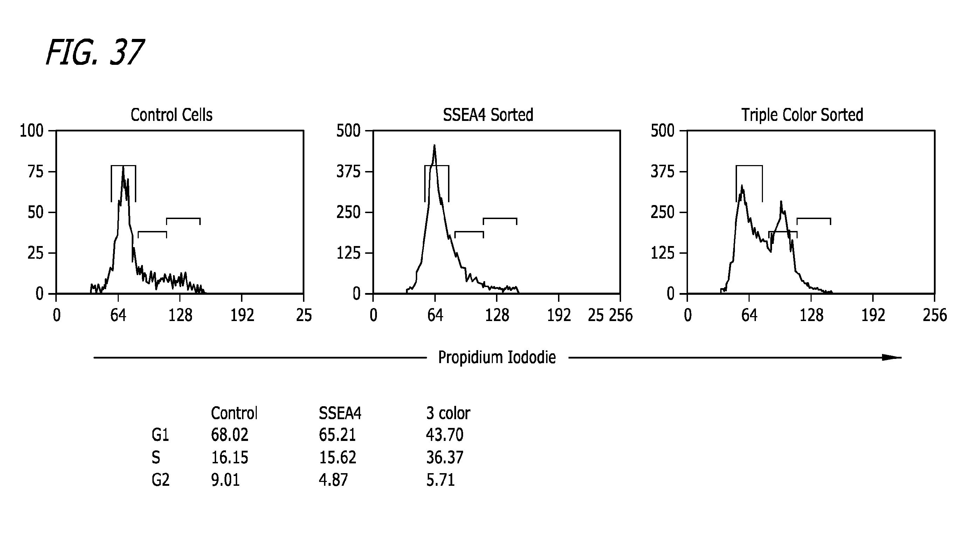

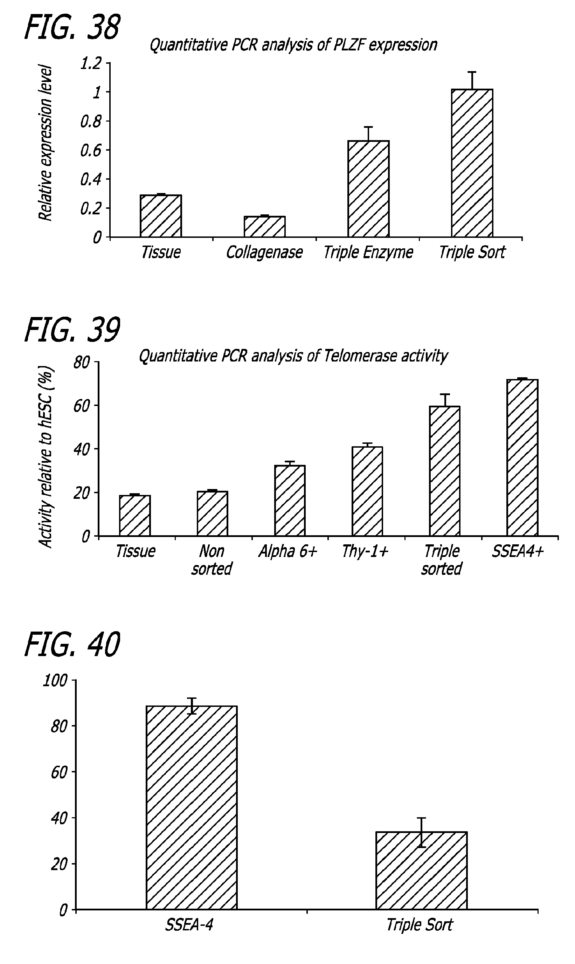

To investigate the cell division status of the respective cell populations, the DNA content of the two populations was investigated using flow cytometry (Figure 37). This analysis showed that the SSEA-4 positive cell population had a cellular DNA content resembling that of cells in G0-G1 stage of the cell cycle. In contrast, cells having the Thy-1 and α6-integrin cell surface markers had two discrete and different DNA contents, resembling either the G0-G1 stage of the cell cycle or the S phase. The data show that SSEA-4+ cell population with SSC cell surface markers represents a quiescent population of progenitor germline stem cells, while the Thy-1+ and α6-integrin+ population of cells represents an actively dividing population of SSCs (Figure 40).

The results show clearly that germline spermatogonial stem cells in the adult primate testis possess molecular and phenotypic characteristics similar but distinct from SSC in rodents. Immunohistological examination using a variety of stem cell, germ cell and spermatogonial stem cell specific markers revealed that in the primate GFR-α1 is specifically expressed at the surface of spermatogonial stem cells along the basement membrane of the semniferous tubules. GFR-α1 is the receptor for GDNF which is an important regulator of self renewal of SSC. GFR-α1 positive cells were VASA positive indicating that they are germ cells. Colocalization of α6-integrin with GFR-α1 was 80% in cells located within adult primate seminiferous tubules. FACS cell populations enriched by selecting α-6integrin positive cells showed a very high level of co-localization with GFR-α1, confirming the findings using immunohistochemical methods to identify germline stem cells in testes sections. Expression of α6-integrin on primate SSC indicates that this marker is conserved among the species as mouse, marmoset and human SSC also possess this marker on their cell surface. Localization of some α6-integrin positive cells within interstitial cells outside the tubules indicates that this marker alone can not be used for isolation of highly pure populations of SSC from adult primate testes.

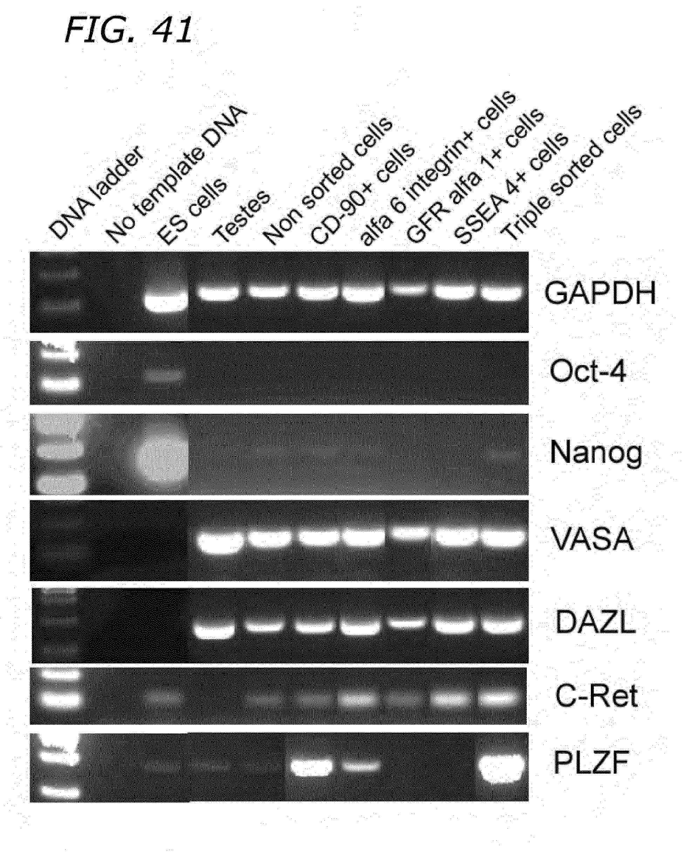

SSCs share some but not all phenotypic and molecular characteristics with other stem cells, in particular hematopoetic stem cells. The flow cytometry analysis, using a variety of cell surface markers, revealed that in the adult Rhesus monkey testes, there are distinct cell populations expressing α-6-integrin and Thy-1 and the majority of cells in primate testes were c-Kit negative. Immunohistochemical staining of primate testes also showed that all the cells along the basement membrane of seminiferous tubule were c-Kit negative indicating that α6-integrin positive cells are c-Kit negative. Sorting for α-6 integrin or Thy-1 alone resulted in enrichment of SSC markers as shown by immunohistochemical staining, RT-PCR and telomerase assay. Interestingly, sorting the α6-integrin+, Thy-1+ and c-Kit-cells resulted to the highest expression level of SSC marker PLZF as shown by quantitative RT-PCR (Figure 38) and the most elevated telomerase activity (Figure 39), indicating that combination of these markers enrich SSC in several folds. In addition, there were also a clear population of SSEA-4 positive cells in the primate testes, which also showed a high level of telomerase activity and expressed high level of both germ and SSC markers (Figure 41).

Immunohistochemical staining showed that SSEA-4 positive cells also located at the basement membrane of seminiferous tubules and are highly co-localized with α6-integrin and GFR-α1. Flow cytometric analysis showed that there are about 5-7% of α6-integrin+, Thy-1+, c-Kit sorted cells in adult primate testes while only 2-3% SSEA-4 positive cells are present. This is also consistent with immunohistochemical data on testes sections showing that there are significantly less SSEA4 positive cells found per tubule cross section than the α6-integrin positive cells. This also ndicates that SSC in primate testis have a phenotypic characteristics of α6-integrin+, Thy-1+ and c-Kit- with SSEA4+. SSEA-4 is stage specific embryonic antigen and is predominantly found in pluripotent cells like embryonic stem cells. Interestingly all the SSEA-4 positive cells co-expressed germ cell marker VASA, however only a fraction of these cells co-localize with GFR-α1 indicating that this marker expresses only on subpopulations of spermatogonial stem cells in monkey testis.

Morphological analysis of primate testes based on the density of the nuclear staining revealed that there are two types of undifferentiated spermatogonia in this species, A_{dark} and Aₚₐₗₑ. A_{dark} spermatogonia are thought to be the reserve stem cells and not actively dividing and Aₚₐₗₑ spermatogonia are shown to be the active SSC in primate testes.

Using DNA dye propidium Iodide (PI) in combination with flow cytometry we found that SSEA-4 positive population of germline stem cells have different DNA contents from the Thy-1+, α6-integrin+ cells. While SSEA-4 positive cells had DNA profile similar to the actively dividing cells, Thy-1+, α6-integrin+ cells showed an accumulated number of cells arrested in the S phase of the cell cycle. Moreover, SSEA-4 positive cells showed significantly higher proliferation activity as shown by PCNA staining than the Thy-1+, α6-integrin+ cells.

Pluripotent marker Nanog which has an essential role in maintaining ES cells in their undifferentiated stage was abundantly expressed in primate testes. Nanog expression in all germ cells and not only in SSC indicates a different role for this transcription factor in germline stem cells compared to ES cells. It has been shown that deletion of nanog in germ cells induces apoptosis rather than differentiation indicating that nanog is a survival factor for germ cells.

It is shown that 1 in 3000 cells in the adult mouse testes are SSC. The percentage of SSC in the adult monkey testes based on immunohistochemical staining with SSC specific markers GFRα1 and PLZF is very similar to what is described for rodents. The spermatogonial transplantation study also showed that in the adult monkey testes there are about 0.3% SSCs.

Demonstrated herein is that triple sorted (CD90+,CD49f+, c-Kit-) cells and SSEA4 positive cells show molecular and phenotypic characteristics of SSCs, however only the SSEA4 positive cells repopulated recipient testes after spermatogonial transplantation. This indicates that SSEA4 positive cells might represent the actively dividing SSC and triple sorted cells might resemble quiescent stem cells. Interestingly both SSEA-4 and Triple sorted cells expressed C-ret, receptor of GDNF, while only triple sorted cells showed PLZF expression. Both GDNF and PLZF are known to be major regulators of spermatogonial stem cell self renewal. While GDNF regulates SSC self renewal through up regulation of BCL6b transcription factor, PLZF maintains SSC self renewal with a yet unknown mechanism. Promyelocytic leukemia zinc factor (PLZF) is shown to inhibit cell growth at the G1/S transition and transit through S-phase by suppression of cyclin A which is available in a variety of cell types. PLZF is also shown to inhibit P21 another regulator of G1/S transition. Thus a high level of PLZF results in blockage of cell cycle and quiescence. Retinoic acid receptor alfa RAR-alfa is shown to reverse the cell cycle inhibition induced by PLZF by enhancing the expression of cyclin A.

MATERIALS AND METHODS

Primate germ cell substantial purification by flow cytometry: Flow cytometry sorting was accomplished using an InFlux Cell Sorter. For surface characterization and sorting, cells were stained with antibody reagents specific for stem cell surface markers and spermatogonial stem cell markers in non-primate species including anti-CD90-FITC, anti-CD49f-PE, and anti-CD117-APC. For these marker analyses, cells were stained for 30 minutes in complete medium on ice, washed once in cold staining buffer, resuspended in complete culture medium and kept on ice until cytofluorimetric analysis.

Primate germ cell magnetic sorting: The population of primate germ cells was enriched by tagging with magnetic microbeads and passing the cells through a magnetic column. Freshly isolated primate testicular cells were labeled with biotinylated antibodies for SSEA4 or for alpha-6-integrin and Thy-1 (Ebioscience, Abcam, BD Pharrmigen, respectively). Once biotinylated, the cells were labelled with streptavidin magnetic microbeads (Miltenyi Biotec). Magnetically labeled cells were selected for by passing the cells through a column in the presence of a magnet. Magnetically labeled cells were removed from the column by removing the column from the magnet, freeing the cells to be washed off of the column. This process was successful in enriching the population of cells positive for each of the markers up to 22X the original percentage in freshly isolated cells. In addition, magnetic sorting could provide a population as high as 90% purely labeled cells. This enrichment process was used in conjugation with fluorescent flow cytometry. By magnetically sorting the cell isolation before performing fluorescent flow cytometry the amount of time needed to sort out fluorescently labeled cells was greatly reduced and the number of fluorescently labeled cells that could be sorted out was greatly increased.

Primate germ cell immunohistochemical staining: Tissues were fixed overnight in 4% paraformaldehyde (PF; Electron Microscopy Science); transferred into 20% sucrose (Sigma) and frozen in OCT (VWR). Cryosections were prepared at 8 µm thickness and stored at -80°C. Sorted cells were fixed in 4% PF (Electron Microscopy Science), re-suspended in 100mM sucrose at approximately 25,000cells/10µl; 10µl aliquots were transferred onto ornithine/lysine-coated glass slides; and, the slides were placed on a 37°C hot plate until dry. Slides were stored at -80°C until analysis.

For immunohistochemical staining, the cells in testicular sections and in FACS sorted samples were permeabilized using 0.1 % Triton-X100 and blocked in either a solution containing 2% BSA and 5% Sheep Serum, or alternatively, in a solution containing 2% BSA, 5% Goat Serum and 0.1% Triton-X100. DAPI (Invitrogen) was used for nuclear visualization. Following multiple washes in 1X PBS + 2%BSA, cells were preserved using Permafluor (Beckman Coulter). Distribution of surface markers in tissue sections and sorted cells was evaluated using an Olympus BX-61 microscope fitted with SlideBook™ imaging software. For localization of primate testicular cells in mouse or primate tissues, 50 different seminiferous tubules were analyzed. For each different marker staining procedure, 3 to 4 different sections were analyzed; and, for FACS cell samples at least 200 different cells were analyzed in at least three different aliquots.

Primate germ cell RNA extraction, RT-PCR analysis and QRT-PCR analysis: Total cellular RNA was isolated using RNeasy mini kit (Qiagen) according to the manufacturer's recommendations. The isolated RNA was then transcribed to cDNA using the Quantitect RT kit (Qiagen) and purified with the QIAquick PCR purification kit. For each RT-PCR reaction, 20ng of cDNA template was used in a 25µL reaction volume with HotStar Taq Plus and with the different respective primers. All target cDNAs were amplified for 30 cycles. Amplification products were identified by size on a 2% agarose gel. For QRT-PCR, 5ng of cDNA template was used in a 25µL reaction volume with Quantitect Sybr Green PCR master mix (Qiagen) and the samples were amplified using a BioRad iCycler. Each sample was assayed in triplicate and normalized to a GAPDH control.

Primate germ cell telomerase assay: The SYBR Green real time quantitative telomeric repeat amplification protocol (RQ-TRAP) was employed, as adapted from Wege et al (2003). Tissue or cells pellets were washed once in PBS, resuspended and homogenized in 1 x Chaps lysis buffer containing RNaseOut Inhibitor (Invitrogen), at a final concentration of 1000 cells/µL and 400units/mL of the RNaseOut Inhibitor. After 25 min of incubation on ice, the cell lysates were centrifuged at 4°C in a microfuge at 16,000 rpm for 10 min. The supernatant was transferred to a fresh microcentrifuge tube and the protein concentrations determined by measuring absorbance at 280nm using an ND-1000 spectrophotometer (Nanodrop). Telomerase reaction volumes were 25µL in a solution containing 500ng protein lysate, Quantitect SYBR Green PCR mix, 1µg TS primer, 0.5µg ACX primer and nuclease-free distilled water. Each sample was tested in triplicate along with a no template control (lysis buffer), a positive control (ESC cells), and a standard curve prepared from aliquots of human ESC lysate that contained 1000ng, 200ng, 40ng, 8ng or 1.6 ng of protein. Using the iCycler iQ5 (Bio-Rad), the reactions were incubated for 20 min at 25°C, for 15 min at 95°C, and amplified in 40 PCR cycles under the following cycle conditions: 30 sec at 95°C and 90 sec at 60°C. The threshold cycle values (Ct) were determined from semi-log amplification plots (log increase in fluorescence versus cycle number) and compared with standard curve. The software default setting for the threshold was 10 times the mean of the standard deviation of the fluorescence reading of each well over the first 10 cycles, excluding cycle 1. Telomerase activities for different primate testicular cell samples were read from the standard curve and/or expressed as a percentage of the values recorded with human ESC lysate standards.

**Table 2. MEM-X Primate Media Composition**

| **Component** | **Final Concentration** |
|---|---|
| DMEM/F12 | N/A |
| Testosterone | 50 ng/mL |
| Estradiol | 50 ng/mL |
| Bovine Serum Albumin | 5 µg/mL |
| Sodium Pyruvate | 30 µg/mL |
| Hydrocortisone | 0.05mM |
| D/L Lactic Acid | 1 µl/mL |
| Glutamine | 1X |
| MEM Vitamin | 2X |
| MEM NEAA | 1X |
| Insulin-Transferrin-Selenine | 1X |
| Penicillin/Streptomycin | 1X |
| Epidermal Growth Factor | 20 ng/mL |
| basic Fibroblast Growth Factor | 10 ng/mL |
| human Leukemia Inhibitory Factor | 10 ng/mL |
| Glial Derived Neurotrophic Factor | 40 ng/mL |

### Example 7

### Differentiation of Primate Germline Stem Cells to Dopamine Producing Cells

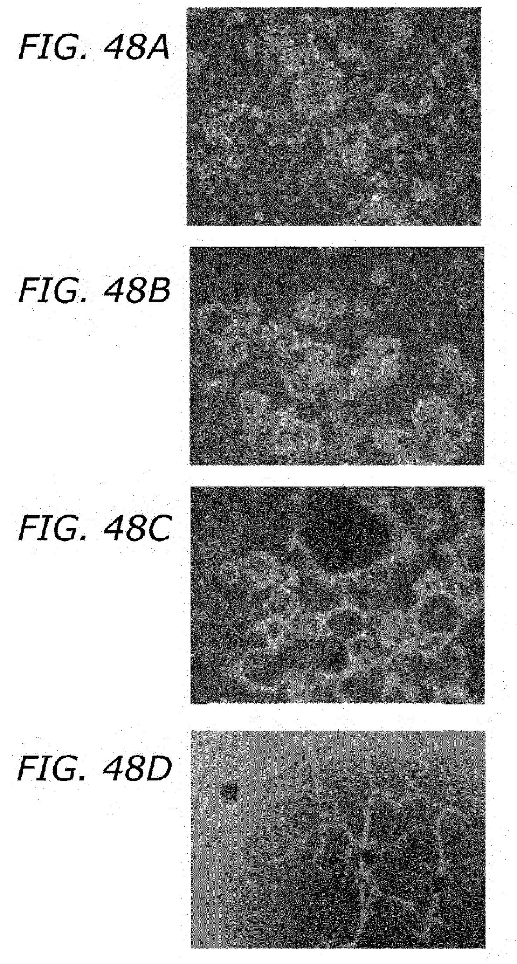

After testes cell isolation and red blood cell lysis, primate cells were plated in low-adhesion 6-well plates in N1 media (DMEMF-12+1x penicillin/streptomycin+1x ITS-X [Gibco]) at a concentration of 2 million cells per well. Cells formed cell aggregates and remained in this media for 5 days (Figure 48A). At this time 10 µM retinoic acid was added to the N1 media for 4 days (Figure 48B). The media was then changed to N2 media (DMEMF-12+1x penicillin/ streptomycin+1x N2-supplement (Gibco) + 10ng/ml FGF + 1ug/ml human fibronectin) for 5 days (Figure 48C). The cell aggregates were then plated in N1 media + 200 µM ascorbic acid into 4-well plates containing a confluent, mitomycin-C treated feeder layer of PA6 stromal cells on top of a glass cover slip coated with 0.1% gelatin. Each well contained approximately 10 to 15 cell aggregates and they remained in this condition for 16 days (Figure 48D) until the media was collected for high pressure liquid chromatography (HPLC) analysis for the presence of dopamine, the cells were fixed for immunocytochemical (ICC) analysis, and cells were sacrificed for RNA analysis.

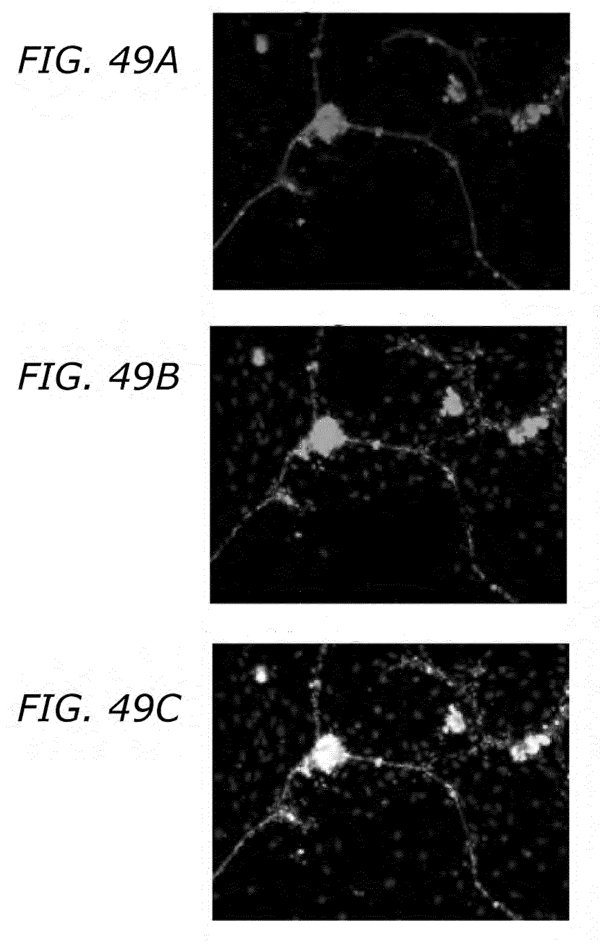

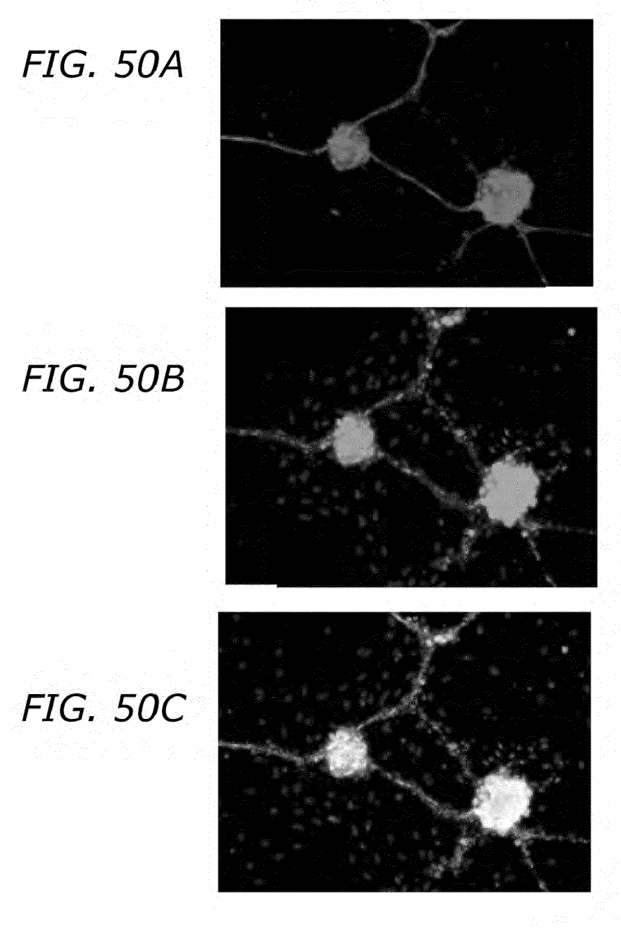

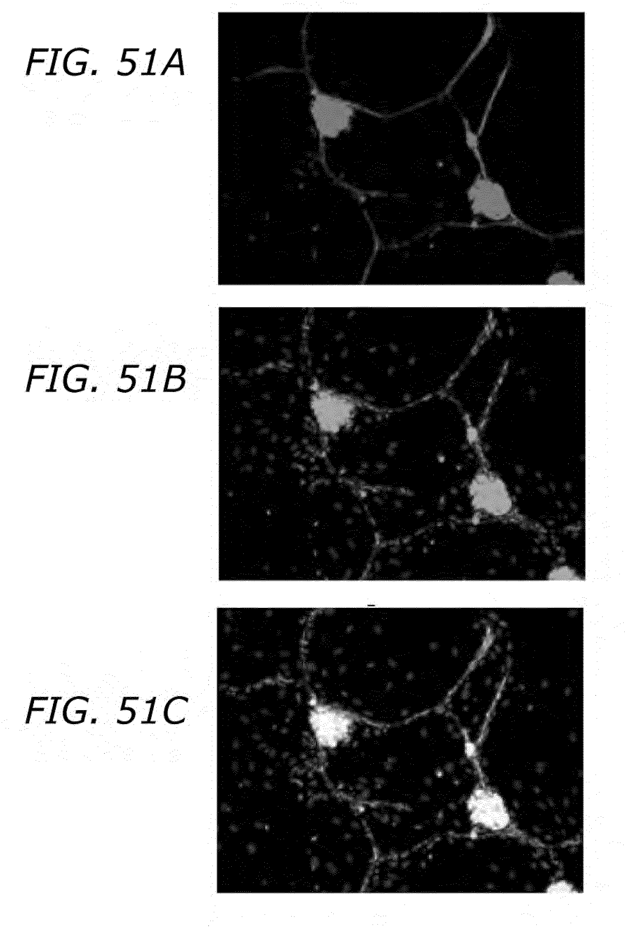

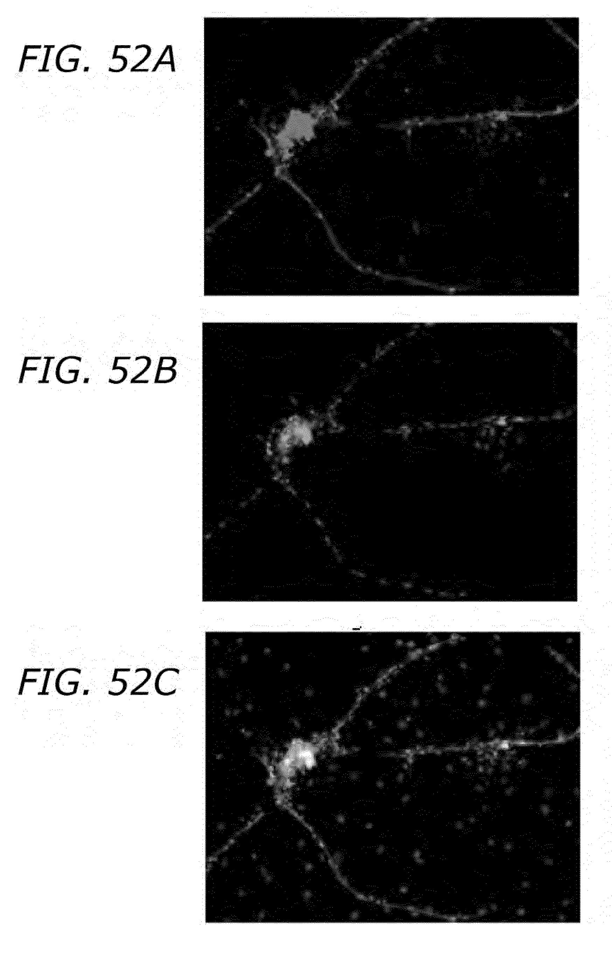

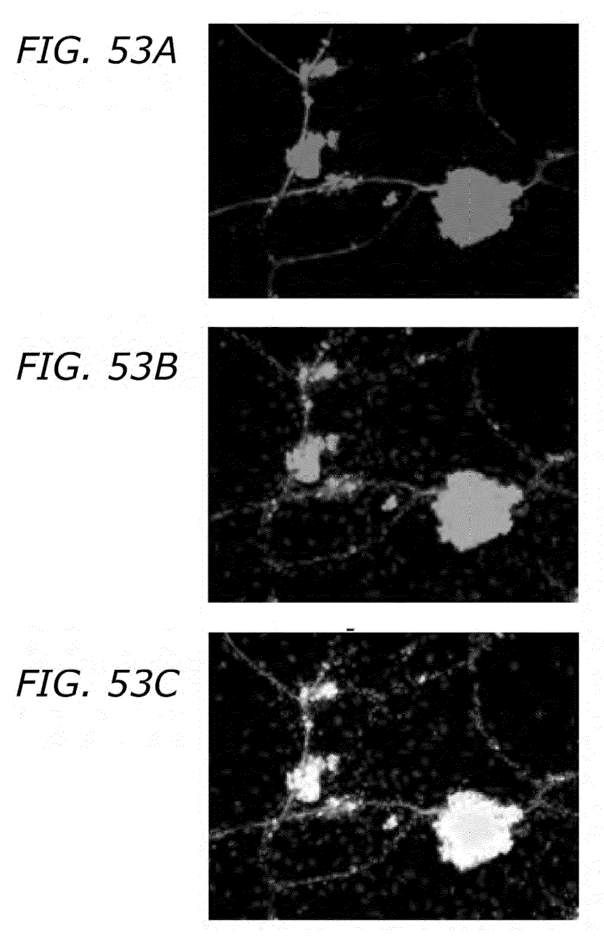

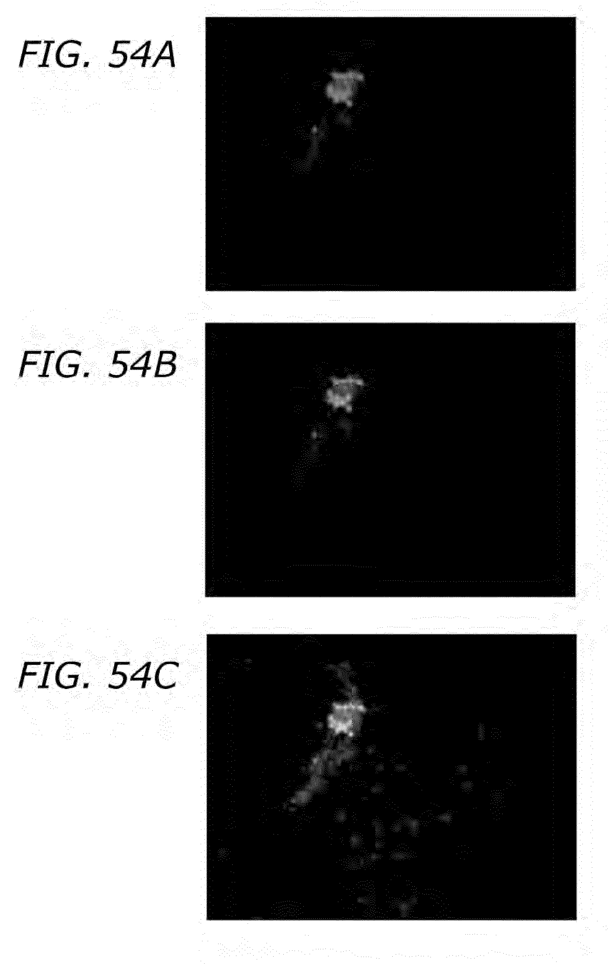

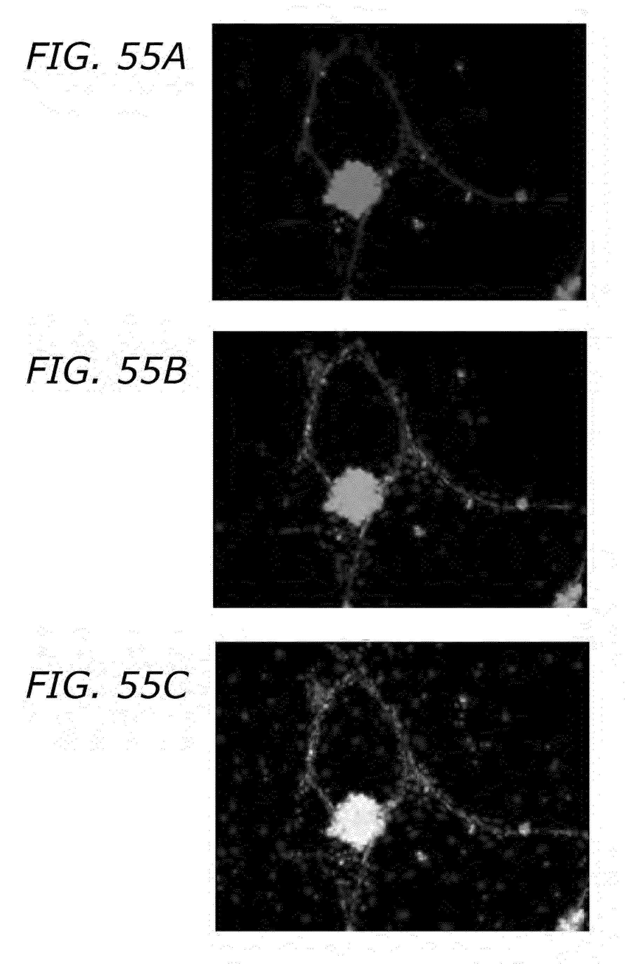

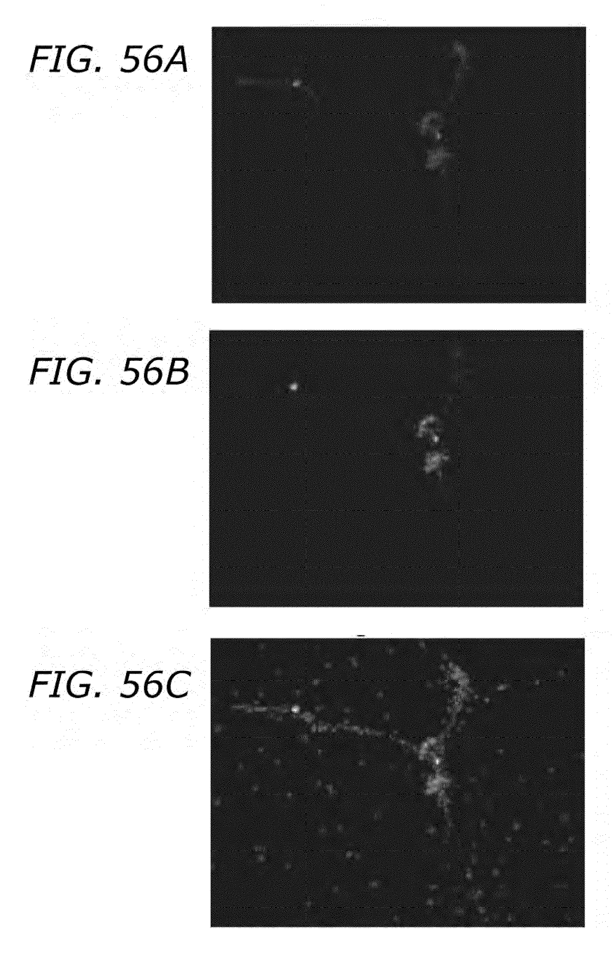

After 16 days in culture on the PA6 feeder layer, cell aggregates had long outgrowths (processes) that made a network of connections among one another (Figure 48D). Media change occurred every 2 to 3 days. Dopamine was not detected using HPLC which may be due to the sensitivity of the assay, too few dopamine producing cells in culture, or lack of dopamine. Representative images of ICC analysis are shown in Figure 49 for dopamine receptor 1 (DR1), Figure 50 for the dopamine receptor 2 (DR2), Figure 51 for tyrosine hydroxylase (TH), Figure 52 for vesicular monoamine transporter (VMAT), Figure 53 for dopa decarboxylase (DDC), and Figure 55 for dopamine transporter (DAT). Figure 54 depicts negative controls for DR1, DR2, TH, VMAT, and DDC. Figure 56 depicts negative controls for DAT. Figures 49A-56A represent staining with a 568 Alexa fluor secondary antibody (red staining). Figures 49B-56B represent human nuclear protein which stains primate nuclei as shown in green using a 488 Alexa fluor secondary antibody. Co-localization of these markers with human nuclear protein is shown in Figures 49C-56C. The cells were all positive for DR1, DR2, TH, VMAT, and DDC. DAT was dimly positive. Negative controls consist of cells stained only with secondary antibody.

### Example 8

### Culture expansion of primate germline stem cells

Germline stem cells after isolation were transferred to MEF plates and cultured in different serum free media including Mouse Serum Free Medium (MSFM), Rat Serum Free Medium (RSFM) or MEM-X^{®} media. The morphological changes of the cells and the number of germ cell colonies per well was counted during culture. Half of the medium was changed every other day.

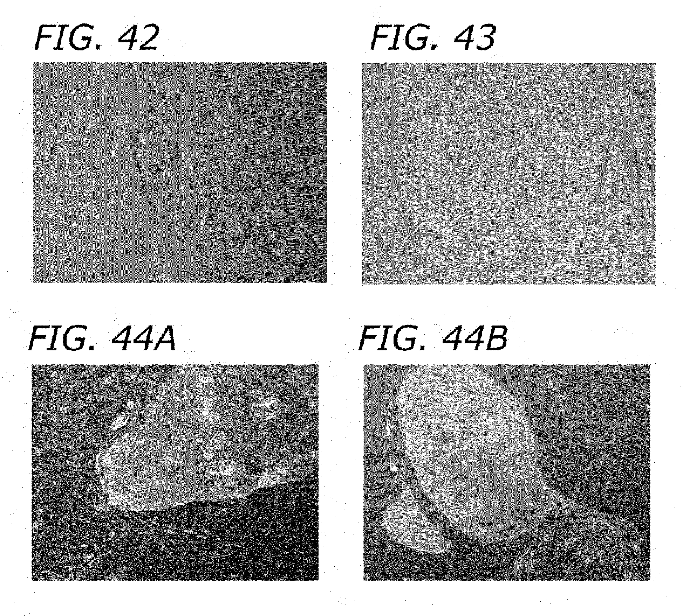

Ten days after culture, flat colonies were appeared in all media (Figures 42 and 43). Colonies in MEM-X maintained their morphology better than other two culture media. The number of colonies found in non sorted population was lower than sorted cells. Among the cell surface markers tested SSEA-4 and triple sorted cells resulted in colony formation. Depletion of SSEA-4 from triple sort resulted to very few colonies; however depletion of triple sort from SSEA-4 did not change colony formation ability. Cells positive for both SSEA-4 and triple sort formed highest number of colonies in culture and cells depleted from SSEA-4 and triple sort did not form any colony.

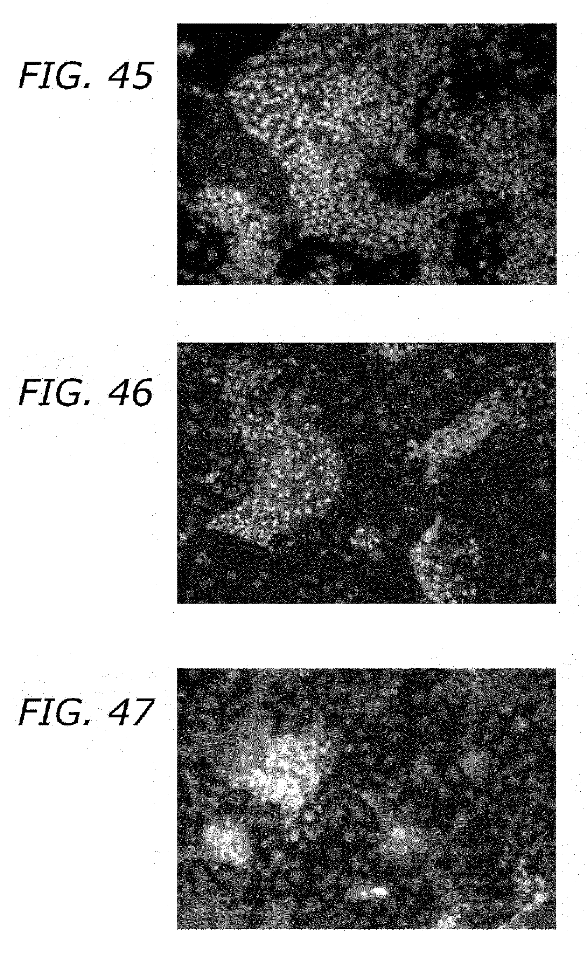

The colonies were then stained for SSEA-4 (Figure 44), GFR-α (Figure 45) and α6-integrin

### Example 9

### Isolation, Purification and Characterization of Female Germline Stem Cells

Mouse ovaries from 40-60 transgenic OG2 post-natal pups, aged 2-5 days, were dissected under a micro dissection microscope and used for cell isolation. Ovaries were first collected in a culture dish containing cold D-PBS supplemented with 4mM EDTA. Using a 5ml pipette, ovaries were then transferred with to a 50ml conical tube. After centrifugation and washing, the D-PBS wash solution was removed and the ovaries were resuspended in collagenase (1 mg/ml) and DNase-I (20 unit/ml); and, placed in a 37°C water bath. Every 10 min, the digesting ovarian tissues were physically disrupted by pipette and at the end of the incubation (30 min) 5 ml of fetal bovine serum (FBS) was added to neutralize the enzymes. The resultant cell suspension was passed through a 40µm strainer to remove tissue debris and the isolated cells were collected by centrifugation at 400xG for 10min. The supernatant enzyme-FBS solution was removed and cells were resuspended in culture medium and kept on ice until use.

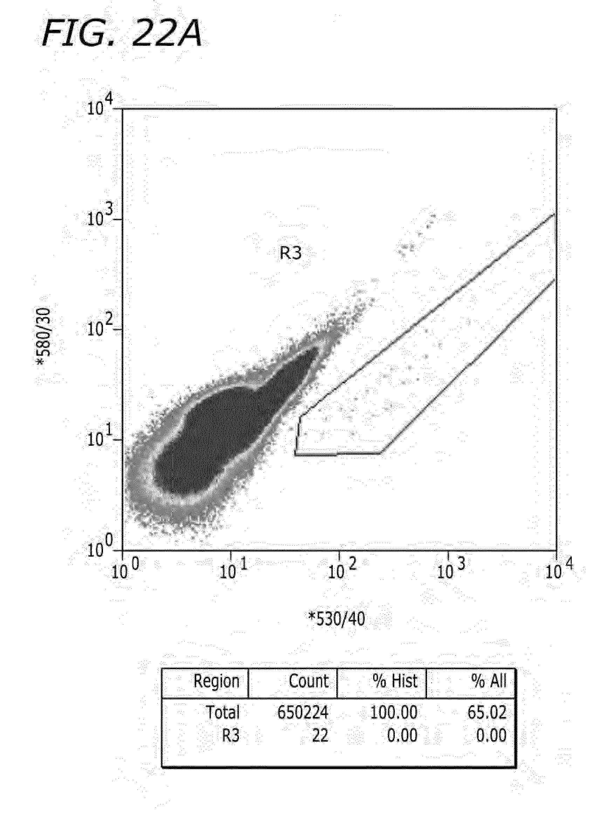

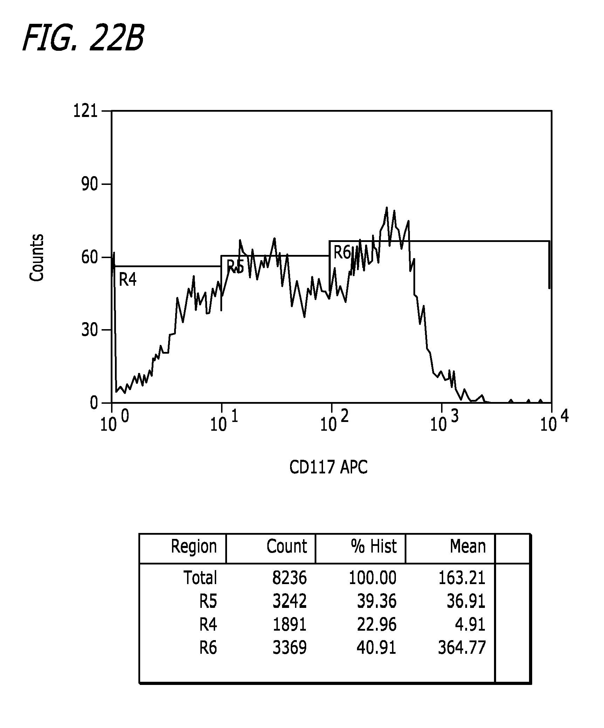

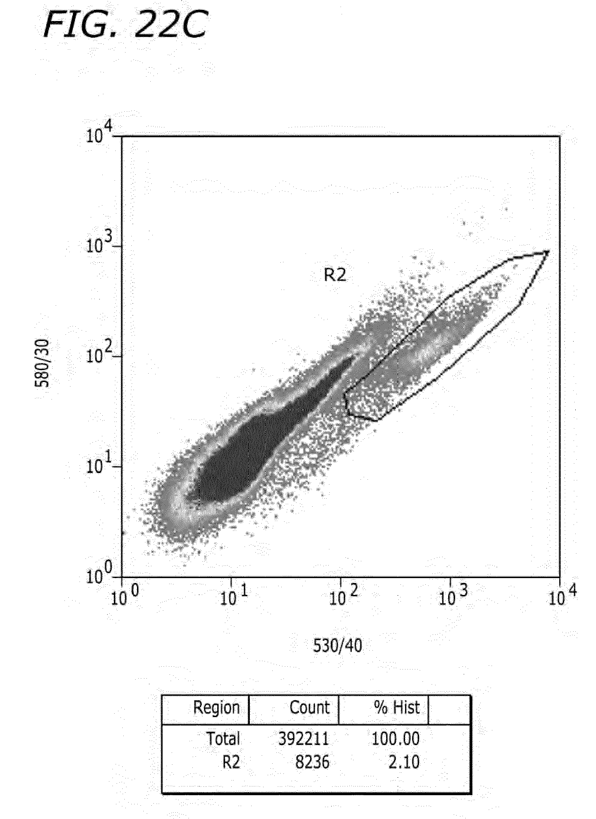

Ovarian germ-line stem cells were substantially purified by collecting GFP-positive cells by FACS identifying green fluorescent intensity (Figure 22A), gating three channels for c-Kit (R2, R3 and R4) (Figure 22B); and then sorting R3 for c-Kit intensity (Figure 22C).

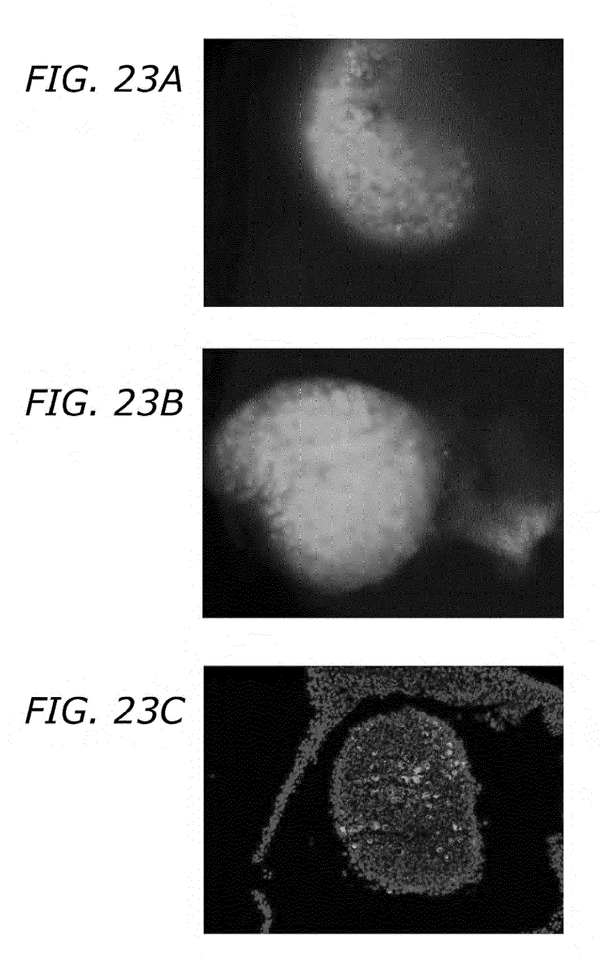

Using FACS analysis, GFP-Oct-4 positive cells were detected in neonatal (Figure 22A) and adult (Figure 22B) mice indicating the presence of germline stem cells in postnatal ovary. The percentage of germline stem cells in the mouse ovary significantly diminished with age. While 1-2 % GFP positive cells were found in the ovaries of the neonatal mice, only 0.05% were present in the adult ovary. Among the Oct-4 positive cells, 60% were negative or expressed low level of c-Kit and 40% showed high level of c-Kit expression (Figure 22C), indicating the presence of two populations among germline stem cells. Immunohistochemical analysis revealed that GFP-Oct-4 positive cells are present throughout the ovarian epithelium (Figures 23A-23C). RT-PCR analysis showed that GFP positive cells isolated from neonatal mouse ovary expressed both pluripotent marker Oct-4 and germ cell markers VASA and c-Kit confirming the presence of germline stem cells in this population, while the GFP negative cells showed only the expression of germ cell markers (Figure 24).

In contrast to expectations, freshly isolated adult or neonatal ovarian cells showed very low telomerase activity. However, RT-PCR analysis confirmed that GFP-positive cells at the onset of culture, like ESC, express Oct-4 (Figure 24). GFP-positive cells expressed Oct-4 (Figure 24, lane 5) while GFP-negative cells did not (Figure 24, lane 6). GFP-positive cells expressed higher levels of VASA (Figure 24, lane 5) than GFP-negative cells. GFP-negative cells expressed higher levels of c-Kit than GFP-positive cells.

The marker c-Kit has been associated with male germline stem cells in certain prior scientific reports. Among the Oct-4 positive cells, 60% were negative or expressed low level of c-Kit and 40% showed high level of c-Kit expression. GFP-positive cells isolated from neonatal mouse ovary expressed both pluripotent marker Oct-4 and germ cell markers VASA and c-Kit. The combined results confirm the presence of germline stem cells in the GFP-positive cell population isolated from the ovaries of OG2 mice.

GFP-positive cells cultured on feeder layers of mouse embryonic fibroblasts (MEF) formed round and flat colonies some of which had clearly defined boundaries (Figure 25A-25C and 25E), but others did not (Figure 25F). Representative of the clear-border and non-clear border colonies were picked (Figure 25B) and passaged on MEFs using collagenase (Figure 25D). After passage, cells assembled into distinctive colonies recognizable by a tight oval central grouping of small round cells surrounded by flat tightly packed cells having a more epitheliod shape (Figure 25G-25I). This colony appearance was continued beyond passage 4 (Figure 25J-25K).

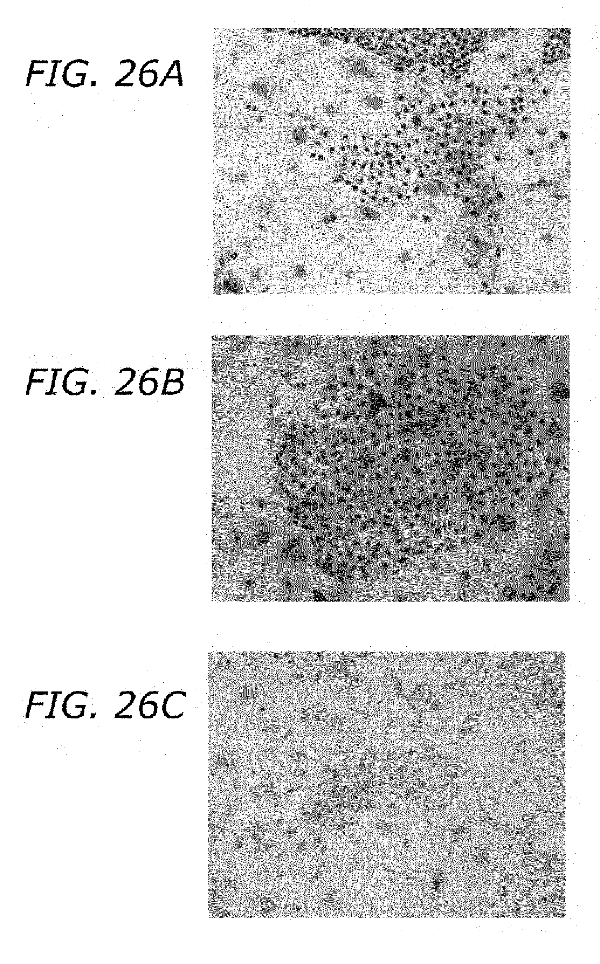

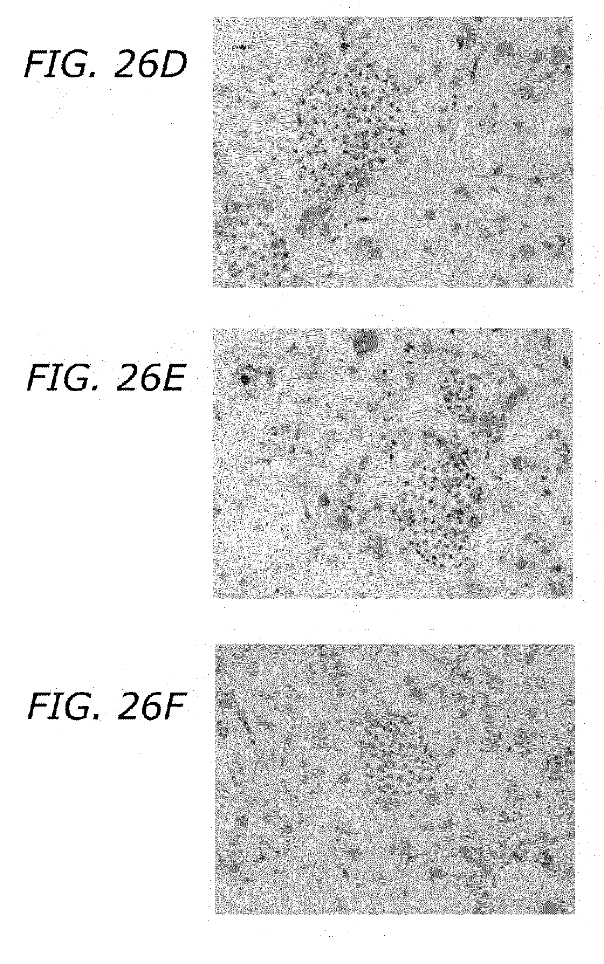

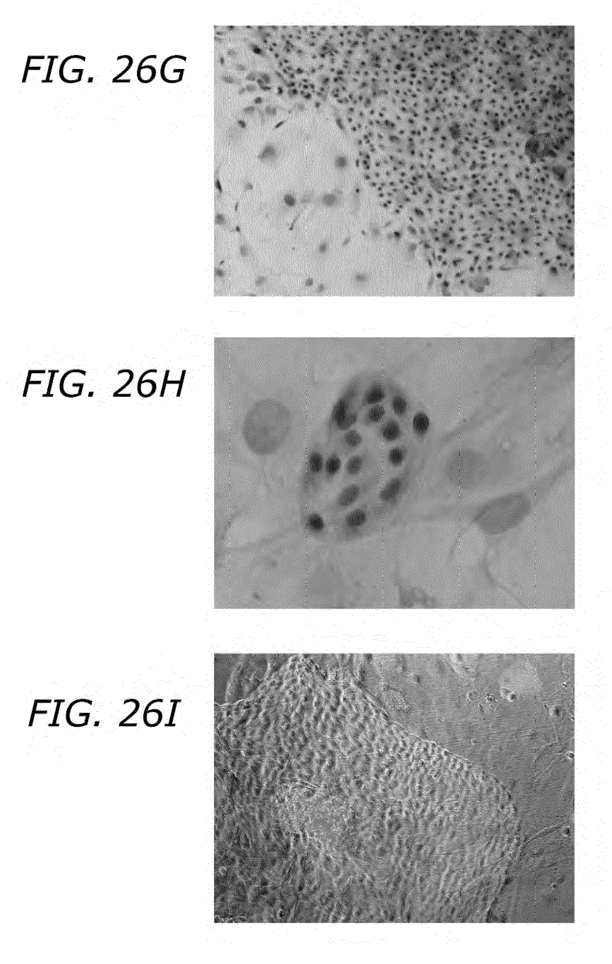

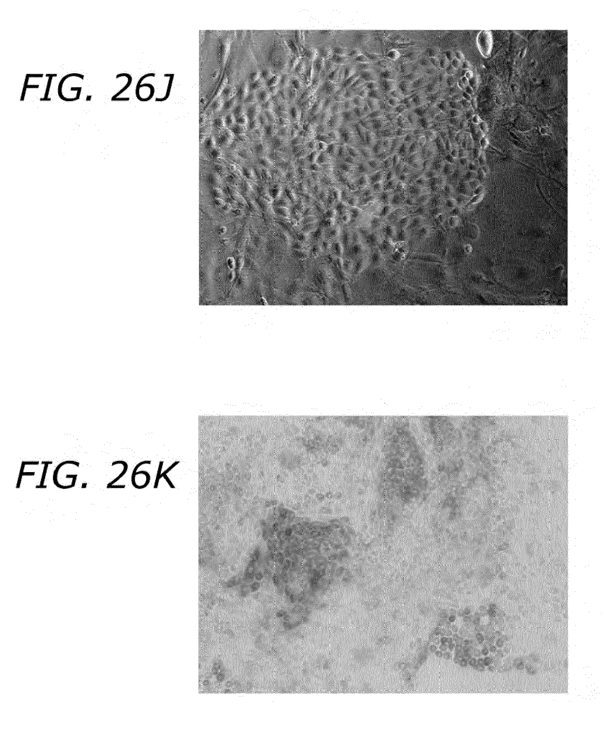

As expected, colonies of GFP-positive ovarian cells stained positive for Oct-4 (Figure 26A, 26B) by comparison with cells in the negative control lacking the second antibody (Figure 26C). Supportive of their identity as germline cells, the cells in these colonies also stained positive for pluripotent transcription factor Nanog (Figure 26D, 26E) by comparison with negative controls (Figure 26F). In addition, these cells also expressed germline specific marker VASA (Figure 26G, 26H) and stem cell marker alkaline phosphatase (Figure 26I-26K). The combined results confirm the isolation, identification, characterization and passage in tissue culture of female germline stem cells.

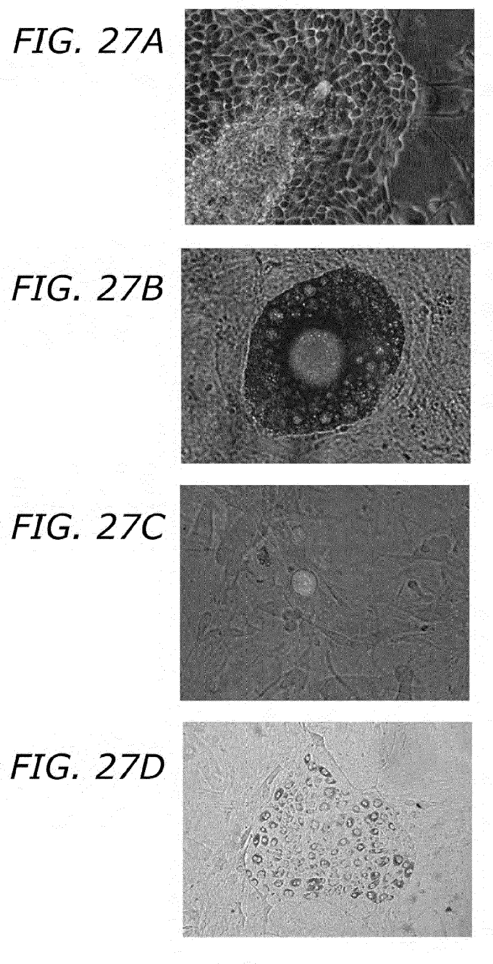

The GFP-positive colonies tolerated enzymatic digestion using collagenase and generated new colonies. However, they did not tolerate trypsinization and the majority of the colonies differentiated after trypsin treatment. GFP negative cells showed only the expression of germ cell markers and not stem cell markers. After several passages (for example, passage 15) these differentiated colonies retained their morphology, but most cells no longer expressed GFP, suggesting down-regulation of the Oct-4 promoter and possible differentiation. Only a few cells in each colony, mainly large cells in the center of the colony, showed GFP expression. With time, these GFP-positive cells appeared to form very large, up to 40µm, oval cells that were resident in structures having morphologic similarity to ovarian follicles (Figure 27A, 27B). Eventually, the oval GFP-positive cells separated from the colony, i.e., taking on the appearance of primary oocytes (Figure 27C). Overall, the results support the notion that isolated and substantially purified ovarian germline stem cells differentiate and mature *in vitro,* giving rise to primary oocytes.

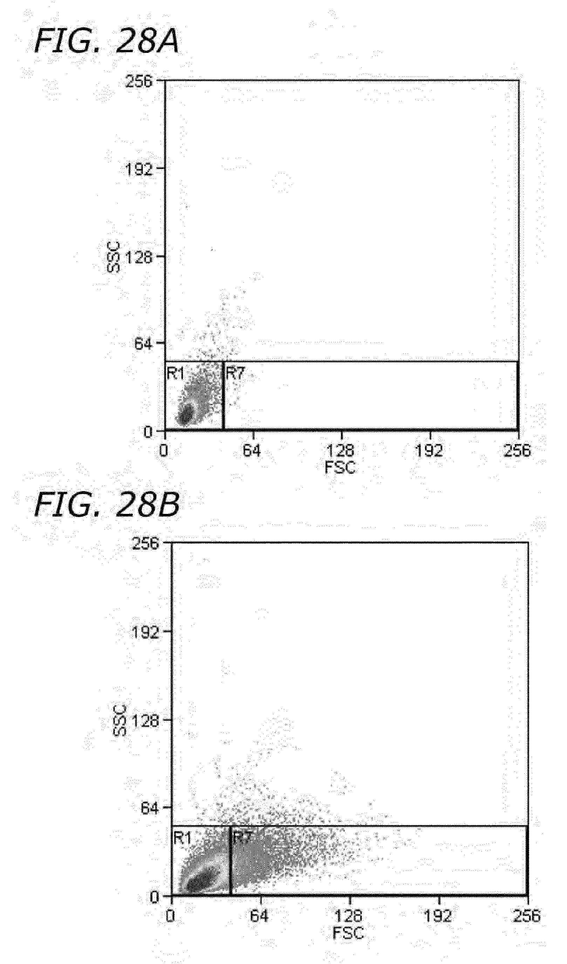

The presence and characteristics of these large oocyte-like cells were confirmed by substantially isolating and purifying them from the cultures of Figure 27 as follows: using standard FACS sizing beads, a gate was generated (R1) showing all the events 15 micrometer (µ) and smaller; MEF cells were homogenous population all accumulated in R1 (Figure 28A); significant numbers of large cells (>15µ) in the germ cell cultures of Figure 206 grown on MEF (Figure 208B). Some of these cells were about 60-70 micrometer in diameter.

MATERIALS AND METHODS

Culture of ovarian germline stem cells: GFP positive cells were cultured on mouse embryonic fibroblast (MEF) feeder in PM-1 medium in a concentration of 5000-10000 per well of a 4-well plate. Culture was maintained at 37°C and half of the medium was changed every other day. Every two weeks cells were transferred either mechanically or enzymatically (collagenase) to a new MEF plate.

Characterization of ovarian germ-line stem cells: Freshly isolated GFP-positive cells were used for telomerase assay and gene expression profiling. For ovarian histology, ovaries were fixed in 4% paraformaldehyde (PFA) in 1 M sucrose overnight at 4°C and mounted in cryostat freezing medium. Five micron sections were prepared and localization of the GFP-positive cells was determined using fluorescent microscopy. Localization of germline stem cells in the ovary was confirmed by Oct-4 and VASA double labeling. For immunocytochemistry (ICC), cultured ovarian germ-line stem cells were fixed in 2% PFA for 30 min at room temperature, washed in PBS and kept at 4°C. To characterize cultured ovarian germ-line stem cells, VASA, Oct-4, Nanog and alkaline phosphatase staining was performed using bright field ICC, as described further below.

RT-PCR and QRT-PCR analysis: Total cellular RNA was isolated using an RNeasy mini kit (Qiagen) according to the manufacturer's recommendations. The isolated RNA was then transcribed to cDNA using a Quantitect RT kit. Transcribed cDNA was purified using QIAquick PCR purification kit. For each RT-PCR reaction, 20ng of cDNA template was used in a 25µL reaction volume with HotStar Taq Plus (Qiagen) and appropriate primers. All targets were amplified for 30 cycles. Amplification products were identified by size on a 2% agarose gel. For QRT-PCR, 5ng of cDNA template was used in a 25µL reaction volume with Quantitect Sybr Green PCR master mix and the reaction mixturures were amplified using a BioRad iCycler. Each sample was assayed in triplicate and normalized to a GAPDH control.

### Example 10

### Identification, characterization and purification of human germ line stem cells

Human testes collected as testicular biopsies from patients with non obstructive azospermia or remnant of testes tissue collected after orchiectomy was used for this study. All the tissues have been donated with the informed consent of the patients. Tissues have been transferred in PBS-antibiotics in wet ice within 24 h of collection. The procedure of processing human testicular tissue is similar to primate as stated in Example 6.

Before cell isolation, a tissue sample was taken for ICC, and two small pieces of testes were taken for RNA and DNA extractions. Following cell isolation and determination of viability and cell number samples were taken for RNA and DNA analysis. Methods for ICC, RNA and DNA extractions are similar to the primate as stated in Example 6. In addition, cells were labeled with cell surface markers previously developed for separation of primate germline stem cells were used by magnetic cell sorting and flow cytometry. Antibodies and methods used for flow cytometry is similar to that used for separation of primate germline stem cells as stated in Example 6.

The population of germ cells was also enriched by tagging with magnetic microbeads and passing the cells through a magnetic column. Freshly isolated testicular cells were labeled with biotinylated antibodies for SSEA4 or for alpha-6-integrin and Thy-1 (Ebioscience, Abcam, BD Pharrmigen, respectively). Once biotinylated, the cells were labelled with streptavidin magnetic microbeads (Miltenyi Biotec). Magnetically labeled cells were selected for by passing the cells through a column in the presence of a magnet (both Miltenyi Biotec).

Magnetically labeled cells were removed from the column by removing the column from the magnet, freeing the cells to be washed off of the column. This process was successful in enriching the population of cells positive for each of the markers up to 22X the original percentage in freshly isolated cells. In addition, magnetic sorting could provide a population as high as 90% purely labeled cells. This enrichment process was used in conjugation with fluorescent flow cytometry. By magnetically sorting the cell isolation before performing fluorescent flow cytometry the amount of time needed to sort out fluorescently labeled cells was greatly reduced and the number of fluorescently labeled cells that could be sorted out was greatly increased.

Sorted cells were then used for RT-PCR and DNA analysis. Also in some samples cells were subjected to spermatogonial transplantation assay using immunodefficient mice as recipients. Technique of spermatogonial stem cell transplantation is similar to the mouse and primate as stated in Examples 5 and 6.

Immunohistochemical staining on frozen sections prepared from both testicular biopsies and remnant testes tissue revealed that there are many alpha-6 integrin positive cells at the basement membrane of tubular cross sections. Also SSEA-4 positive cells and GFR-alfa-1 positive cells were found at the basement membrane of the seminiferous tubules.

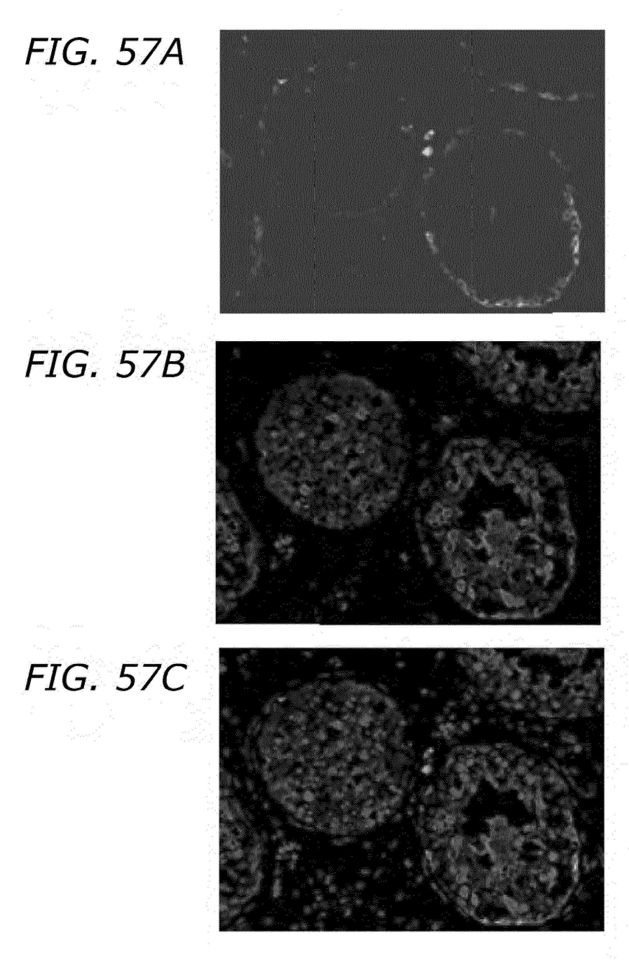

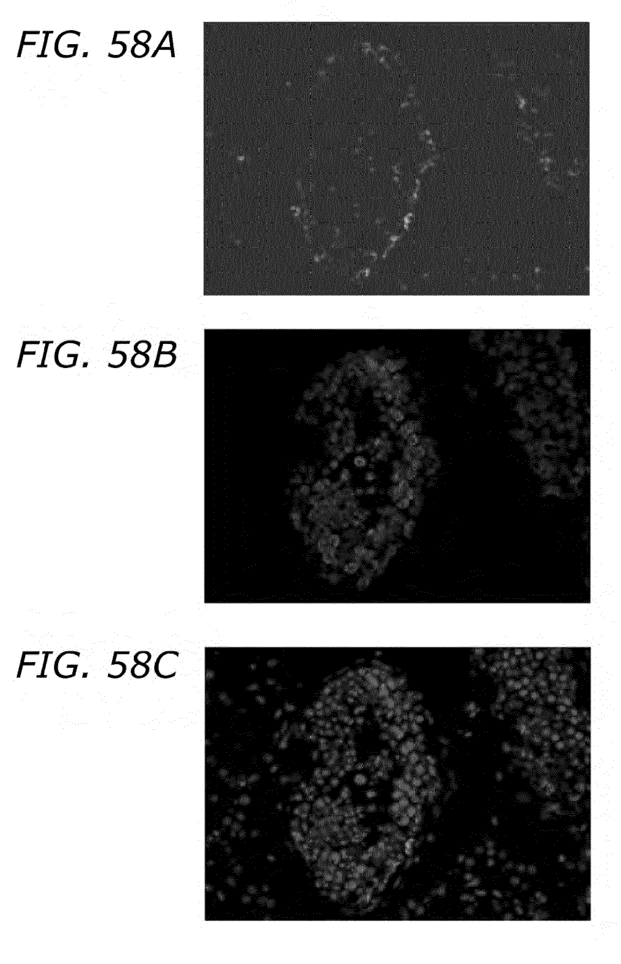

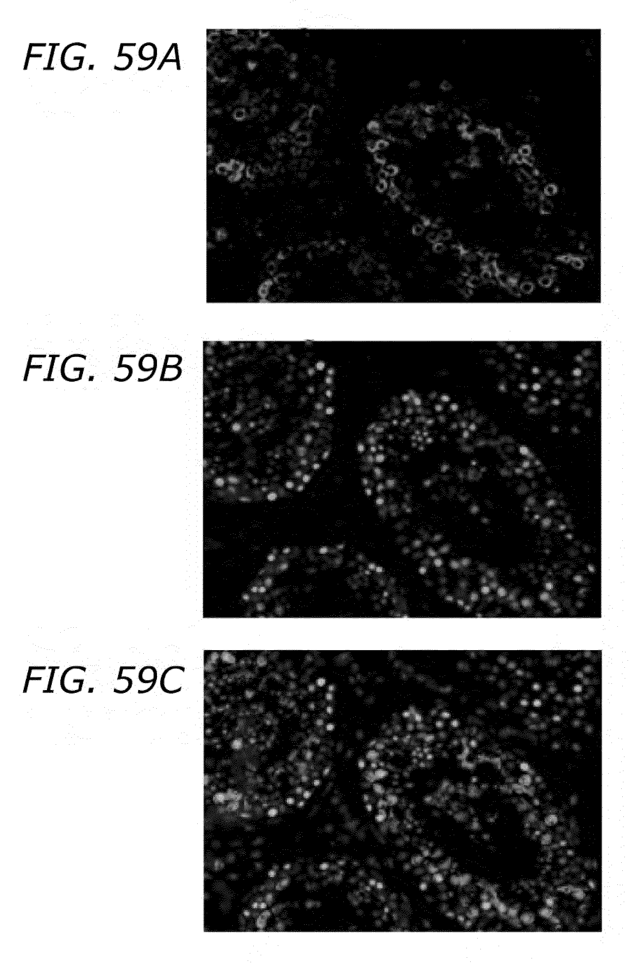

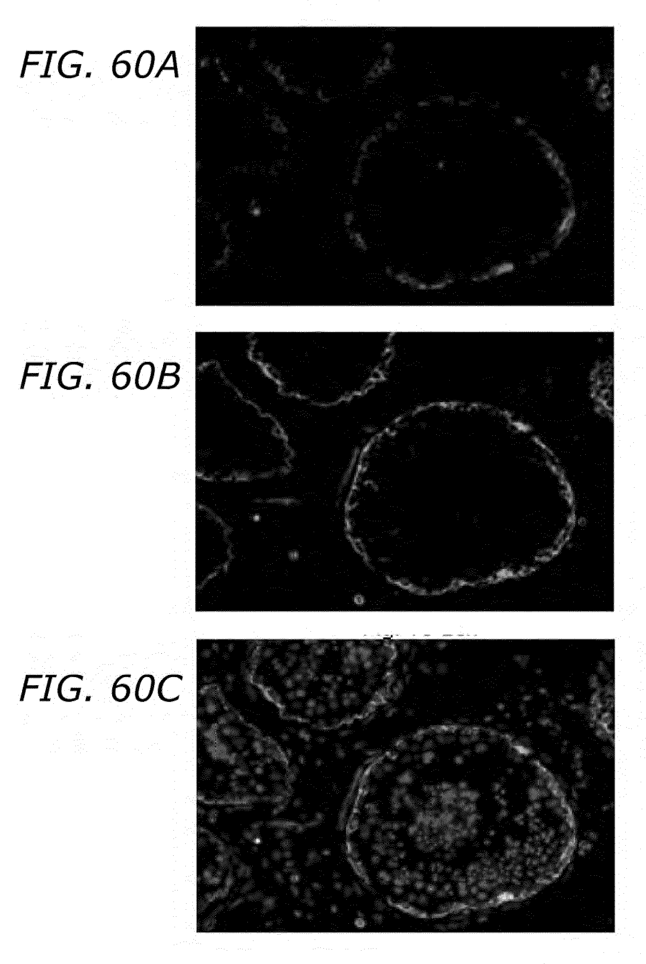

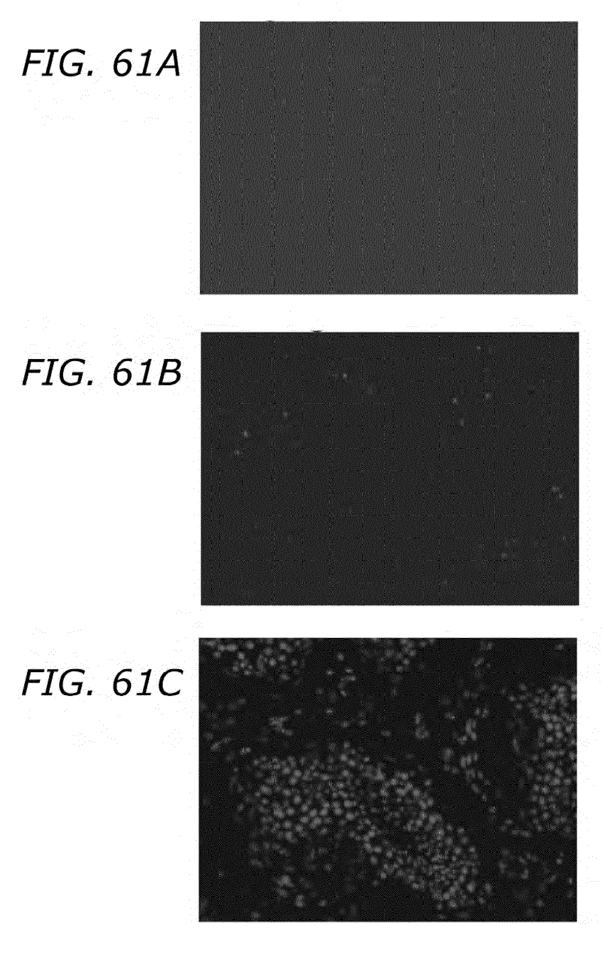

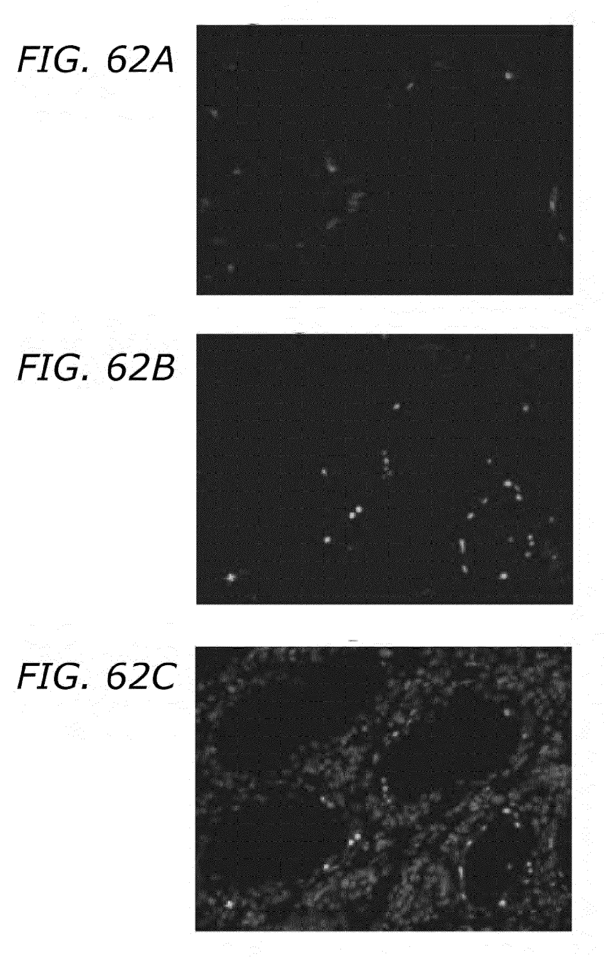

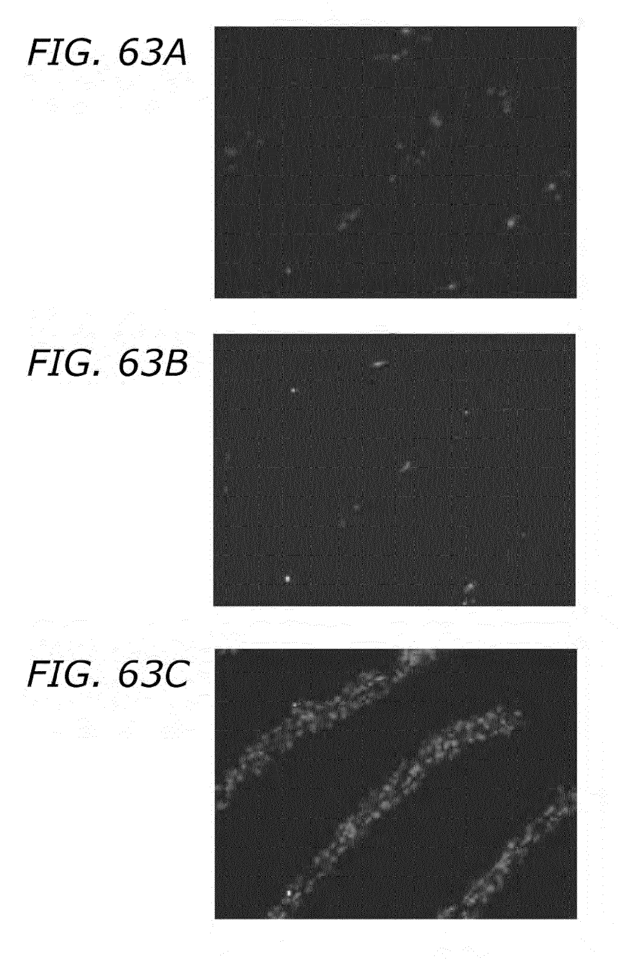

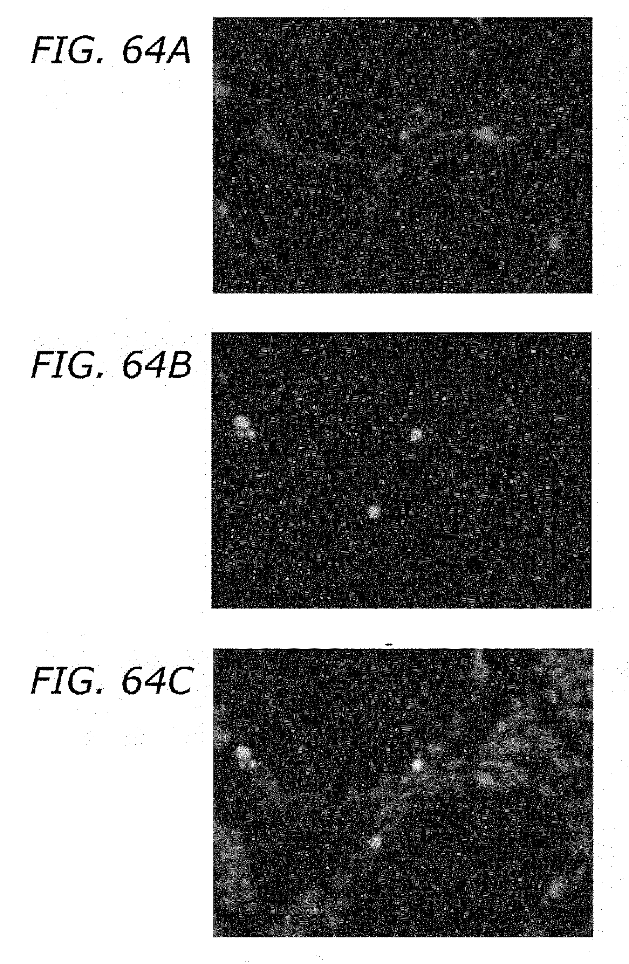

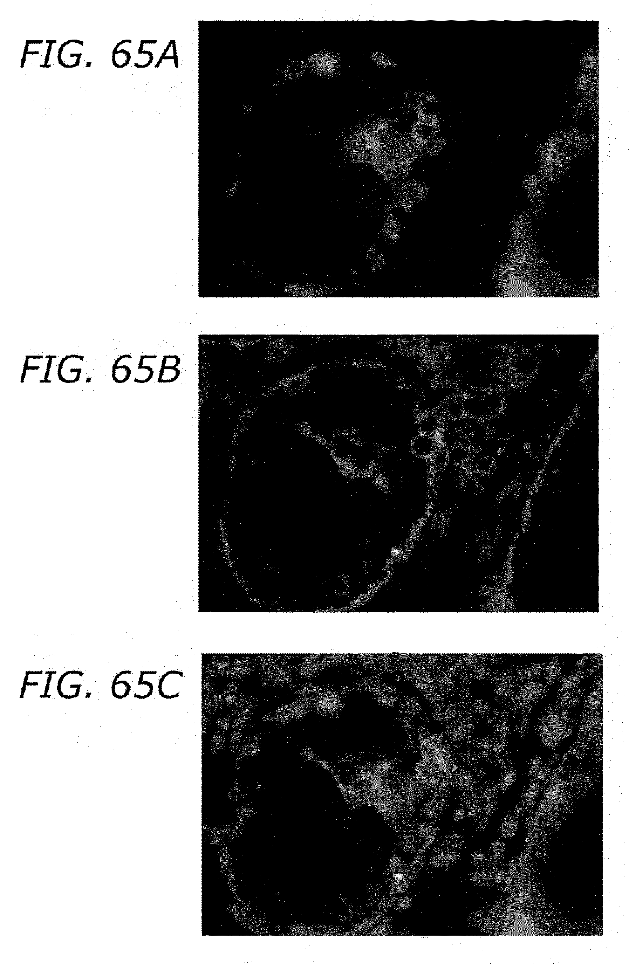

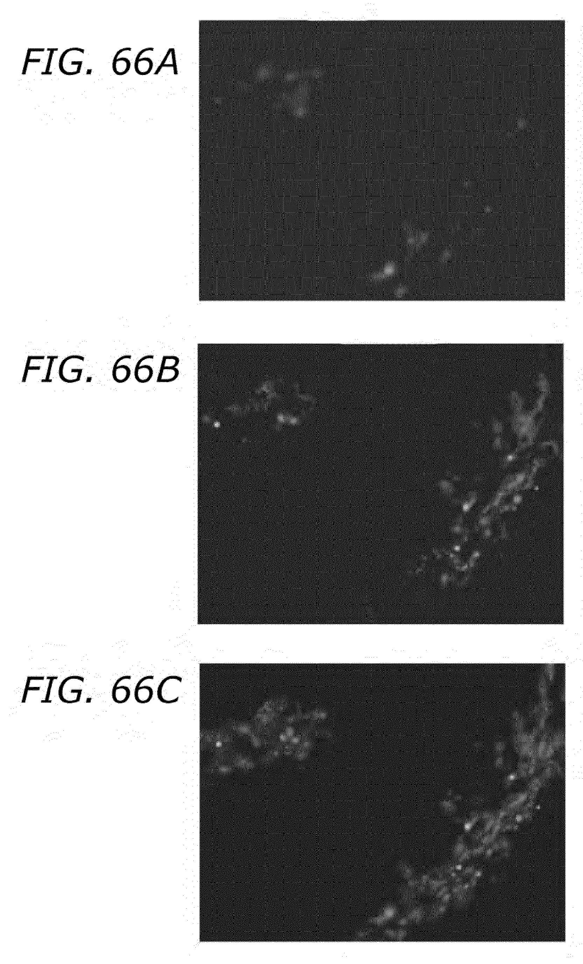

Human THT-1 stained with SSEA4 (Figure 57A) and Vasa (Figure 57B) and the two merged (Figure 57C). THT2 stained with GFR-alpha (Figure 58A) and Vasa (Figure 58B) and the two merged (Figure 58C). THT-1 stained for VASA (Figure 59A) and Nanog (Figure 59B) and the two merged (Figure 59C). Human bHT-1 stained for SSEA4 (Figure 60A) and alpha-6 (Figure 60B) and the two combined (Figure 60C). Negative controls for (Figures 57-60) consist of human testis sections stained only with secondary antibody Figure 61A-C). Human THT-2 SSEA4 positive magnetic bead sorted cells were transplanted into busulfan treated recipient mouse testes and after one month were sectioned and stained for the following markers: SSEA4 (Figure 62A) and human nuclear protein (Figure 62B) and the two merged (Figure 62C), alpha-6 (Figure 64A) and human nuclear protein (Figure 64B) and the two merged (Figure 64C), SSEA4 (Figure 65A) and alpha-6 (Figure 65B) and the two merged (Figure 65C) Negative controls for (Figure 62) and (Figures 64 and 65) consist of human THT-2 transplanted cells in mouse testis sections stained only with secondary antibody (Figure 63A-C) and (Figure 66A-C) respectively. All stains contain a general nuclear dye.

Flow cytometry analysis confirmed immunohistochemical observation and positive populations for alpha-6 integrin were found in samples collected from human testes. In addition distinct population of Thy-1 positive cells were found. Co-localization of Thy-1 and alfa-6 integrin showed that there are three subpopulation of Thy-1 positive cells within human testes: 1) aThy-1 medium and integrin low, 2) a Thy-1 high and integrin medium and 3) a Thy-1 high and integrin negative. Most of the integrin positive cells were Thy-1 negative. There were also clear population of SSEA-4 (10-12%) and GFR-alfa (1-5%) cells found in human testes. Magnetic sorting significantly enhanced the percentage of SSEA4 positive cells to 44% indicating a 4 fold increase for this marker.

Quantitative RT-PCR analysis revealed that among the samples tested SSEA-4 positive cells and GFR-alfa positive cells express highest levels of spermatogonial stem cell markers including C-RET PLZF, and TERT and germ cell markers including VASA and DAZL. Telomerase activity is indicative of spermatogonial stem cells. Spermatogonial stem cell transplantation revealed that SSEA-4 positive cells colonize testes of recipient mice and repopulate, indicating that these cells are functional spermatogonial stem cells.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g. "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is herein deemed to contain the group as modified thus fulfilling the written description of all Markush groups used in the appended claims.

Certain embodiments of this invention are described herein, including the best mode known to the inventors for carrying out the invention. Of course, variations on those embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Furthermore, numerous references have been made to patents and printed publications throughout this specification. Each of the above cited references and printed publications are individually incorporated herein by reference in their entirety.

In closing, it is to be understood that the embodiments of the invention disclosed herein are illustrative of the principles of the present invention. Other modifications that may be employed are within the scope of the invention. Thus, by way of example, but not of limitation, alternative configurations of the present invention may be utilized in accordance with the teachings herein. Accordingly, the present invention is not limited to that precisely as shown and described.

## Claims

1. An isolated population of postnatal female primate germline cells consisting of isolated gonadal cells **characterized by**:
the expression of at least one marker selected from the group consisting of GFR-α1, α6-integrin, Thy-1, SSEA-4, CD9, VASA, DAZL, and c-Kit; and
the expression of a level of telomerase of at least 20% of the telomerase activity of embryonic stem cells.

2. The isolated population of postnatal female primate germline cells of claim 1, wherein the cells express SSEA-4, c-Kit, Oct-4, and Nanog.

3. The isolated population of postnatal female primate germline cells of claim 2, wherein said cells further express at least one of Thy-1, alkaline phosphatase, VASA, GFR-α1, and α6-integrin.

4. The isolated population of postnatal female primate germline cells of claim 3, wherein greater than about 50% of the cells express both GFR-α1 and VASA.

5. The isolated population of postnatal female primate germline cells of claim 2 wherein said cells express Oct-4, Nanog, SSEA-4, c-Kit, alkaline phosphatase and VASA.

6. The isolated population of postnatal female primate germline cells according to claim 1, wherein said cells exhibit at least one of the properties selected from the group consisting of:
a cell cycle doubling time of about 72 hours;
expression of germline-specific genes at a higher level than expressed in embryonic stem cells (ESC);
expression of pluripotency genes at a lower level than ESC;
a greater dependent on epidermal growth factor (EDF) and glial cell derived neurotrophic factor (GDNF) for their self renewal than on leukemia inhibitory factor (LIF) or fibroblast growth factor (FGF2); and
a lower level of SSEA-1 expression than ESC.

7. The isolated population of postnatal female primate germline cells of claim 6, wherein the pluripotent genes are selected from the group consisting of Oct-4, Nanog, Dppa-5, Sox2, alkaline phosphatase, and Crypto.

8. The isolated population of postnatal female primate germline cells of claim 1, wherein said cells are capable of differentiating into an oocyte-like cell.

9. The isolated population of postnatal female primate germline cells of claim 1 wherein said telomerase activity is at least 50% the telomerase activity of embryonic stem cells.

10. The isolated population of postnatal female primate germline cells of claim 1, wherein said cells are capable of differentiating into cells of the ectoderm, cells of the endoderm and cells of the mesoderm.

11. A long-term culture of cells comprising the postnatal female primate germline cells of claim 1.

12. A method of isolating a population of postnatal female primate germline stem cells, comprising the steps of:
selecting a postnatal female primate gonadal cell exhibiting a germline stem cell surface marker phenotype; and
determining that said postnatal female primate gonadal cells expresses at least one germline stem cell gene.

13. The method of claim 12 wherein said postnatal female primate gonadal cells express Oct-4, SSEA-4, Nanog, and c-Kit.

14. The method of claim 12, wherein the germline surface marker phenotype comprises at least one phenotype selected from the group consisting of Epithelial Cell Adhesion/Activating Molecule (EpCAM) negative, GDNF receptor (GFR-α1) positive, c-Kit positive, CD9 positive, CD90 positive, CD49f positive, and SSEA-4 positive.

15. The method of claim 12, wherein the postnatal female primate germline stem cell gene is selected from the group consisting of telomerase, VASA, c-RET,GFR-α1, and DAZL.

16. The isolated population of postnatal female primate cells of claim 1 or the method of claim 13, wherein the primate is a human.
